# EUROPEAN PATENT SPECIFICATION

(11) **EP 4 106 793 B1**
(45) Date of publication and mention of the grant of the patent: **18.03.2026**
(21) Application number: 21756608.2
(22) Date of filing: 19.02.2021
(51) Int. Cl.: A61K 31/517, A61K 39/395, A61K 45/06, A61K 35/00, A61K 38/18, A61K 38/21, A61P 35/00

(54) **COMBINATION OF ERLOTINIB WITH ANIFROLUMAB FOR USE IN THE TREATMENT OF NON-SMALL CELL LUNG CANCER**
KOMBINATION VON ERLOTINIB MIT ANIFROLUMAB ZUR VERWENDUNG BEI DER BEHANDLUNG VON NICHT-KLEINZELLIGEM LUNGENKREBS
COMBINAISON D'ERLOTINIB ET D'ANIFROLUMAB POUR UNE UTILISATION DANS LE TRAITEMENT DU CANCER DU POUMON NON À PETITES CELLULES

(30) Priority: 19.02.2020 US 202062978777 P; 03.03.2020 US 202062984624 P
(43) Date of publication of application: 28.12.2022
(73) Proprietor: United States Government as Represented by The Department of Veterans Affairs, Washington, DC 20420 (US)
(72) Inventor: HABIB, Amyn, Dallas, TX 75216 (US)
(74) Representative: CH Kilger Anwaltspartnerschaft mbB
(86) International application number: PCT/US2021/018718
(87) International publication number: WO 2021/168202

(56) References cited:
- US-A1- 2018 264 129
- US-A1- 2019 016 808
- LEE HO-JUNE ET AL: "Drug Resistance via Feedback Activation of Stat3 in Oncogene-Addicted Cancer Cells (Article plus Supplemental Information)", vol. 26, no. 2, 1 August 2014 (2014-08-01), US, pages 207 - 221, XP055811008, ISSN: 1535-6108, Retrieved from the Internet <URL:https://ars.els-cdn.com/content/image/1-s2.0-S1535610814002256-mmc3.pdf> DOI: 10.1016/j.ccr.2014.05.019
- PARK JI SOO ET AL: "A phase Ib study of the combination of afatinib and ruxolitinib in EGFR mutant NSCLC with progression on EGFR-TKIs", LUNG CANCER, ELSEVIER, AMSTERDAM, NL, vol. 134, 1 August 2019 (2019-08-01), pages 46 - 51, XP085732729, ISSN: 0169-5002, [retrieved on 20190528], DOI: 10.1016/J.LUNGCAN.2019.05.030
- NIE PEIPEI ET AL: "Synergistic Induction of Erlotinib-Mediated Apoptosis by Resveratrol in Human Non-Small-Cell Lung Cancer Cells by Down-Regulating Survivin and Up-Regulating PUMA", vol. 35, no. 6, 1 January 2015 (2015-01-01), CH, pages 2255 - 2271, XP093127601, ISSN: 1015-8987, Retrieved from the Internet <URL:https://www.karger.com/Article/Pdf/374030> DOI: 10.1159/000374030
- GARRIDO-LAGUNA I ET AL: "A phase I/II study of decitabine in combination with panitumumab in patients with wild-type (wt)KRASmetastatic colorectal cancer", INVESTIGATIONAL NEW DRUGS, SPRINGER US, NEW YORK, vol. 31, no. 5, 1 October 2013 (2013-10-01), pages 1257 - 1264, XP036727721, ISSN: 0167-6997, [retrieved on 20131001], DOI: 10.1007/S10637-013-9947-6
- JULIE BAUMAN ET AL: "A phase I study of 5-azacytidine and erlotinib in advanced solid tumor malignancies", CANCER CHEMOTHERAPY AND PHARMACOLOGY, SPRINGER, BERLIN, DE, vol. 69, no. 2, 8 September 2011 (2011-09-08), pages 547 - 554, XP035006929, ISSN: 1432-0843, DOI: 10.1007/S00280-011-1729-2
- GREEN COLIN ET AL: "Combination of the vascular disrupting agent DMXAA (AS1404) with cetuximab produces synergistic antitumor activity in lung cancer xenografts", CANCER RESEARCH, 1 May 2007 (2007-05-01), pages 1 - 3, XP093127442, Retrieved from the Internet <URL:https://aacrjournals.org/cancerres/article/67/9_Supplement/3996/537746/Combination-of-the-vascular-disrupting-agent-DMXAA> [retrieved on 20240205]
- SATOSHI OKAZAKI ET AL: "Predictive value of TLR7 polymorphism for cetuximab-based chemotherapy in patients with metastatic colorectal cancer", INTERNATIONAL JOURNAL OF CANCER, JOHN WILEY & SONS, INC, US, vol. 141, no. 6, 21 June 2017 (2017-06-21), pages 1222 - 1230, XP071290032, ISSN: 0020-7136, DOI: 10.1002/IJC.30810
- LUKHELE SABELO ET AL: "Type I interferon signaling, regulation and gene stimulation in chronic virus infection", SEMINARS IN IMMUNOLOGY, W.B. SAUNDERS COMPANY, PA, US, vol. 43, 30 May 2019 (2019-05-30), XP085918268, ISSN: 1044-5323, [retrieved on 20190530], DOI: 10.1016/J.SMIM.2019.05.001
- CARBONE MARIA ET AL: "Topical Plant Polyphenols Prevent Type I Interferon Signaling in the Skin and Suppress Contact Hypersensitivity", INTERNATIONAL JOURNAL OF MOLECULAR SCIENCES, vol. 19, no. 9, 6 September 2018 (2018-09-06), Basel, CH, pages 2652, XP093127435, ISSN: 1422-0067, DOI: 10.3390/ijms19092652
- BORDEN ERNEST C: "Interferons [alpha] and [beta] in cancer: therapeutic opportunities from new insights", NATURE REVIEWS DRUG DISCOVERY, NATURE PUBLISHING GROUP, GB, vol. 18, no. 3, 24 January 2019 (2019-01-24), pages 219 - 234, XP036715679, ISSN: 1474-1776, [retrieved on 20190124], DOI: 10.1038/S41573-018-0011-2
- ZHU YUANYUAN ET AL: "STING: a master regulator in the cancer-immunity cycle", vol. 18, no. 1, 4 November 2019 (2019-11-04), GB, XP093127358, ISSN: 1476-4598, Retrieved from the Internet <URL:http://link.springer.com/article/10.1186/s12943-019-1087-y/fulltext.html> DOI: 10.1186/s12943-019-1087-y
- ANONYMOUS: "AstraZeneca presents positive new data on anifrolumab in lupus at American College of Rheumatology Annual Scientific Meeting", 10 November 2015 (2015-11-10), pages 1 - 6, XP093128273, Retrieved from the Internet <URL:https://www.astrazeneca.com/media-centre/press-releases/2015/AstraZeneca-presents-positive-new-data-on-anifrolumab-in-lupus-at-American-College-of-Rheumatology-Annual-Scientific-Meeting-10112015.html#> [retrieved on 20240206]
- IM JIN S., HERRMANN AMANDA C., BERNATCHEZ CHANTALE, HAYMAKER CARA, MOLLDREM JEFFREY J., HONG WAUN KI, PEREZ-SOLER ROMAN: "Immune-Modulation by Epidermal Growth Factor Receptor Inhibitors: Implication on Anti-Tumor Immunity in Lung Cancer", PLOS ONE, vol. 11, no. 7, 28 July 2016 (2016-07-28), pages e0160004, XP055850258, DOI: 10.1371/journal.pone.0160004

## Description

### CROSS-REFERENCE TO RELATED APPLICATIONS

This Application claims the benefit of U.S. Application No. 62/978,777, filed on February 19, 2020, and U.S. Application No. 62/984,264, filed on March 03, 2020).

### BACKGROUND

Interferon regulatory factor 3 (IRF3) plays a central role in innate immunity. IRF3 is a transcription factor that is expressed constitutively and in response to viral infection, and induces the transcription of type I interferons. IRF3 is activated in response to cytosolic recognition of nucleic acids or tissue damage by a number of pattern recognition receptors (PRRs). In response to viral infection, IRF3 becomes phosphorylated leading to its dimerization and nuclear translocation, leading to induction of Type I interferons and orchestration of the antiviral response. IRF3 is activated by the TANK-binding kinase TBK1 and by IKKε. TBK1 is ubiquitously expressed and activated in response to activation of pattern recognition receptors (PRRs) and associated adaptor signaling proteins such as RIG-I/MAVS, cGAS-STING, and TLR3/4-TRIF.

Activation of IRF3 results in production of Type I interferons, cytokines essential for generating antiviral responses and activating innate immunity. Type I interferons include interferon-α and interferon-β and bind to the IFNAR, composed of IFNAR1 and IFNAR2 chains. Type I interferons play a tumor suppressive role. Indeed, IFNs have been used for treating certain types of cancer (kidney cancer, melanoma, chronic myeloid leukemia) and are thought to function through multiple mechanisms including promotion of anti-tumor immunity, anti-angiogenesis, promoting inflammation in the tumor microenvironment and a direct role in suppression of proliferation and apoptosis in tumor cells. Importantly, homozygous deletion of genes is common in 9p21.3, the locus for the type I interferon gene cluster. Homozygous deletion of the type I interferon genes is widespread in cancer, including about 10% of NSCLC. Importantly, Type I IFN loss confers a worse prognosis in multiple cancer types.

The EGFR is widely expressed in non-small cell lung cancer (NSCLC) and is an important target in NSCLC. EGFR inhibition using tyrosine kinase inhibitors (TKIs) is highly effective initially in the subset of patients with EGFR-activating mutations, who comprise about 10-15% of NSCLC patients in Western populations. However, the majority of NSCLC tumors express EGFR wild type (EGFRwt) and do not respond to EGFR inhibition. Nevertheless, EGFR ligands are commonly expressed in lung cancer. Furthermore, a constitutive overexpression-induced EGFRwt signaling has also been reported. Thus, it is possible that EGFRwt could play an oncogenic role in lung cancer. Moreover, secondary resistance inevitably develops in initially responsive EGFR mutant tumors. The major mechanisms of resistance to EGFR inhibition in NSCLC include EGFR mutations such as the T790M mutation and activation of other RTKs such as Met. In addition, EGFR inhibition triggers a rapid adaptive response in NSCLC that likely contributes to secondary resistance, but may also induce primary resistance to treatment. This adaptive response is broad and may involve bypass signaling pathways such as STAT3 or NF-κB. It was recently reported that a rapid TNF-driven adaptive response plays a key role in resistance to EGFR inhibition in NSCLC and in glioma. Combination treatments including the EGFR inhibitor erlotinib have been previously studied in the treatment of NSCLC (Lee H-J. et al., Cancer Cell, 2014, vol. 26:2, pages 207-221, DOI: 10.1016/j.ccr.2014.05.019; Nie P. et al., Cellular Physiology and Biochemistry, 2015, vol. 35:6, pages 2255-2271, DOI: 10.1159/000374030; Bauman J. et al., Cancer Chemotherapy and Pharmacology, 2011, vol. 69:2, pages 547-554, DOI: 10.1007/S00280-011-1729-2).

In this study we examine an adaptive response to EGFR inhibition in NSCLC mediated by upregulation of Type I IFNs.

Thus, there remains a need for compositions and methods for treating NSCLC that express EGFRwt and/or that are resistant to EGFR inhibition. These needs and others are met by the present invention.

### SUMMARY

The present invention is defined by the claims. Any subject-matter (disclosure) falling outside the scope of the claims is provided for information only. Methods of treatment of the human body by therapy, referred to in this description, are not part of the present invention as such, but are described here in relation to compounds and pharmaceutical compositions for use in said methods of treatment of the human body by therapy, according to the present invention. According to a first embodiment, the present invention relates to a combination for use in the treatment of non-small cell lung cancer (NSCLC), the combination comprising an effective amount of erlotinib and anifrolumab.

According to a further embodiment, the present invention relates to a pharmaceutical composition comprising erlotinib, anifrolumab, and a pharmaceutically acceptable carrier.

In accordance with a more general aspect, which is not part of the present invention, described herein are compounds and compositions for use in the prevention and treatment of disorders of cancers such as, for example, sarcomas, carcinomas, hematological cancers, solid tumors, breast cancer, cervical cancer, gastrointestinal cancer, colorectal cancer, brain cancer, skin cancer, prostate cancer, ovarian cancer, bladder cancer, thyroid cancer, testicular cancer, pancreatic cancer, endometrial cancer, melanomas, gliomas, leukemias, lymphomas, chronic myeloproliferative disorders, myelodysplastic syndromes, myeloproliferative neoplasms, and plasma cell neoplasms (myelomas).

Thus, according to an aspect which is not part of the present invention, disclosed are methods for treating cancer in a subject, the method comprising administering to the subject an effective amount of an agent that modulates epidermal growth factor receptor (EGFR) signaling, or a pharmaceutically acceptable salt thereof, and an agent that modulates interferon (IFN) signaling, or a pharmaceutically acceptable salt thereof.

Also disclosed, according to an aspect which is not part of the present invention, are methods for treating cancer in a patient in need thereof, said method comprising administering to said patient an effective amount of erlotinib and anifrolumab.

Also disclosed, according to an aspect which is not part of the present invention, are pharmaceutical compositions comprising: (a) an agent that modulates EGFR signaling, or a pharmaceutically acceptable salt thereof; (b) an agent that modulates IFN signaling, or a pharmaceutically acceptable salt thereof; and (c) a pharmaceutically acceptable carrier, wherein at least one of the agent that modulates EGFR signaling and the agent that modulates IFN signaling is present in an effective amount.

Also disclosed, according to an aspect which is not part of the present invention, are methods for making a pharmaceutical composition, the method comprising combining: (a) an agent that modulates EGFR signaling, or a pharmaceutically acceptable salt thereof; (b) an agent that modulates IFN signaling, or a pharmaceutically acceptable salt thereof; and (c) a pharmaceutically acceptable carrier, wherein at least one of the agent that modulates EGFR signaling and the agent that modulates IFN signaling is present in an effective amount.

Also disclosed, according to an aspect which is not part of the present invention, are kits comprising an agent that modulates EGFR signaling, or a pharmaceutically acceptable salt thereof, and an agent that modulates IFN signaling, or a pharmaceutically acceptable salt thereof, and one or more of: (a) an agent associated with the treatment of cancer; (b) instructions for administering the agent that modulates EGFR signaling and/or the agent that modulates IFN signaling in connection with treating cancer; and (c) instructions for treating cancer.

### BRIEF DESCRIPTION OF THE FIGURES

The accompanying figures, which are incorporated in and constitute a part of this specification, illustrate several aspects and together with the description serve to explain the principles of the invention.

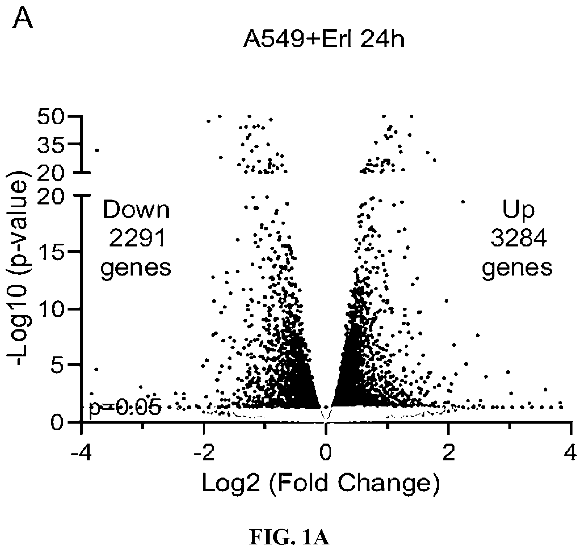

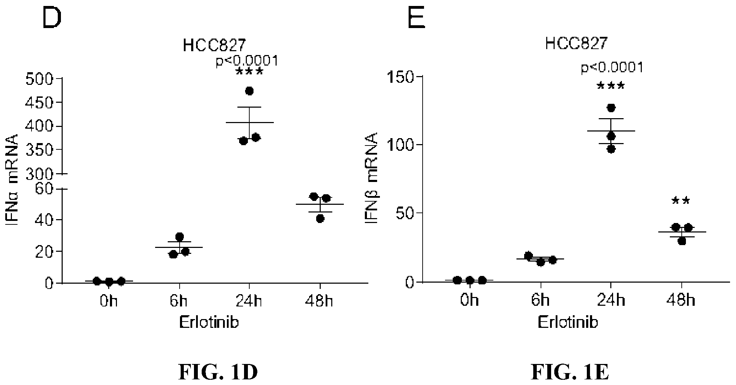

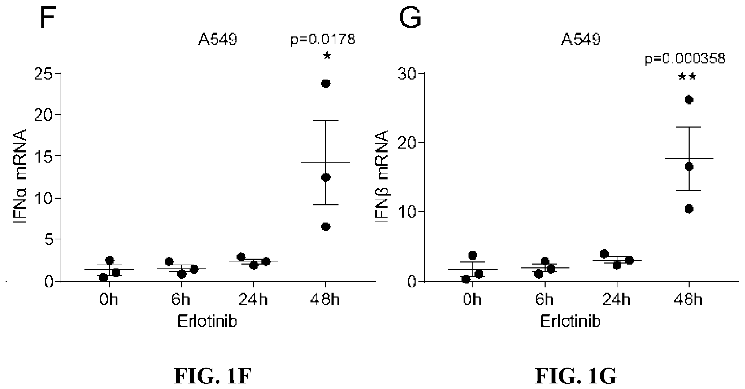

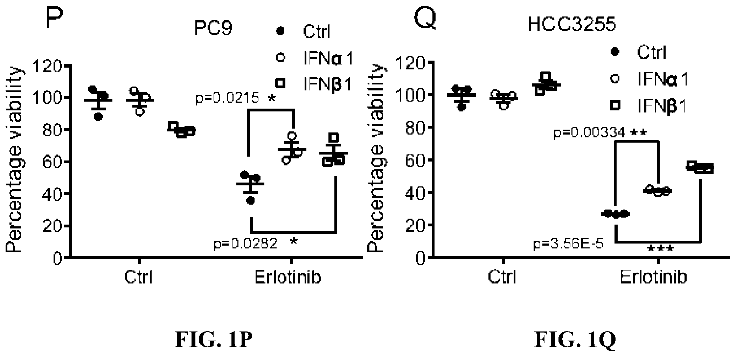

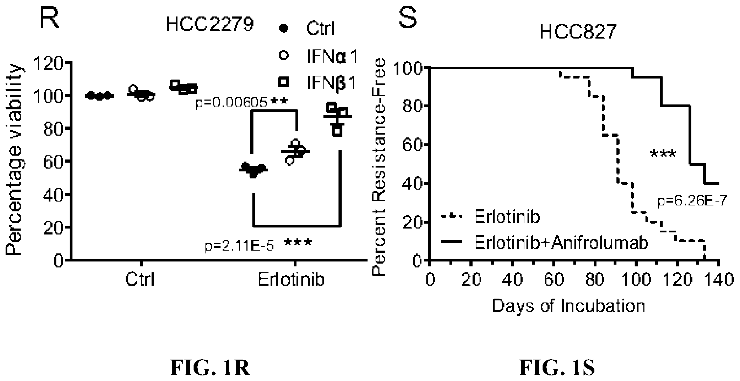

**FIG. 1A-S** show representative data illustrating that EGFR inhibition upregulates IFN, which promotes resistance to EGFR inhibition in NSCLC. ***p<0.001, by log-rank test, n=20. Data represent mean ± S.E.M. n=3 biologically independent experiments. *: p<0.05, **: p<0.01, ***: p<0.001, by two-way ANOVA adjusted by Bonferroni's correction (J-K, M-R), or by one-way ANOVA with Dunnett's test (D-G), or by two-sided t-test (H-I). Western blots are cropped and representative of three independent repeated experiments with similar results. Uncropped images are in Source Data.

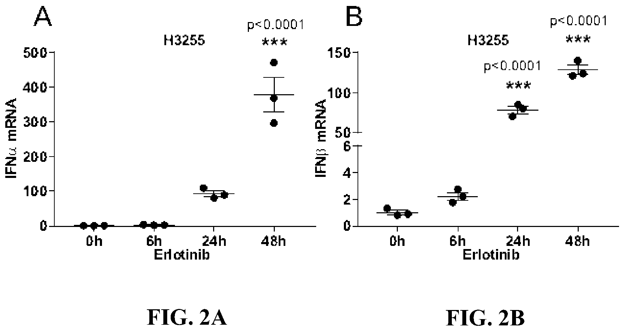

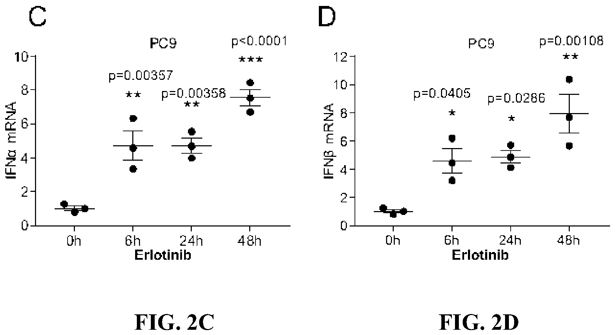

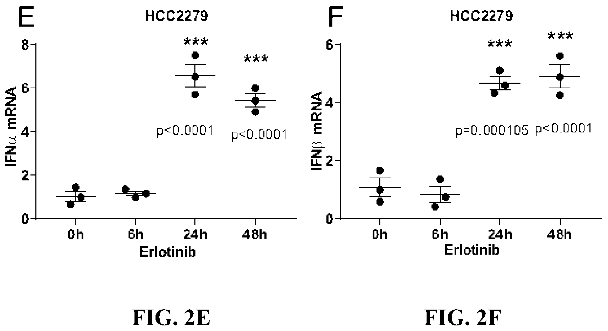

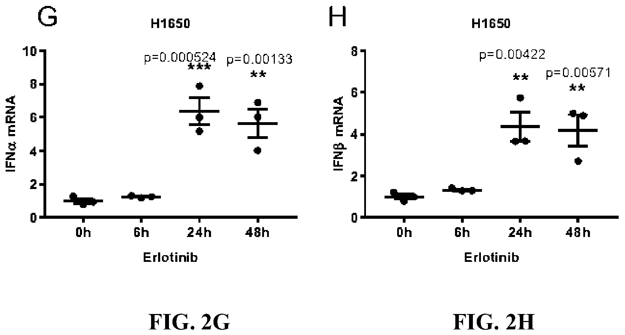

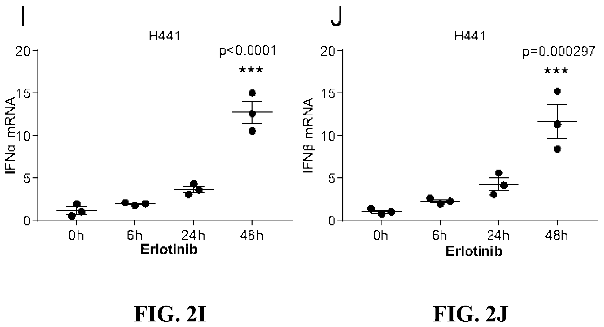

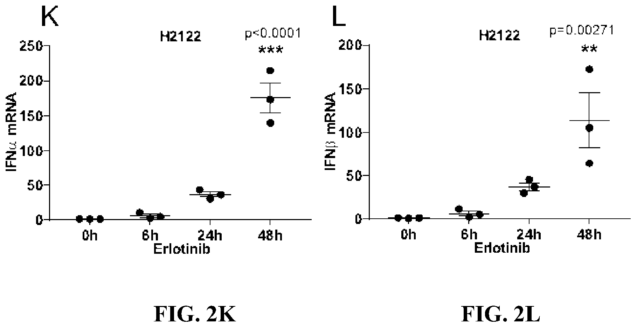

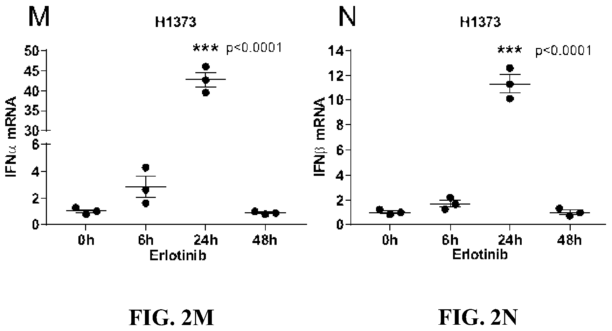

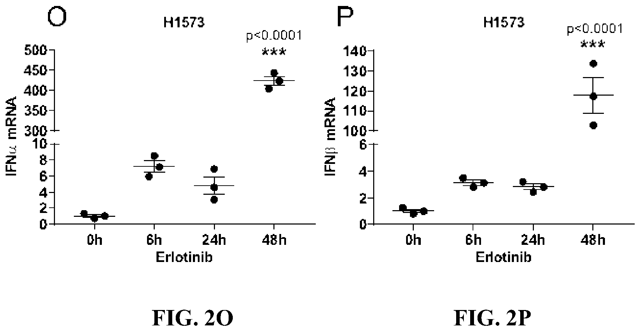

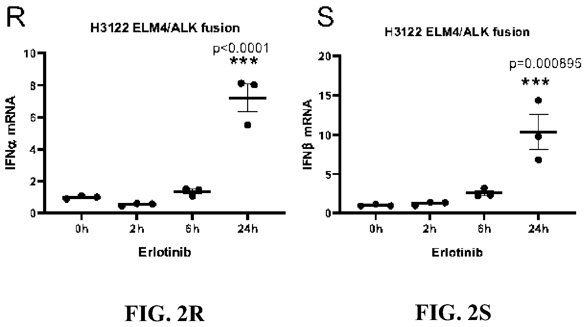

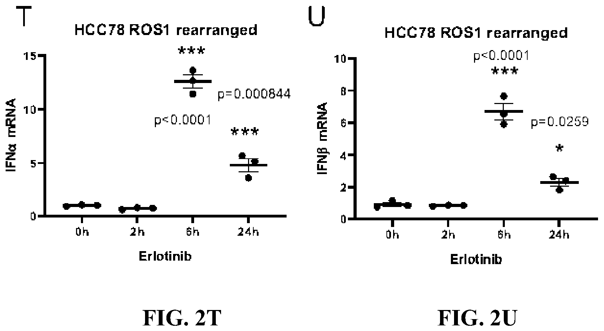

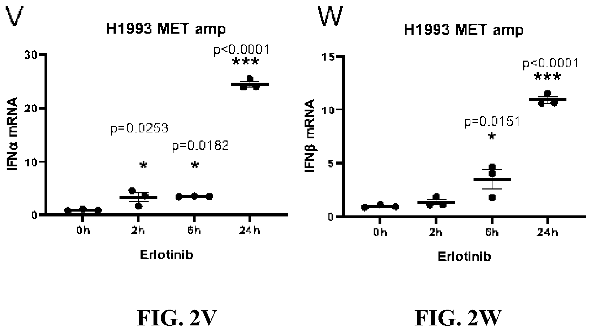

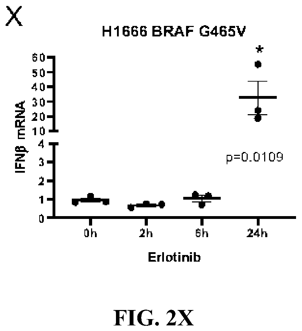

**FIG. 2A-X** show representative data illustrating that EGFR inhibition upregulates IFN mRNA levels in multiple NSCLC cell lines. β-Actin was used as the loading control. Data refers to mean ± S.E.M, n=3 independent repeated experiments. One-way ANOVA with Dunnett's test was used to determine adjusted p value for comparison between untreated and each treated sample. *: p<0.05, **: p<0.01, ***: p<0.001. H1666 is reported to harbor IFNA1 homodeletion in COSMIC (Catalogue Of Somatic Mutations In Cancer)-v90 http://cancer.sanger.ac.uk/cosmic (Updated 5 September 2019), also in Data from a CPRIT (Cancer Prevention & Research Institute of Texas)-funded NGS (next generation sequencing) project by Dr. John Minna, UT Southwestern Medical Center, and Data from Dr. Adi Gazdar, UT Southwestem Medical Center. All other cell lines used in this research were searched on those databases above and confirmed to harbor neither IFNA1 nor IFNB1 homodeletion. Western blots are representative of 3 independent experiments with similar results. Cropped images are shown. Uncropped Western blot images are shown in Source Data

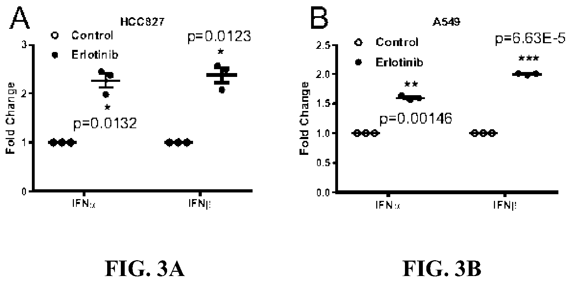

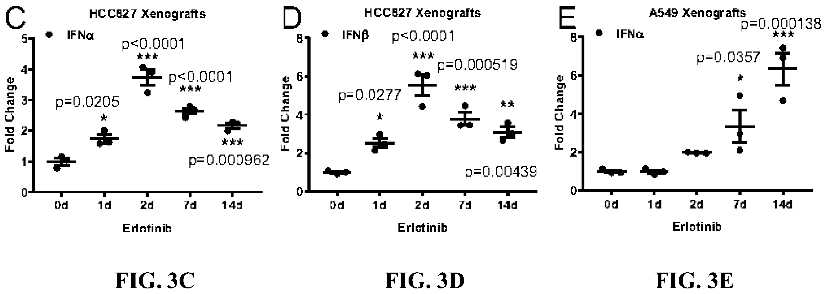

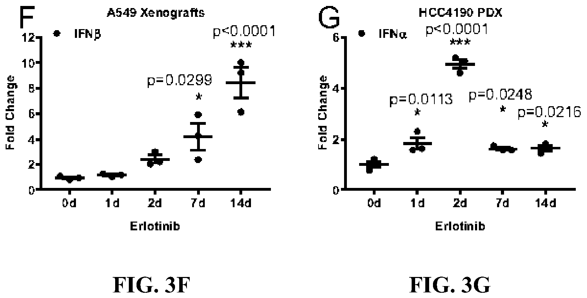

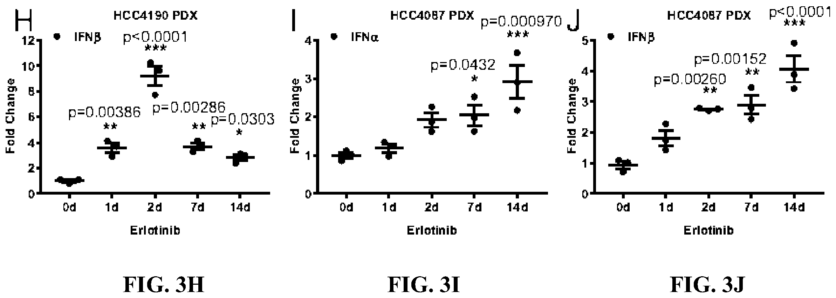

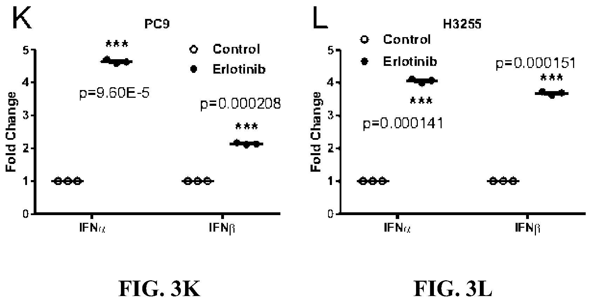

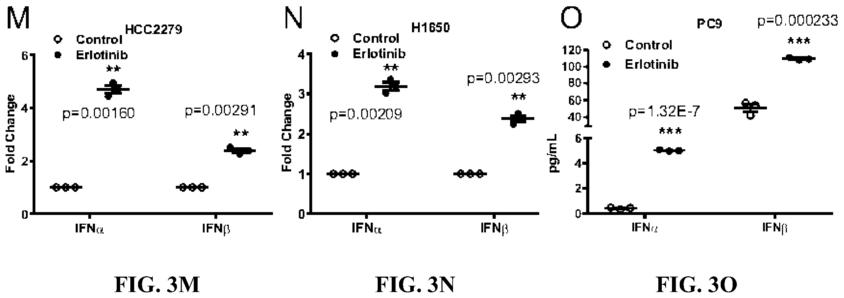

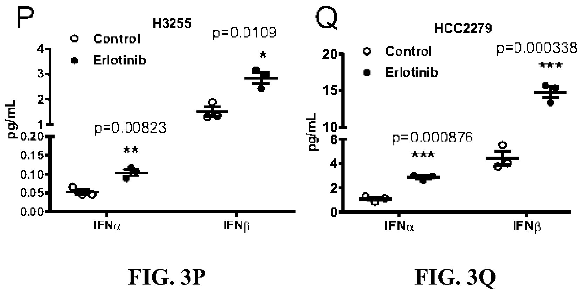

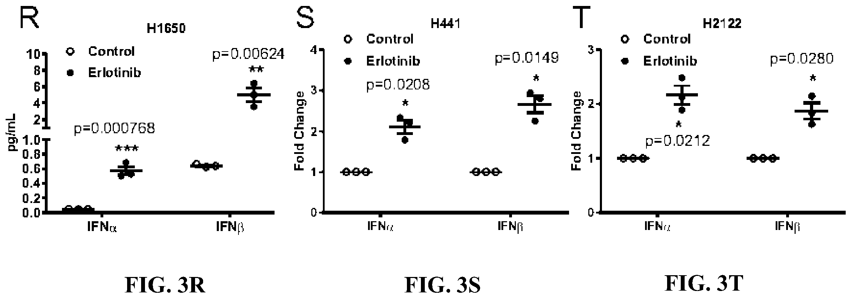

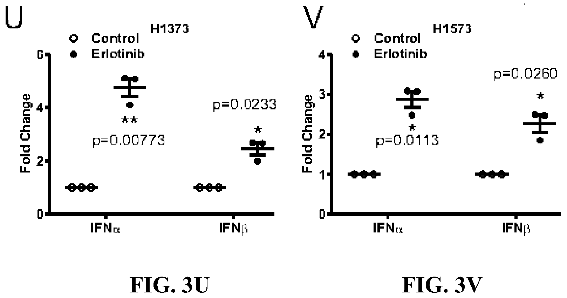

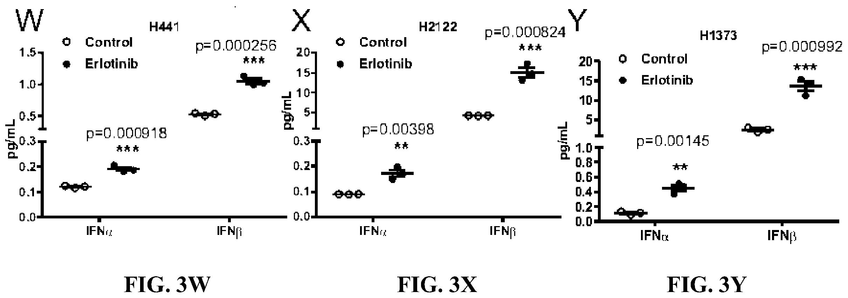

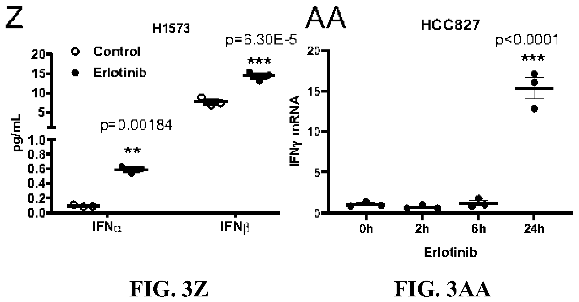

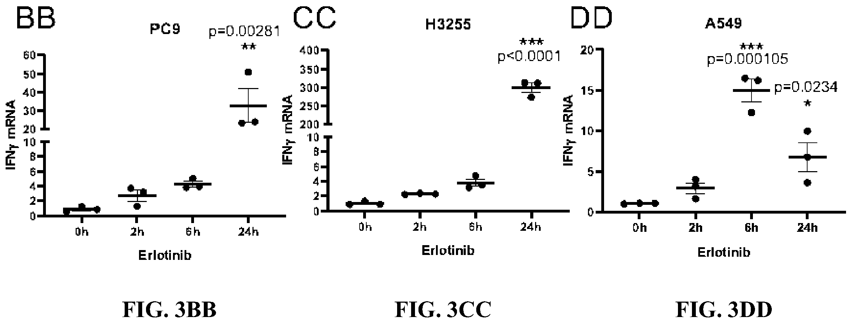

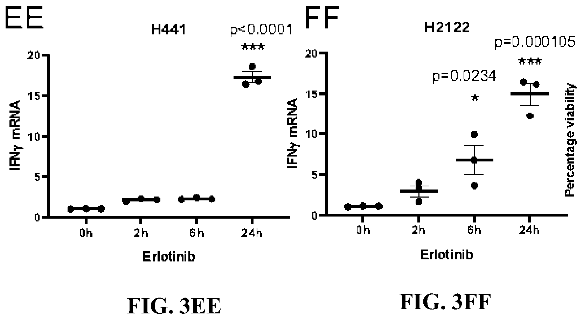

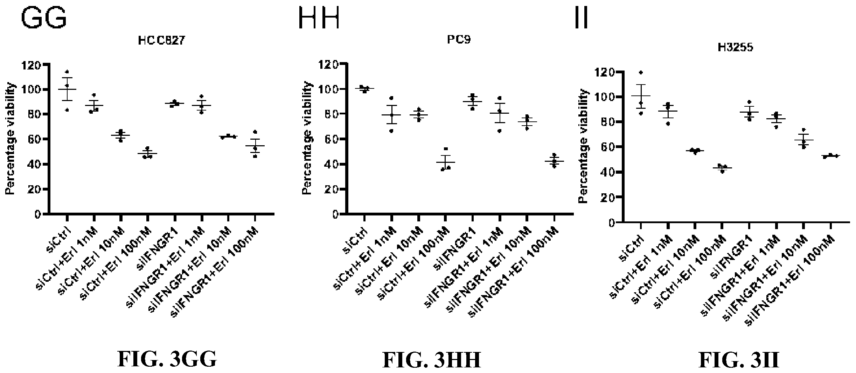

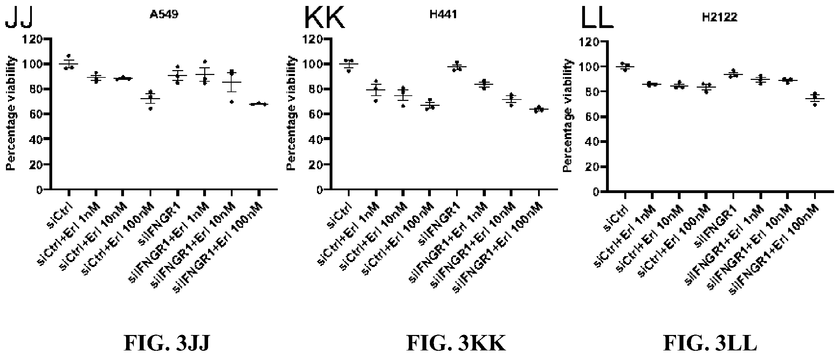

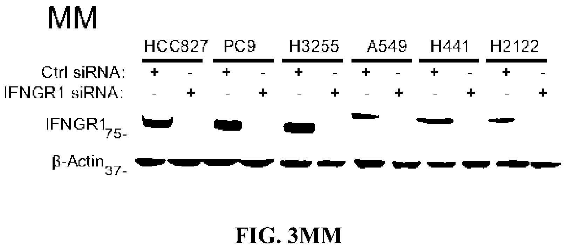

**FIG. 3A****-MM** show representative data illustrating the EGFR inhibition upregulates IFNs in multiple NSCLC cell lines and *in vivo.* Data refers to mean ± S.E.M, n=3 independent repeated experiments. ELISA was analyzed by two-sided t-test, and one-way ANOVA with Dunnett's test for animal tumors. In qPCR, one-way ANOVA with Dunnett's test was used to determine adjusted p value for comparison between untreated and each treated sample. In AlamarBlue assay, two-way ANOVA adjusted by Bonferroni's correction was used. #: p>0.05, *: p<0.05, **: p<0.01, ***: p<0.001. Western blots are representative of 3 independent experiments with similar results. Cropped images are shown. Uncropped Western blot images are shown in Source Data.

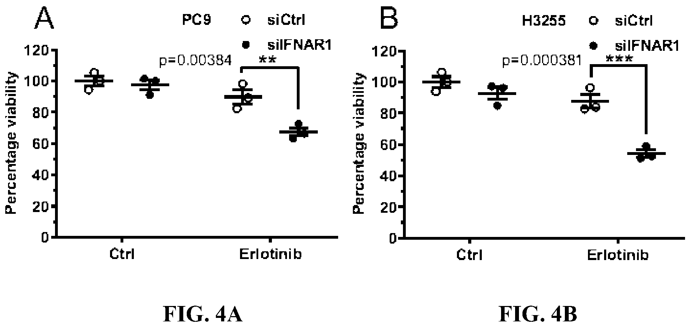

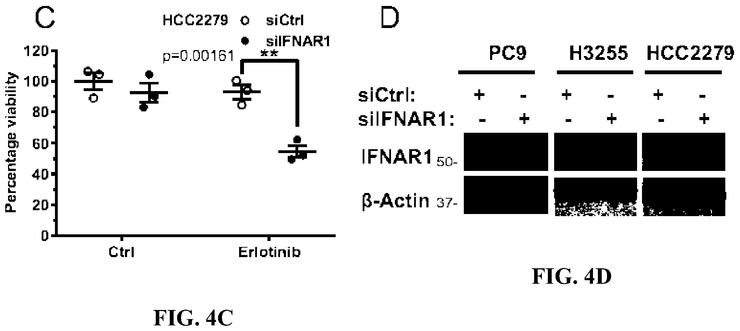

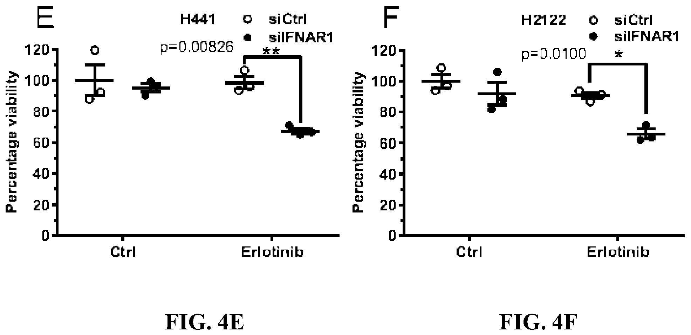

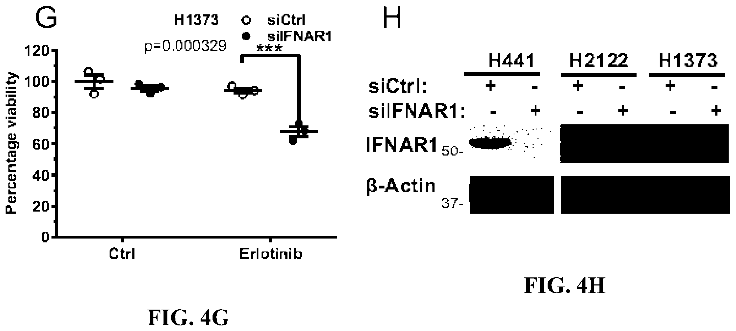

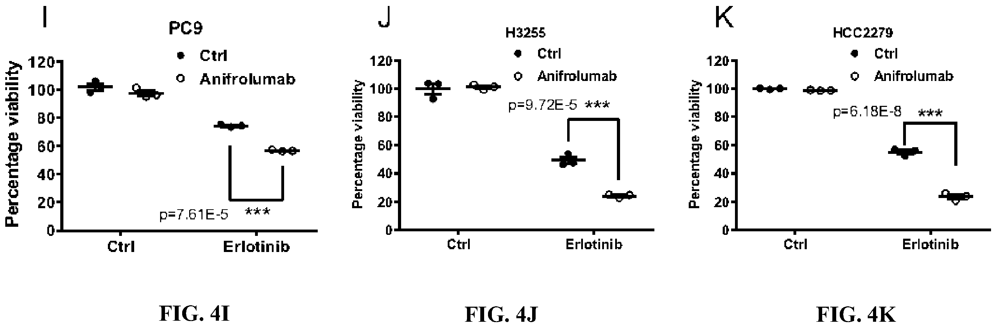

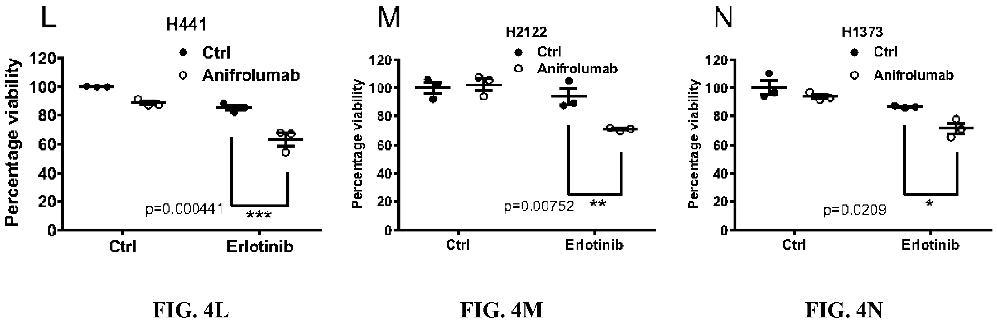

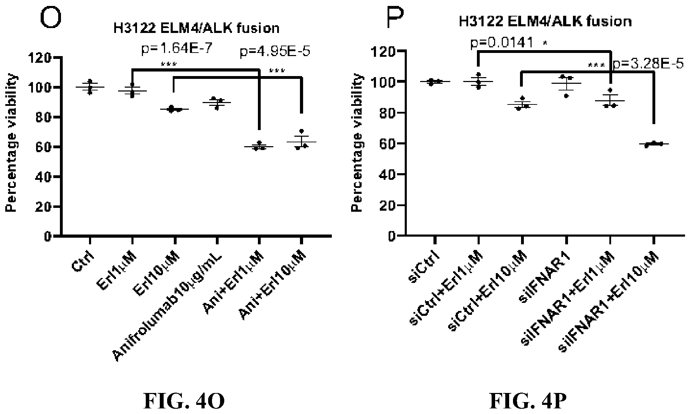

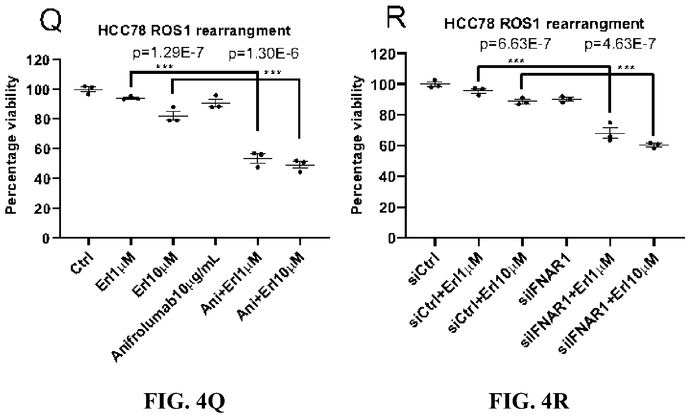

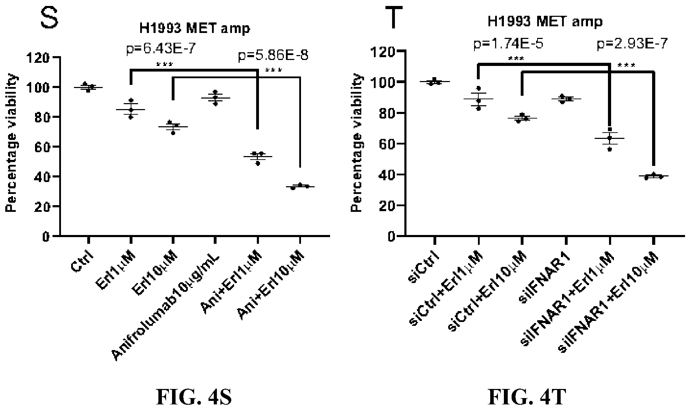

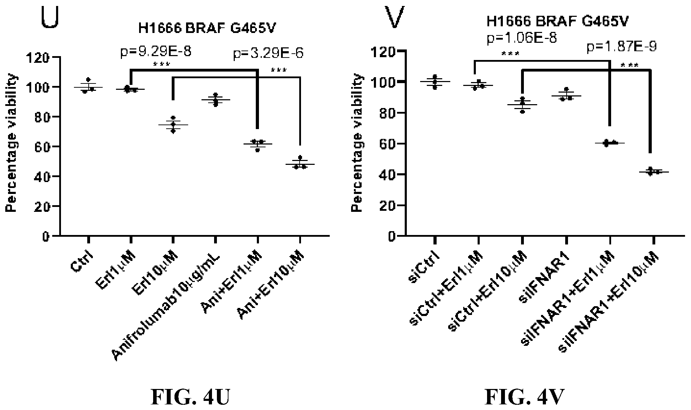

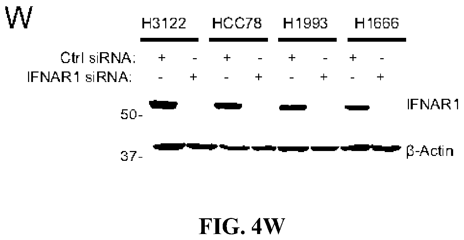

**FIG. 4A-W** show representative data illustrating that Type I IFNs promote resistance to EGFR inhibition in multiple NSCLC cell lines. Data represents mean ± S.E.M. of three independent repeated experiments. *: p<0.05, **: p<0.01, ***: p<0.001, by two-way ANOVA adjusted by Bonferroni's correction. Western blots are representative of 3 independent experiments with similar results. Cropped images are shown. Uncropped Western blot images are shown in Source Data.

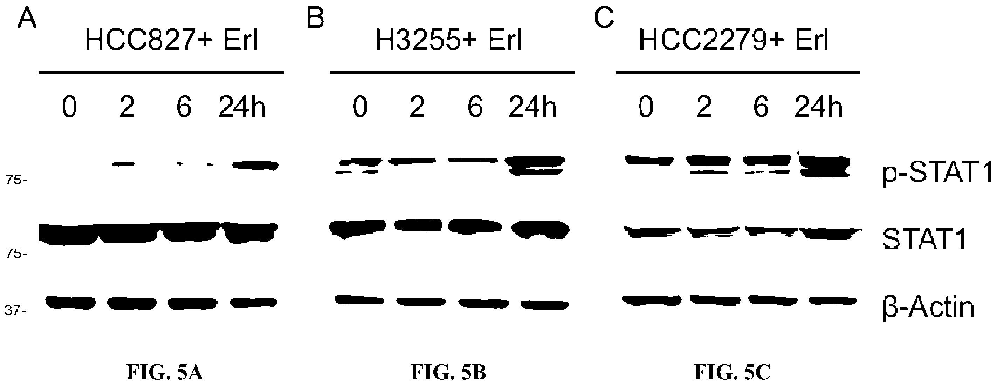

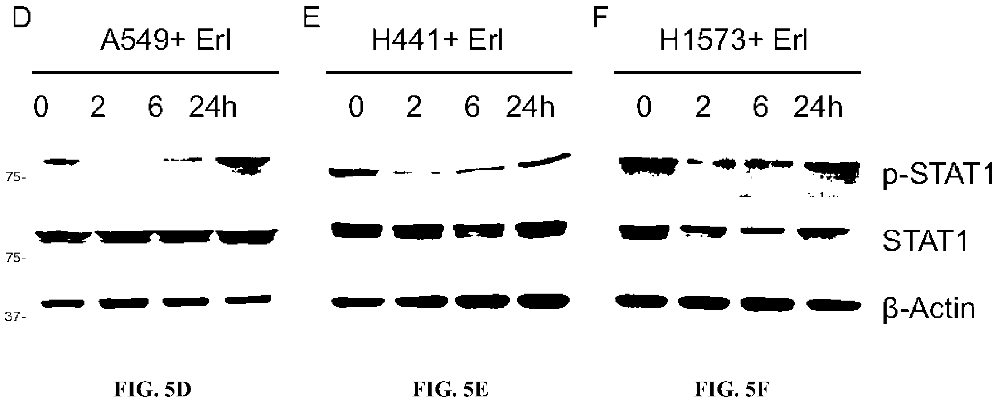

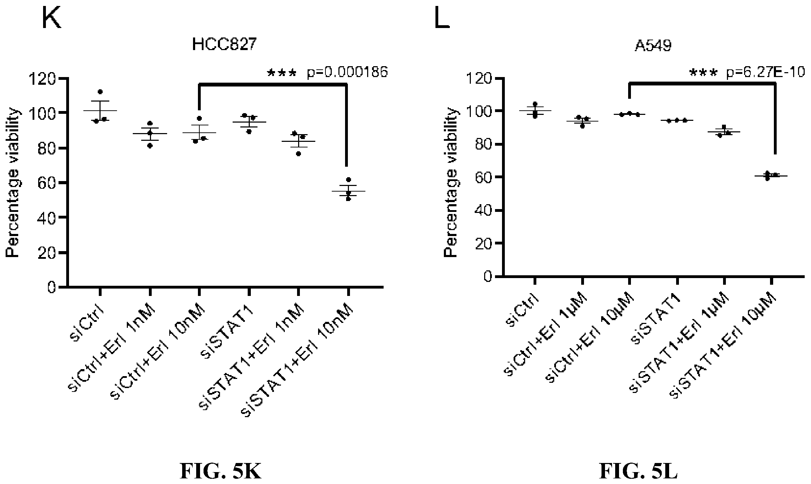

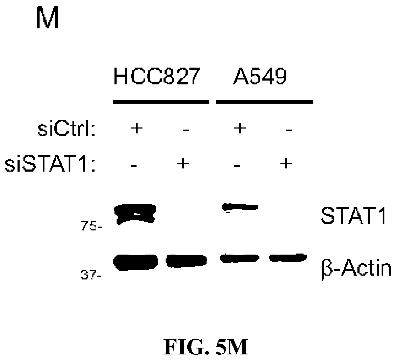

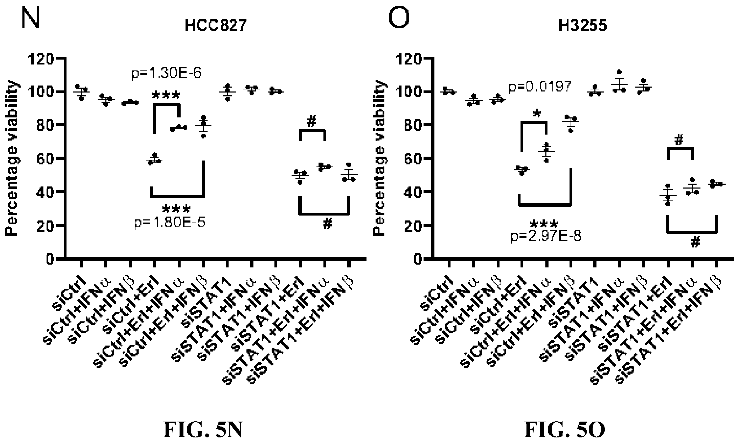

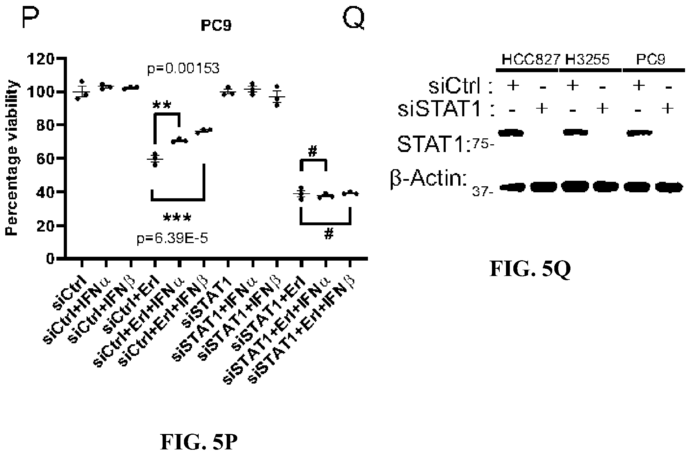

**FIG. 5A-Q** show representative data illustrating that STAT1 activation is involved in pro-survival effect of Type I IFNs in the context of EGFR inhibition. Data represent mean ± S.E.M. n=3 biologically independent experiments.#: p>0.05, *: p<0.05, **: p<0.01, ***: p<0.001, by two-way (K-L) or three-way (N-P) ANOVA adjusted by Bonferroni's correction. Western blots are cropped and representative of three independent repeated experiments with similar results. Uncropped Westem blots are shown in Source Data.

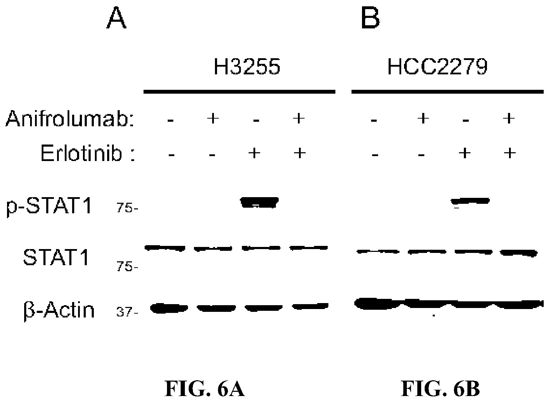

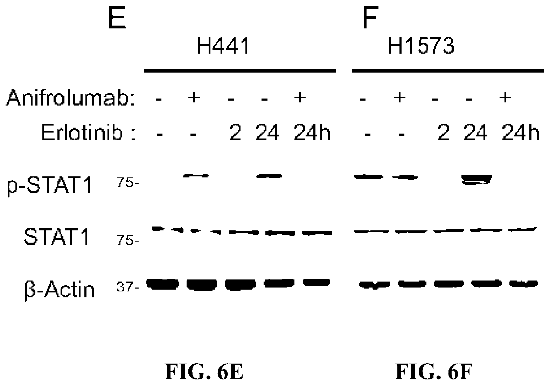

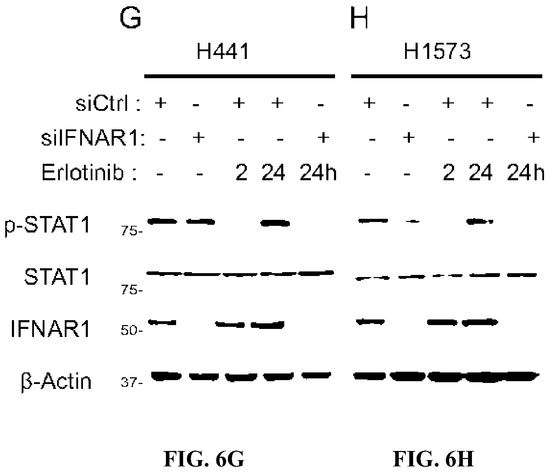

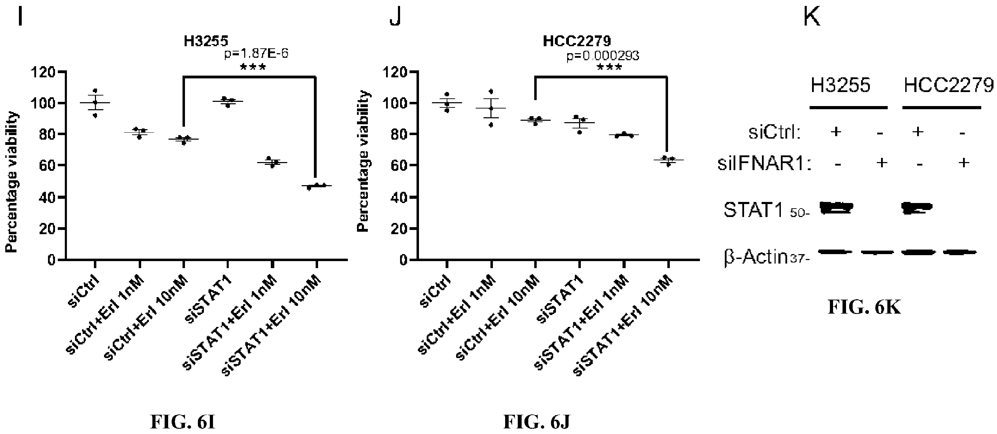

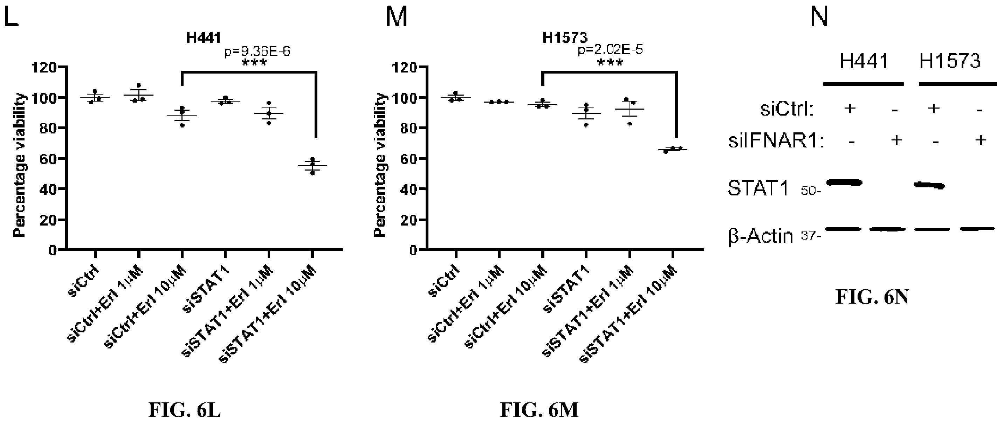

**FIG. 6A-N** show additional representative data illustrating that STAT1 activation is involved in pro-survival effect of Type I IFNs in the context of EGFR inhibition. Data represent mean ± S.E.M. n=3 independent repeated experiments. #: p>0.05, *:p<0.05, **: p<0.01, ***: p<0.001, by two-way ANOVA adjusted by Bonferroni's correction. Western blots are representative of 3 independent experiments with similar results. Cropped images are shown. Uncropped Western blot images are shown in Source Data

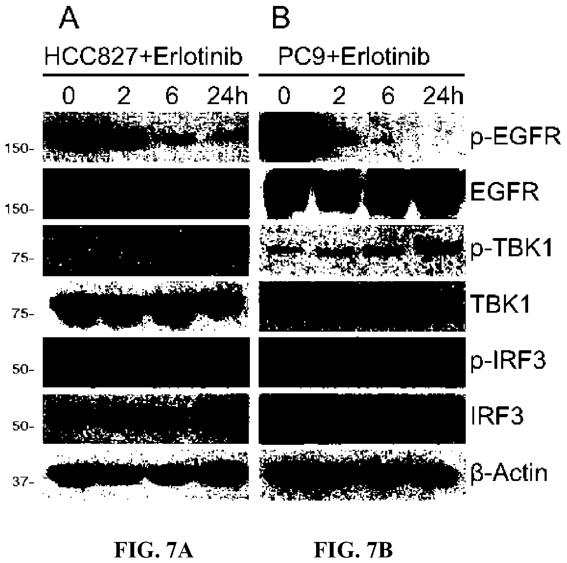

**FIG. 7A-M** show representative data illustrating that EGFR inhibition triggers a biologically significant TBK1-IRF3 pathway in EGFR mutant NSCLC. Data represent mean ± S.E.M. of three biologically independent experiments or animal tumor sizes. #: p>0.05, *: p<0.05, **: p<0.01, ***: p<0.001, by two-sided t-test for F-G, or by two-way ANOVA adjusted by Bonferroni's correction for the others. Western blots are cropped and representative of three independent repeated experiments with similar results. Uncropped Western blots are shown in Source Data.

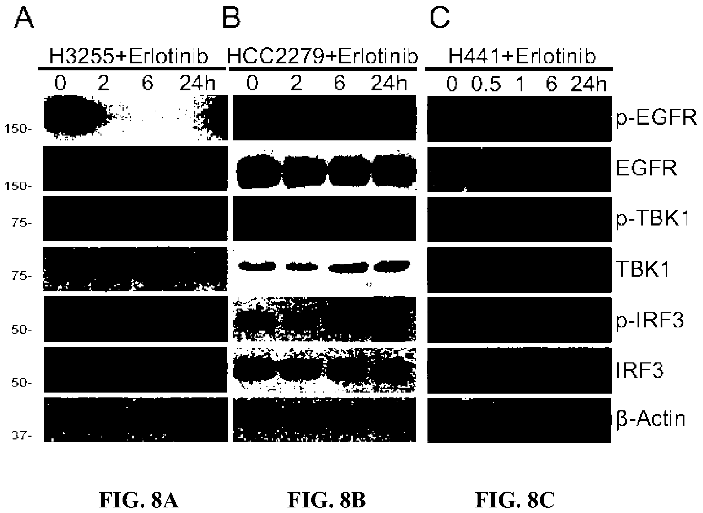

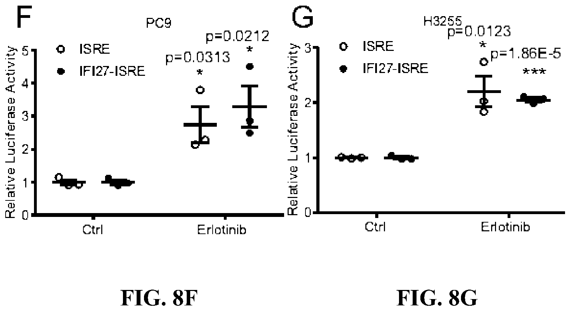

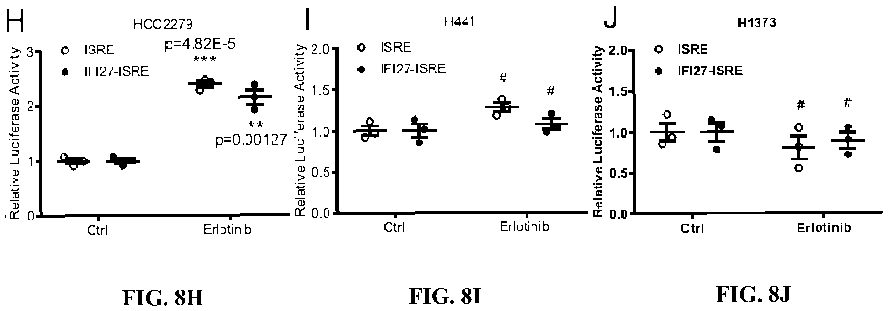

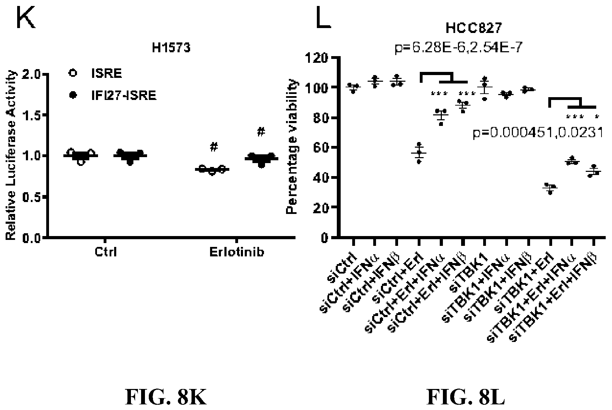

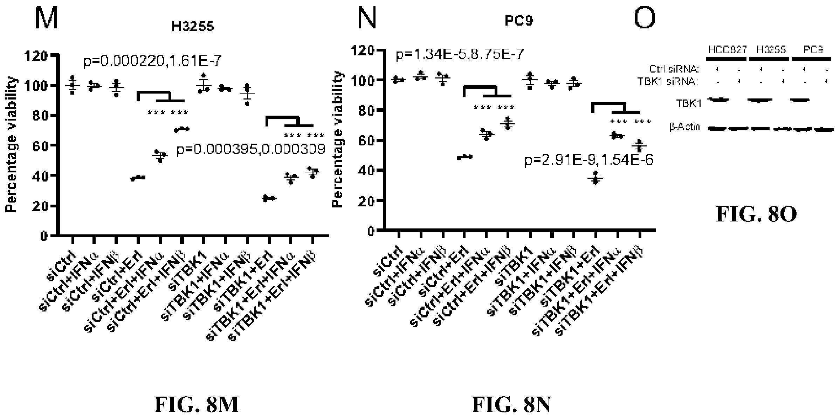

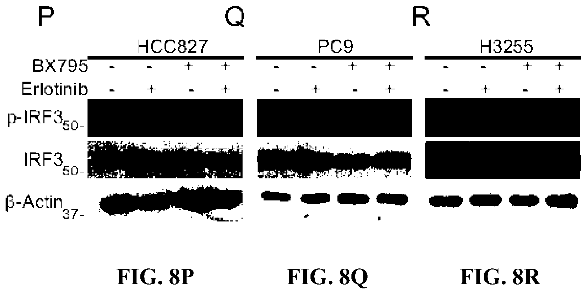

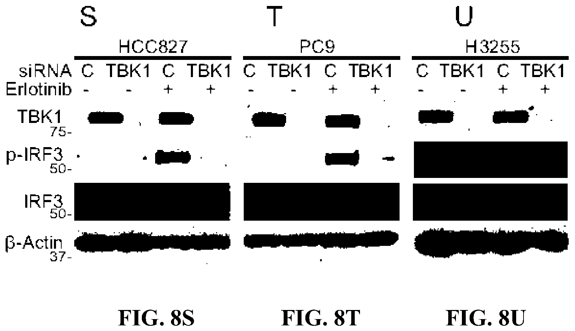

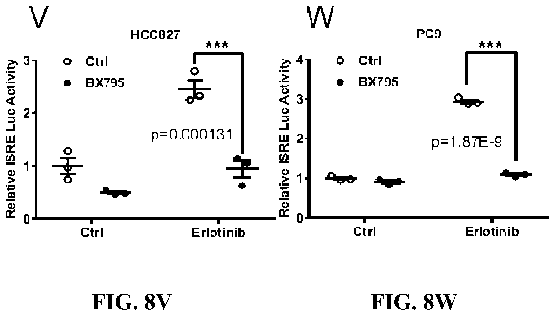

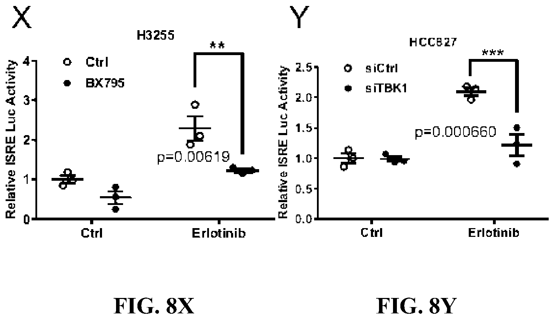

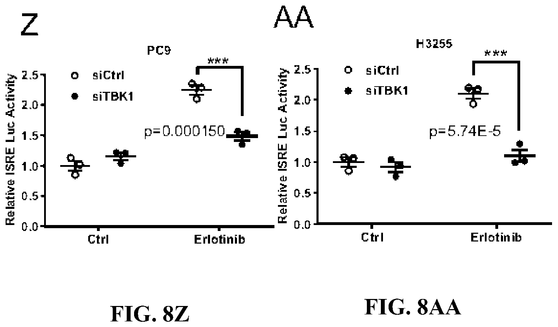

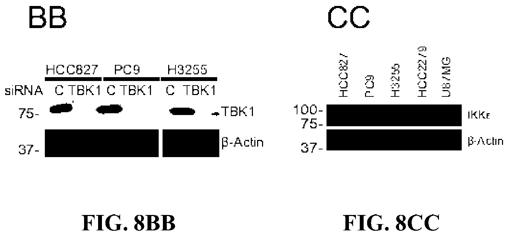

**FIG. 8A****-CC** show representative data illustrating representative data illustrating that EGFR inhibition activates TBK1-IRF3 axis in EGFR mutant but not in EGFR wt NSCLC and illustrating the lack of IKKε expression in lung cancer cell lines. Data represents mean ± S.E.M. of three independent biological replicates. #: p>0.05, *: p<0.05, **: p<0.01, ***: p<0.001, by two-sided t-test unadjusted for multiple comparisons in luciferase assay (F-K), three-way (L-N) and two-way ANOVA (V-AA) adjusted by Bonferroni's correction. Western blots are representative of three independent experiments with similar results. Cropped images are shown. Uncropped images are shown in Source Data.

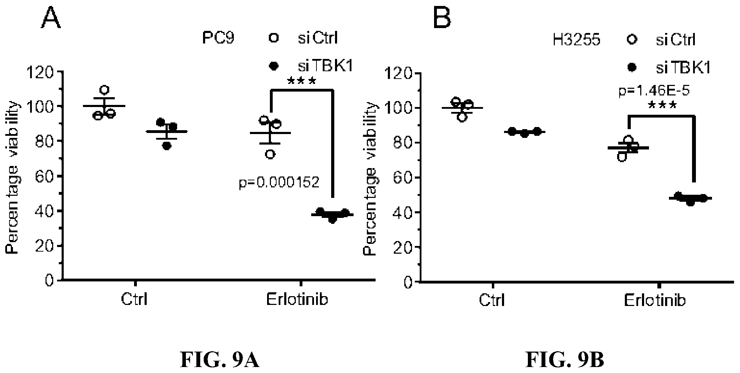

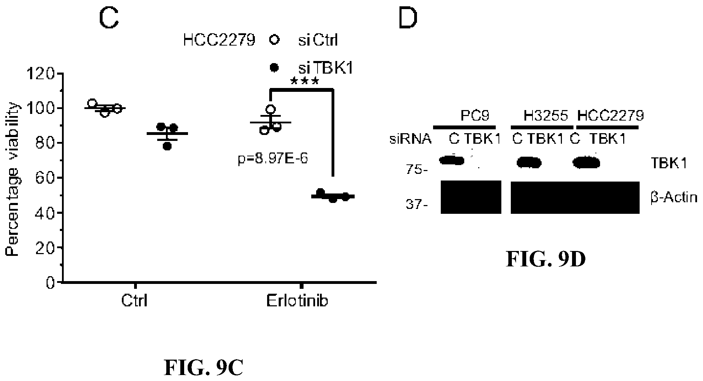

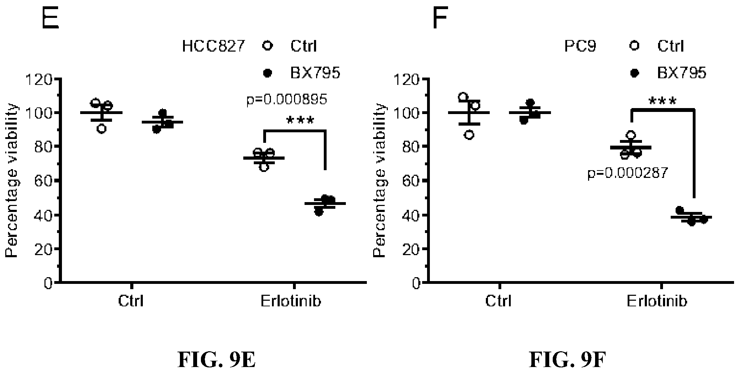

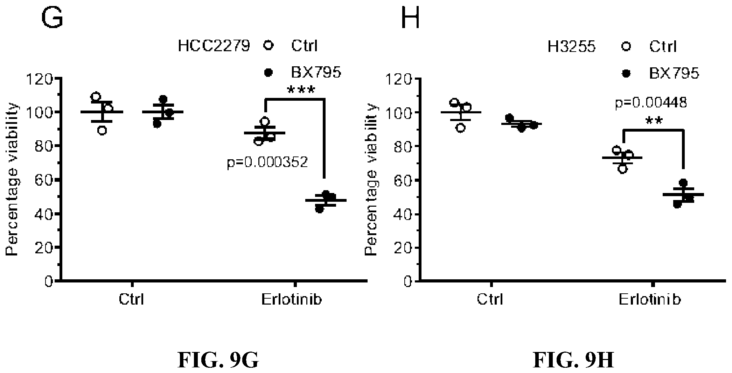

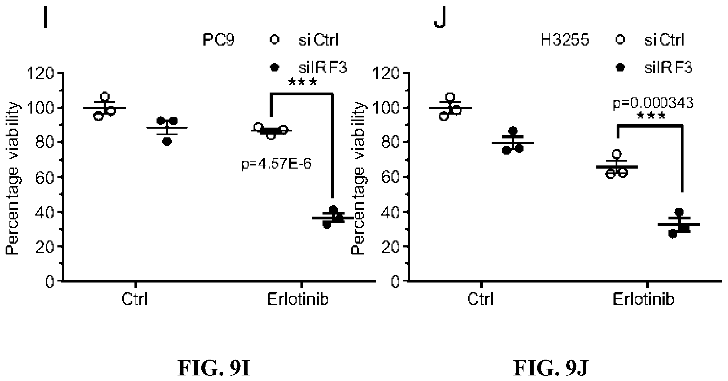

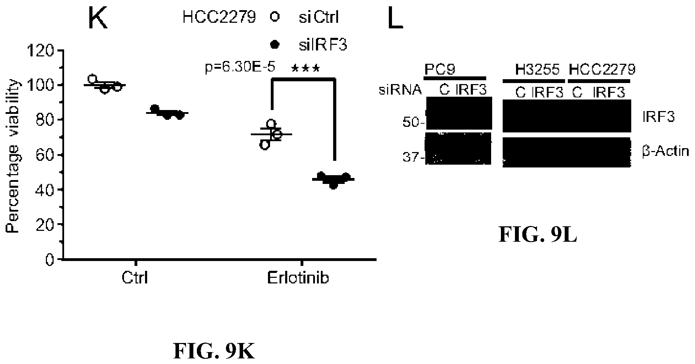

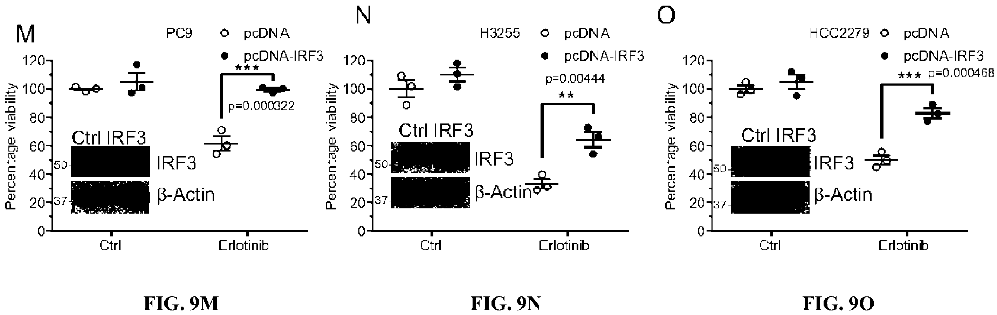

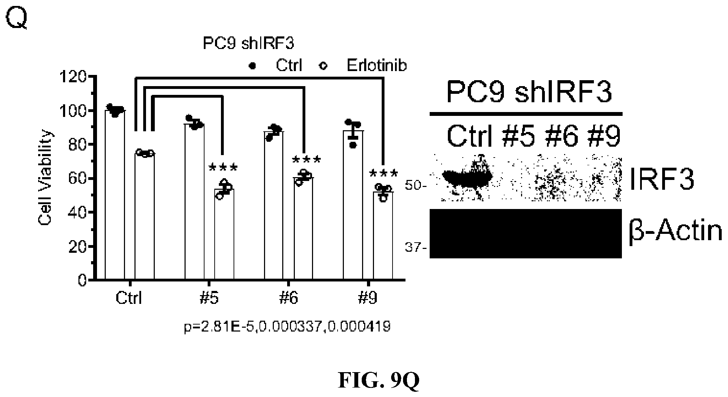

**FIG. 9A-R** show representative data illustrating the biological significance of EGFR induced TBK1/IRF3 activation in EGFR mutant NSCLC. Data refers to mean ± S.E.M of three independent biological replicates or tumor sizes. *: p<0.05, **: p<0.01, ***: p<0.001, by two-way ANOVA adjusted by Bonferroni's correction. Western blots are representative of 3 independent experiments with similar results. Cropped images are shown. Uncropped Western blot images are shown in Source Data.

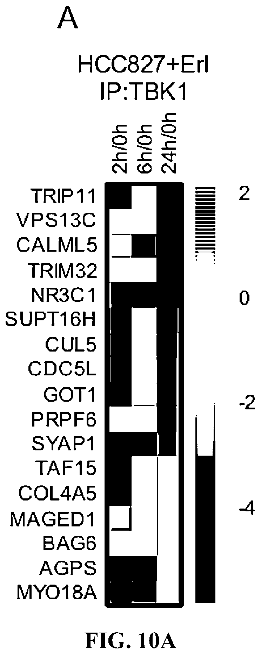

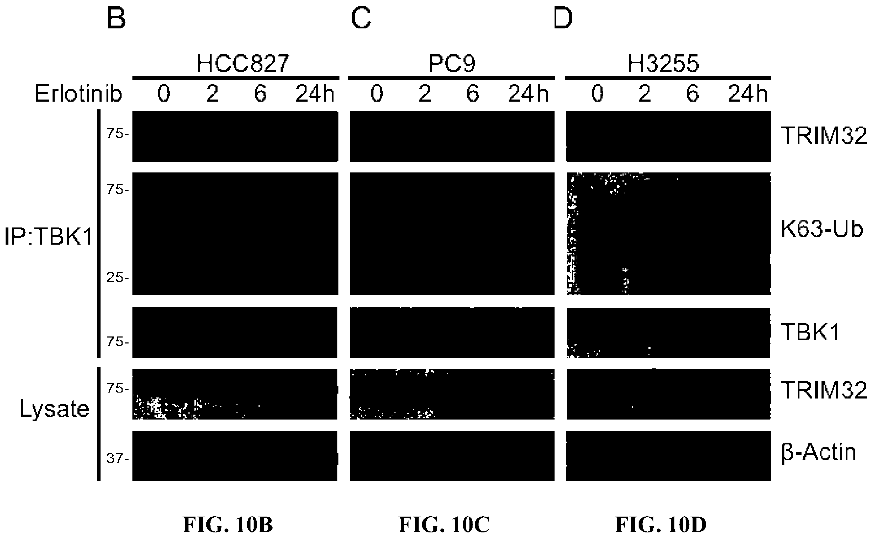

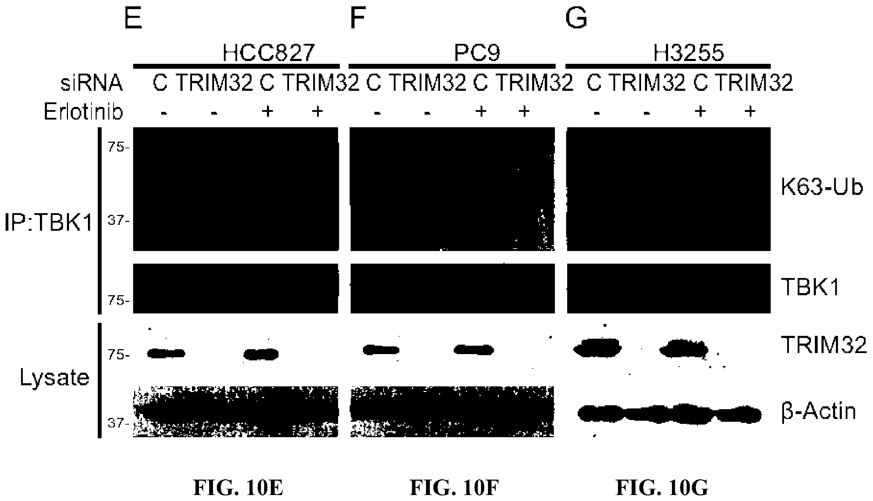

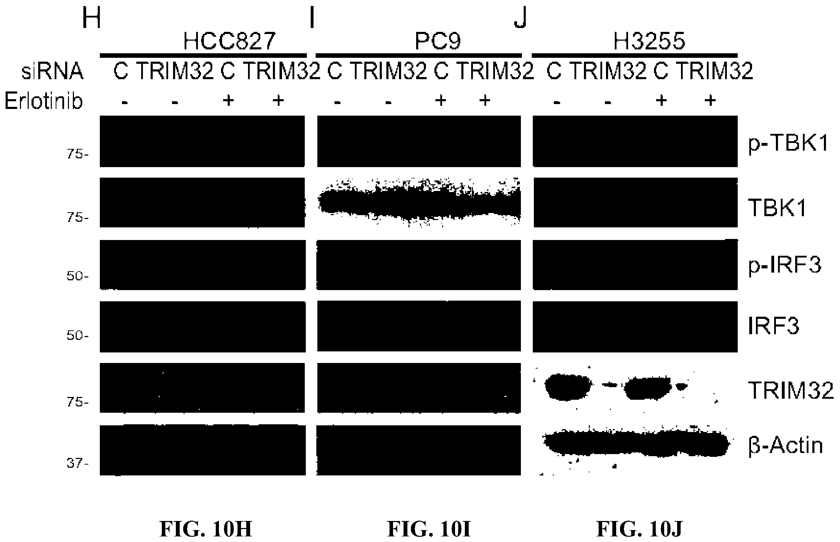

**FIG. 10A-J** show representative data illustrating that TRIM32 is required for EGFR inhibition induced activation of TBK1 and IRF3.

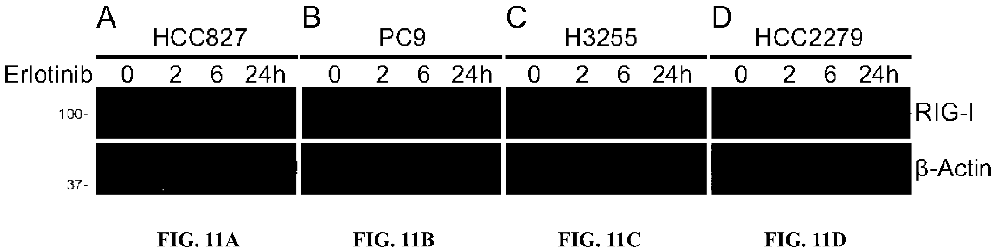

**FIG. 11A-N** show representative data illustrating that RIG-I is upregulated when EGFR is inhibited in EGFR mutant NSCLC lines. Data refers to mean ± S.E.M. of 3 biologically independent experiments. **: p<0.01, ***: p<0.001, by two-way ANOVA adjusted by Bonferroni's correction. Western blots are cropped and representative of three independent repeated experiments with similar results. Uncropped Western blots are shown in Source Data.

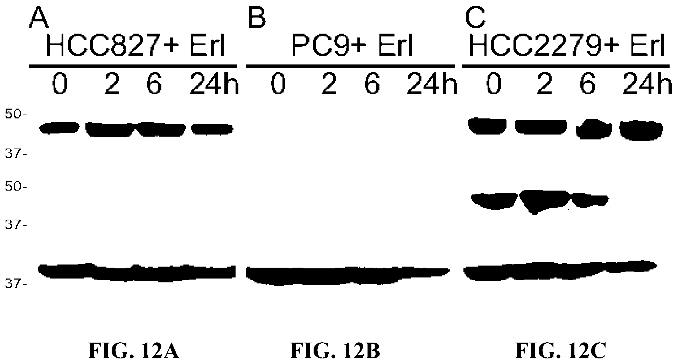

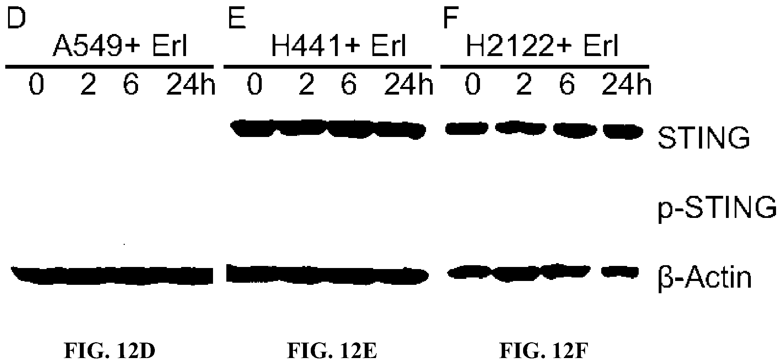

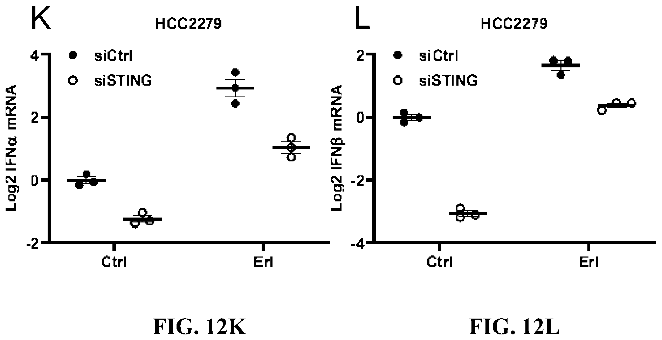

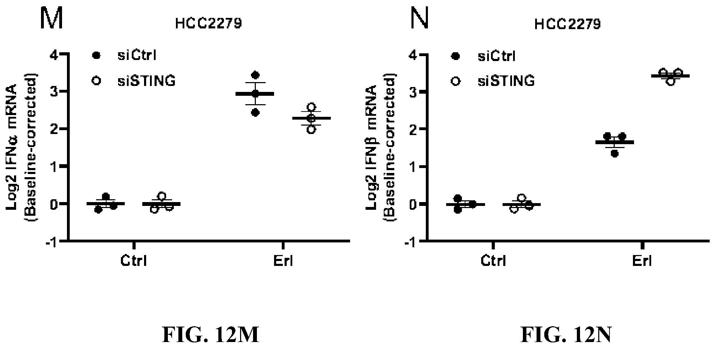

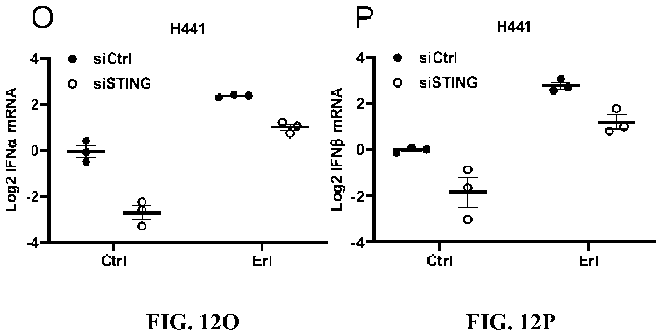

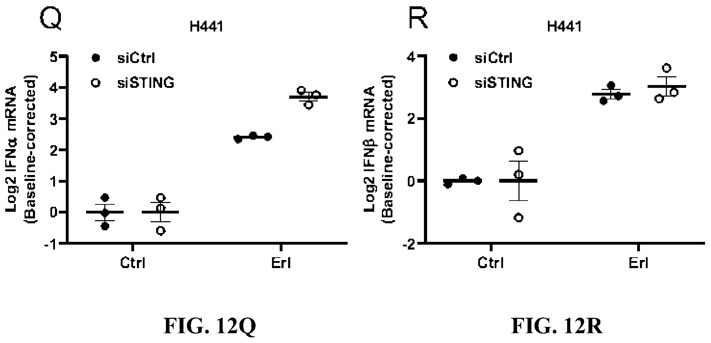

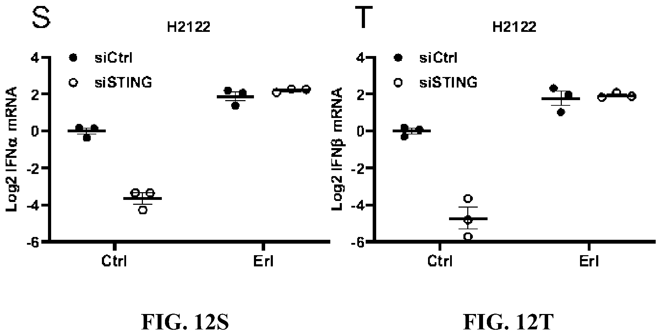

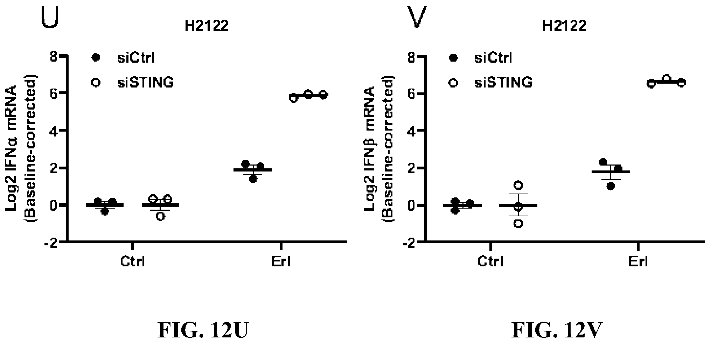

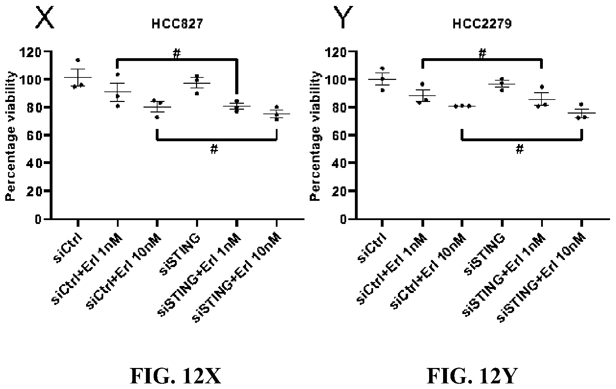

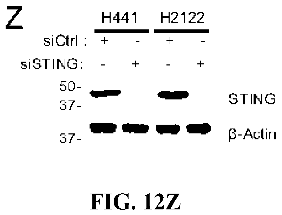

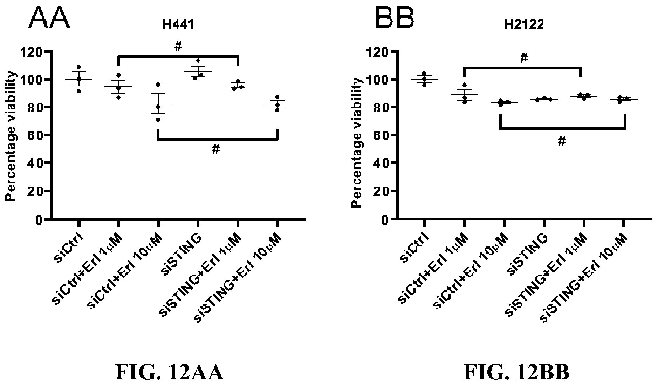

**FIG. 12A****-BB** show representative data illustrating that STING is not involved in response to EGFR inhibition. Data represents to mean ± S.E.M. of three independent biological replicates. #: p>0.05, *: p<0.05, **: p<0.01, ***: p<0.001, by two-way ANOVA adjusted by Bonferroni's correction. Western blots are representative of 3 independent experiments with similar results. Cropped images are shown. Uncropped Western blot images are shown in Source Data.

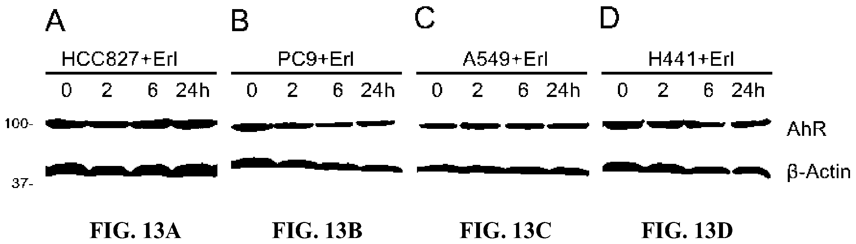

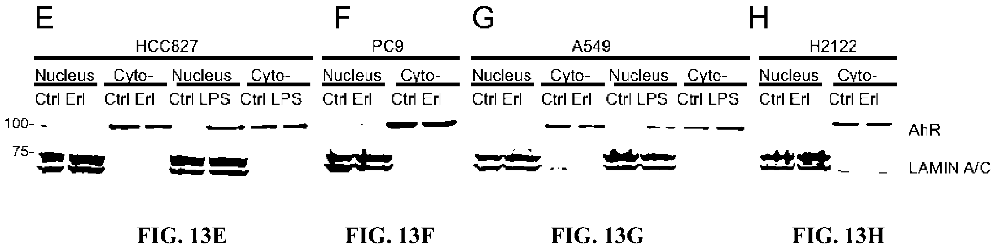

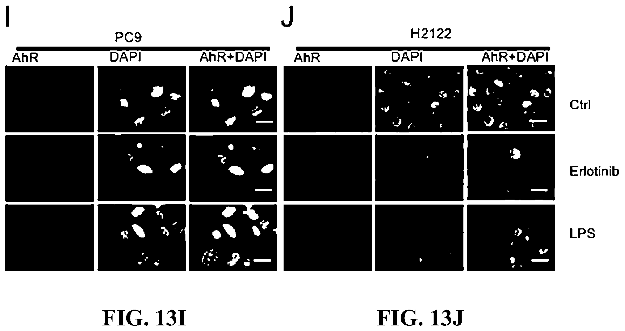

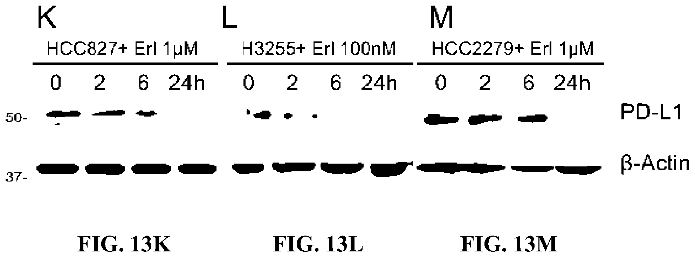

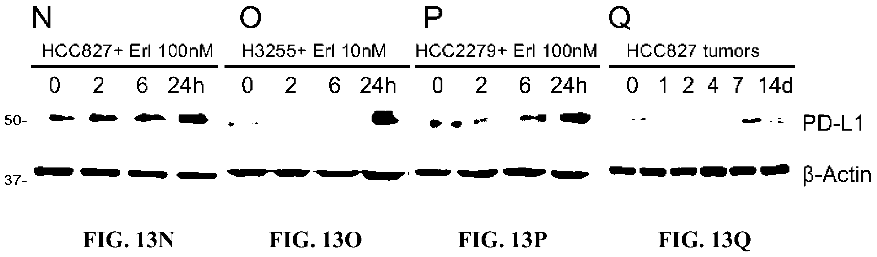

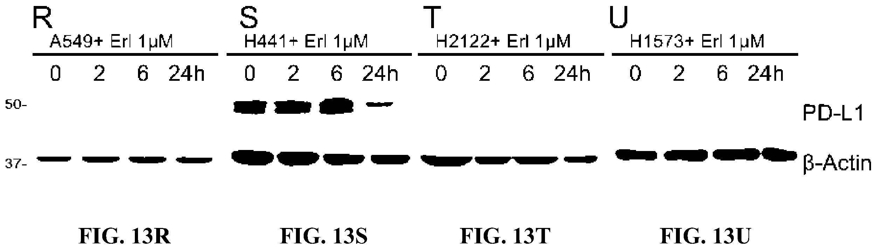

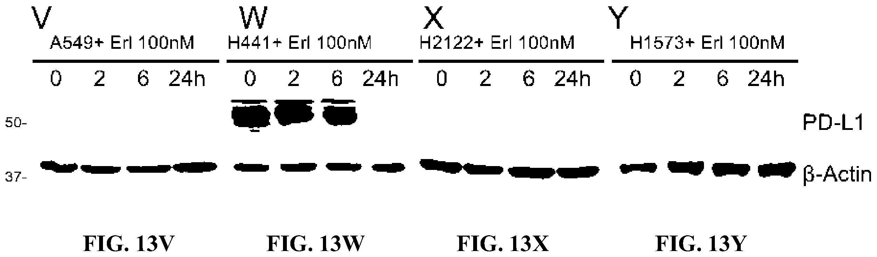

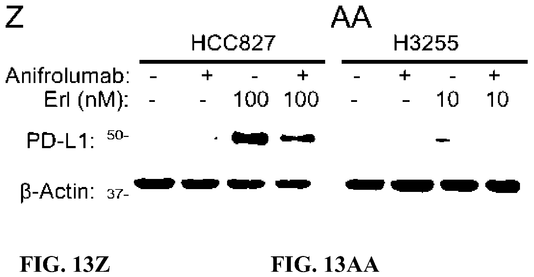

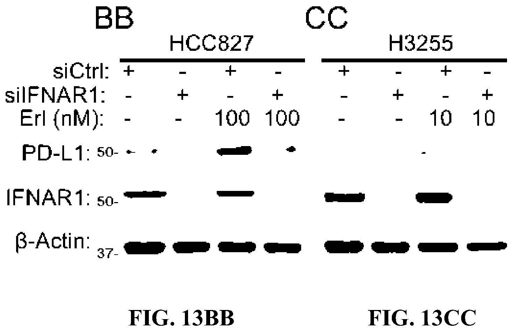

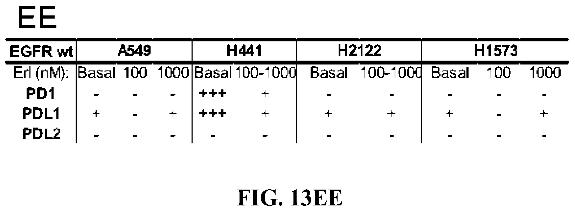

**FIG. 13A****-EE** show representative data illustrating that AhR is not activated in response to EGFR inhibition and illustrating regulation of PD-L1 by EGFR inhibition. Signal strength was represented by symbols. -: undetected, +: weak, ++: expressed, +++: strong signals. Western blot and Immunofluorescent staining images are representative of 3 independent experiments with similar results. Cropped images are shown. Uncropped Western blot images are shown in Source Data.

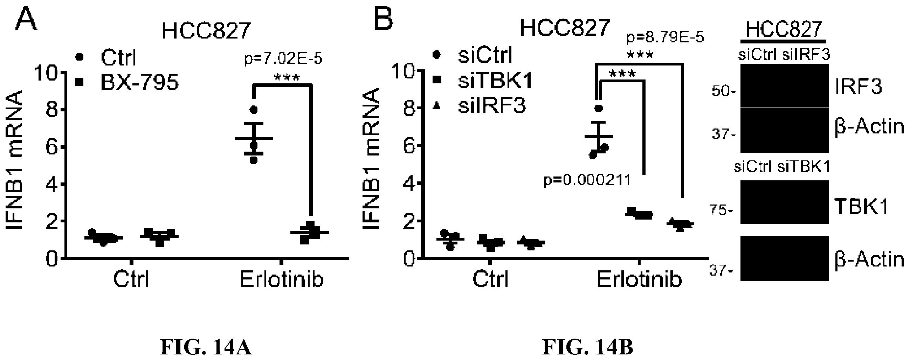

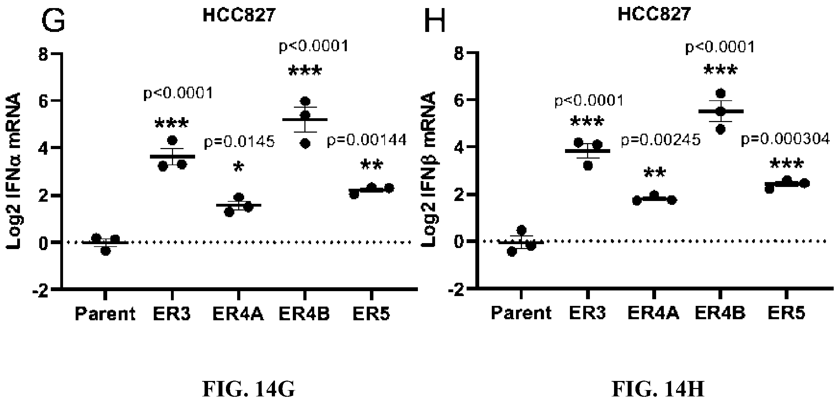

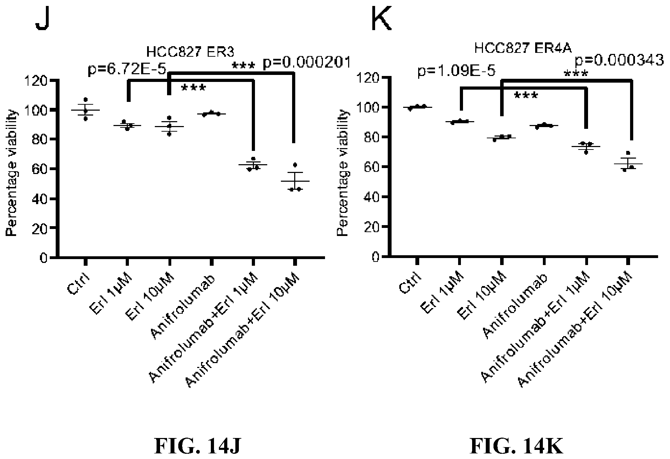

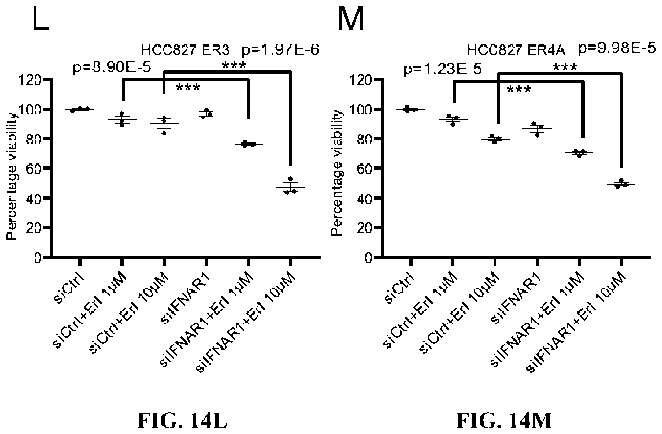

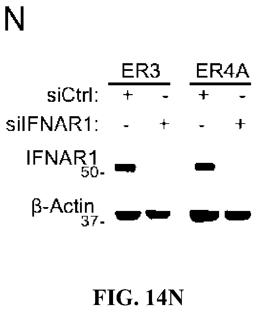

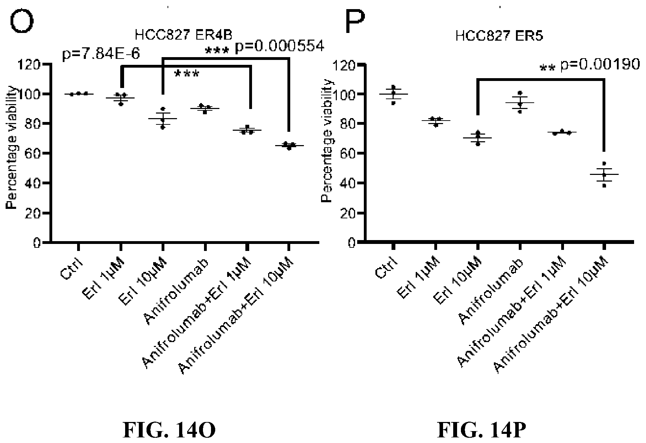

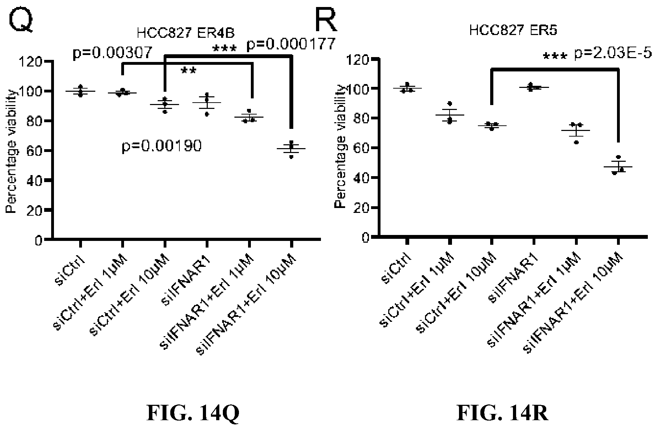

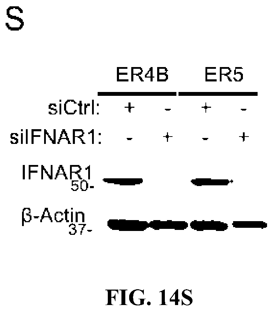

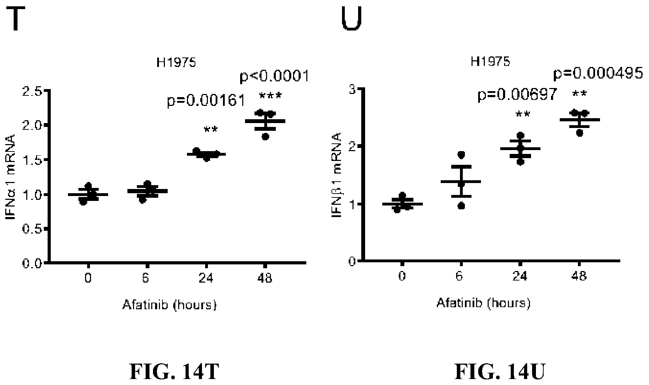

**FIG. 14A-X** show representative data illustrating that EGFRwt and EGFR mutant NSCLC upregulate Type I IFNs via distinct pathways; The role of IFN signaling in secondary resistance to EGFR inhibition. Data indicates mean ± S.E.M. of three biologically independent replicates #: p>0.05, *: p<0.05, **: p<0.01, ***: p<0.001, by one-way ANOVA adjusted by Dunnett's correction (G,H,T, and U) or two-way ANOVA adjusted by Bonferroni's correction (others). Western blots are cropped and representative of three independent repeated experiments with similar results. Uncropped Western blots are shown in Source Data.

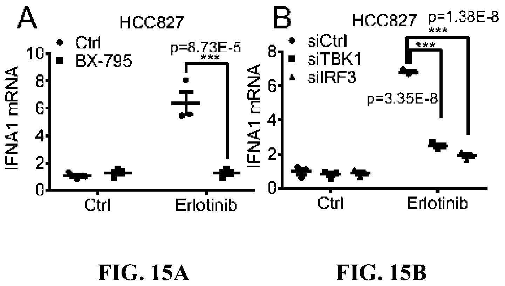

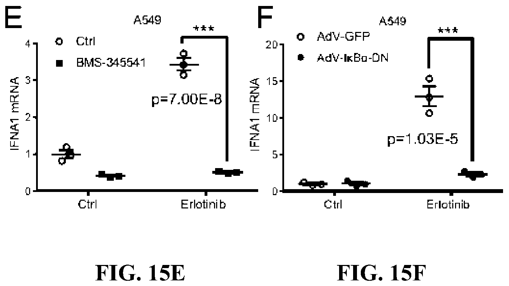

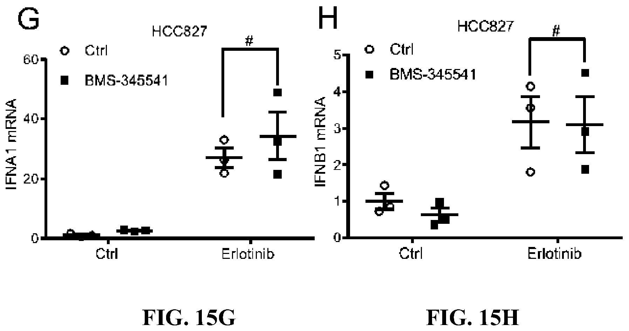

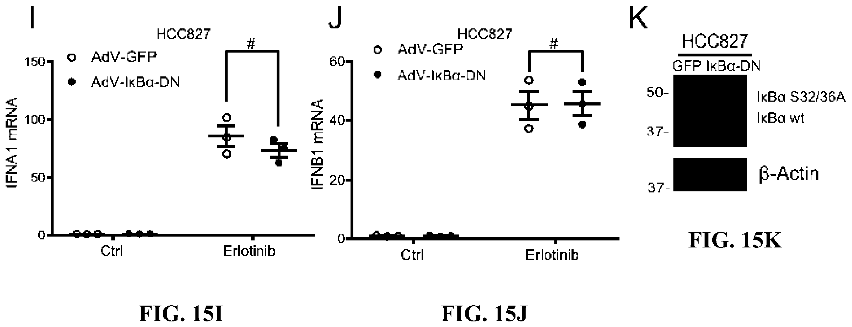

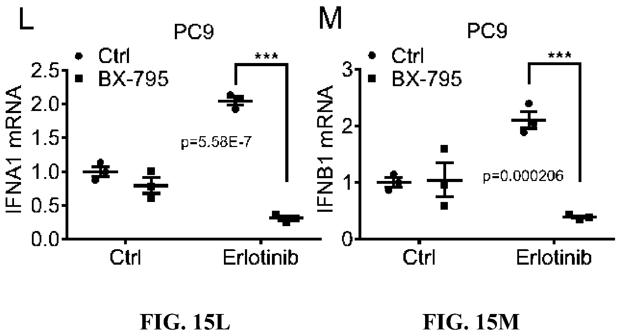

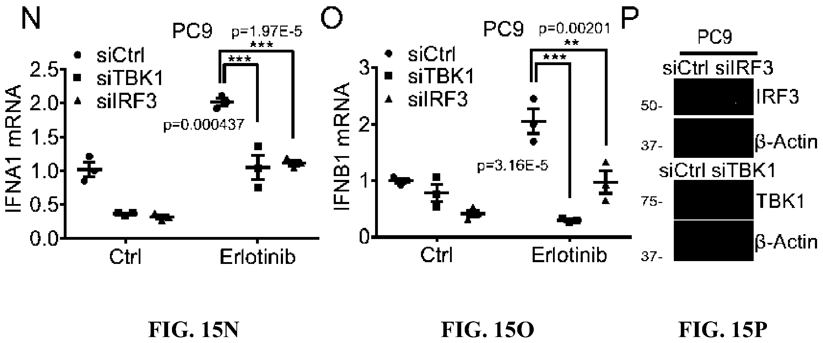

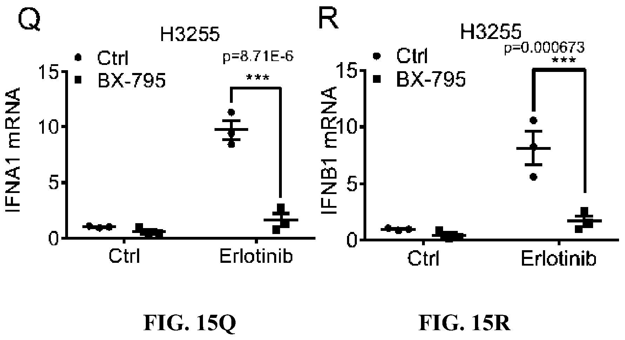

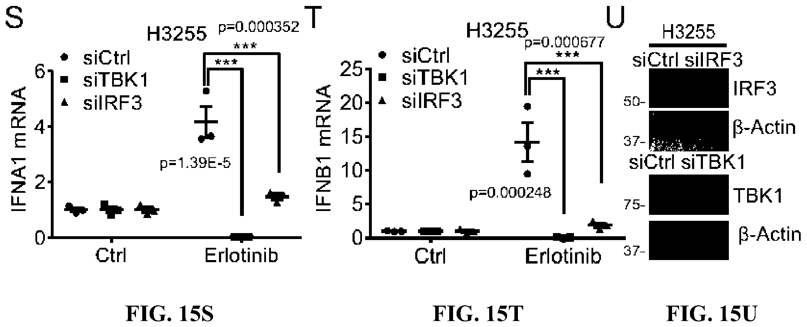

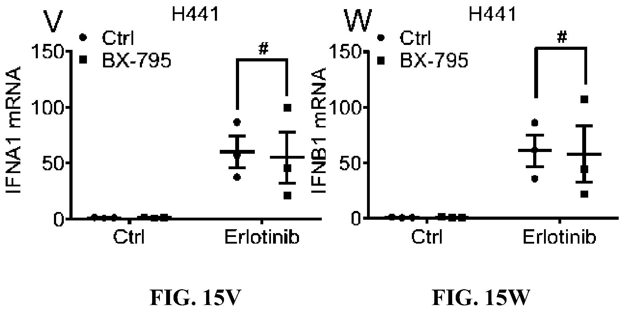

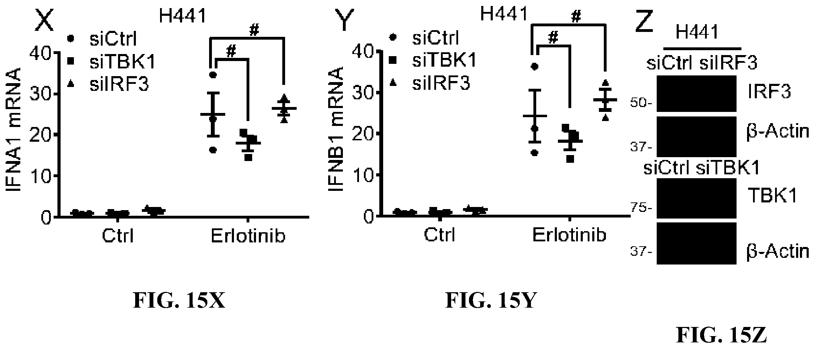

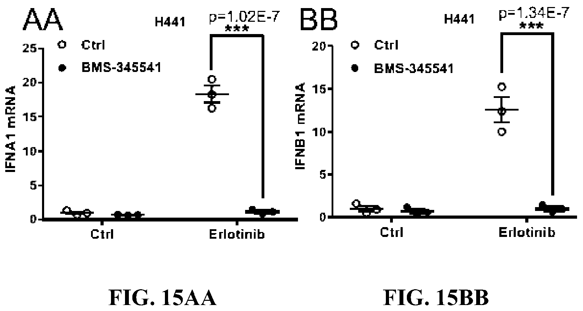

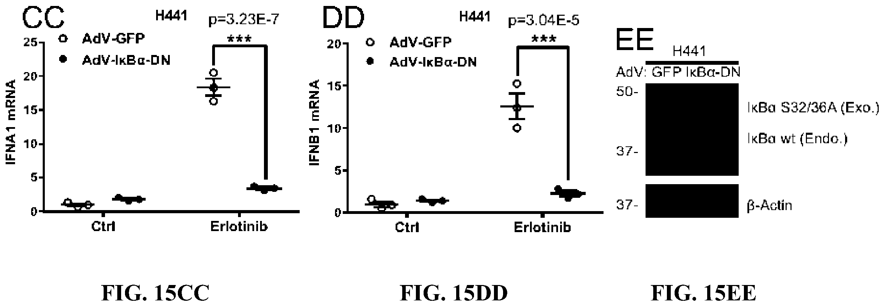

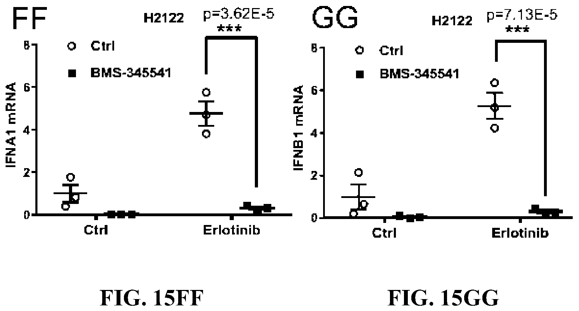

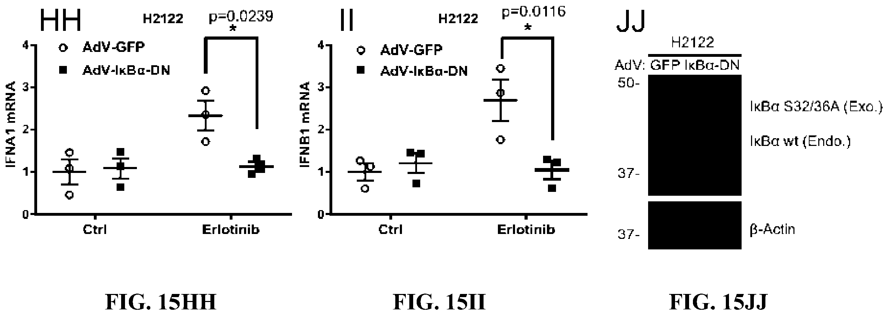

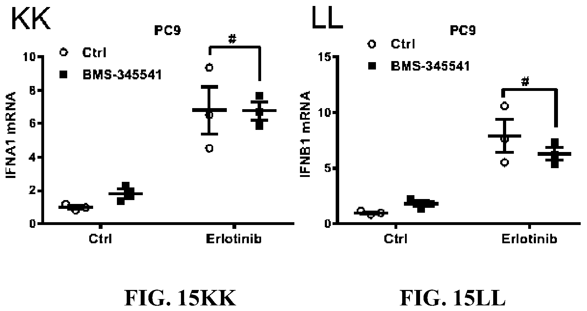

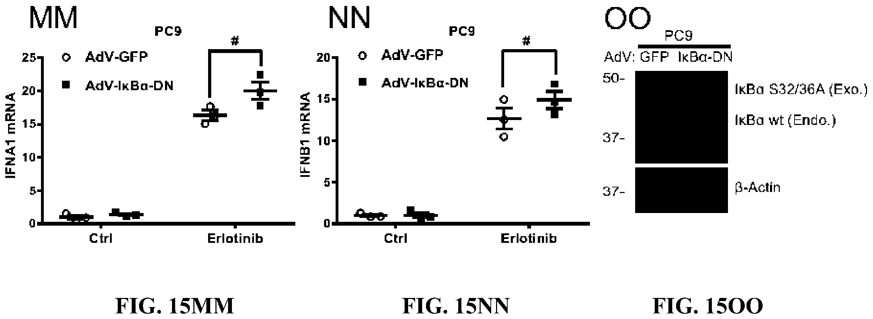

**FIG. 15A****-OO** show representative data illustrating distinguished mechanisms of EGFR inhibition induced Type I IFN regulation. Data indicates mean ± S.E.M of three independent biological replicates. #: p>0.05, *: p<0.05, **: p<0.01, ***: p<0.001, by two-way ANOVA adjusted by Bonferroni's correction. Western blots are representative of 3 independent experiments with similar results. Cropped images are shown. Uncropped images are shown in Source Data.

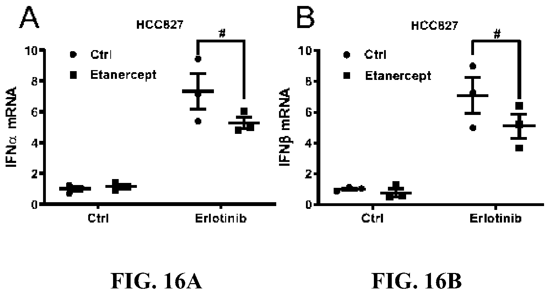

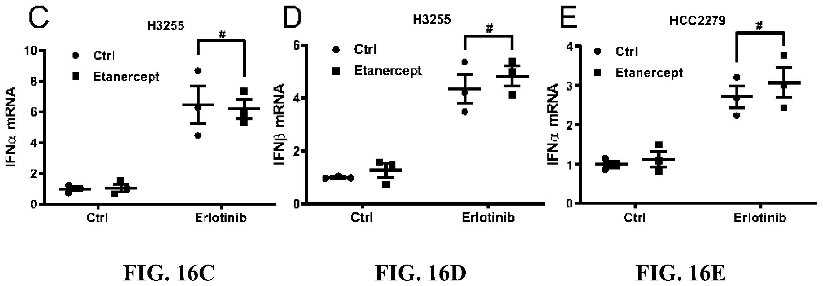

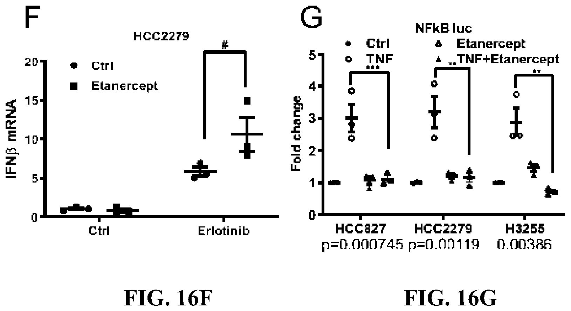

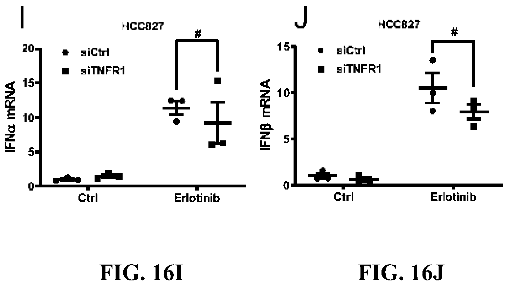

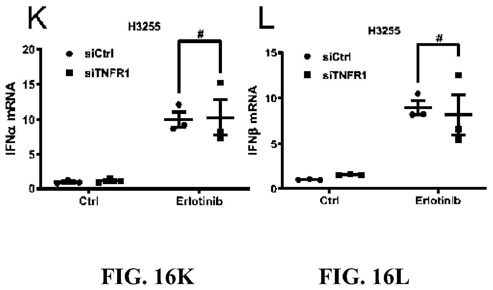

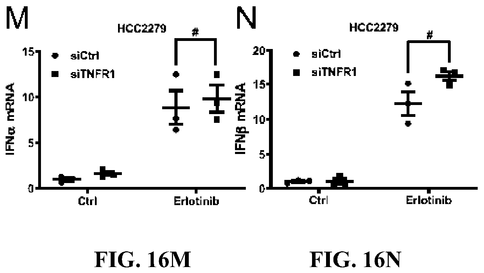

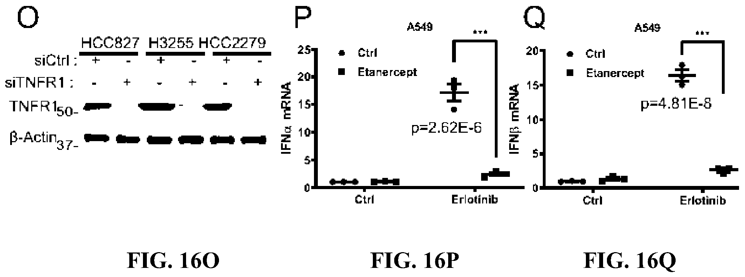

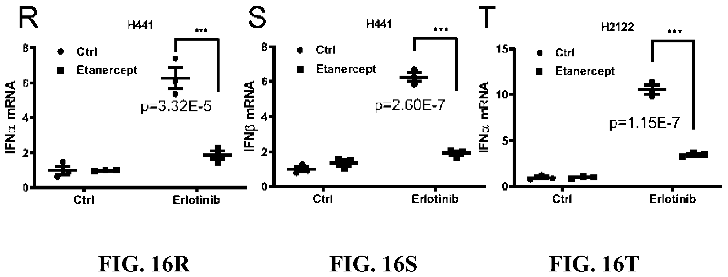

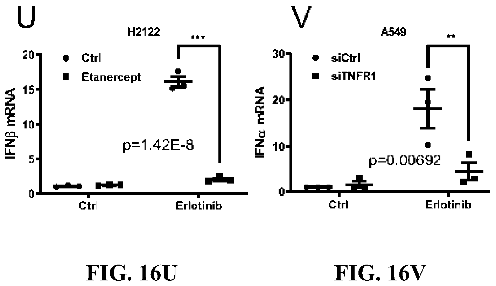

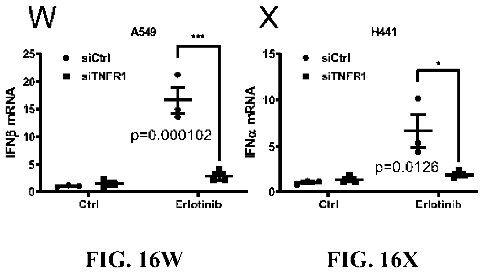

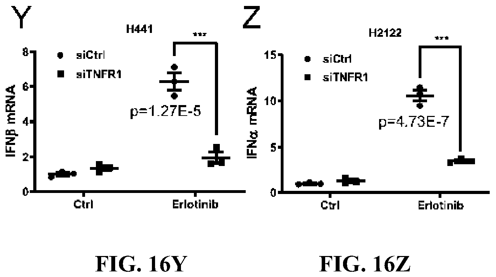

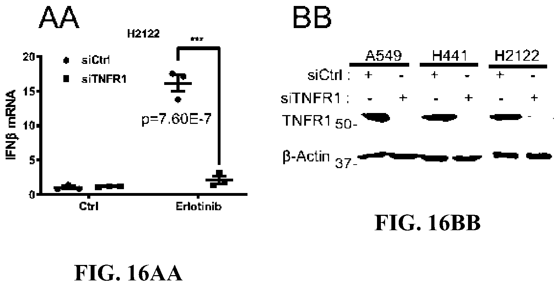

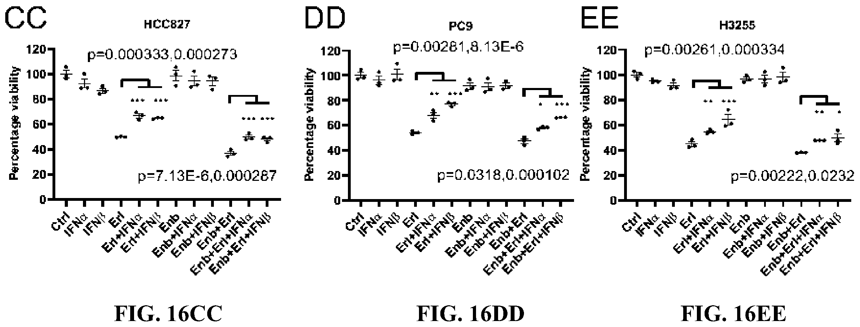

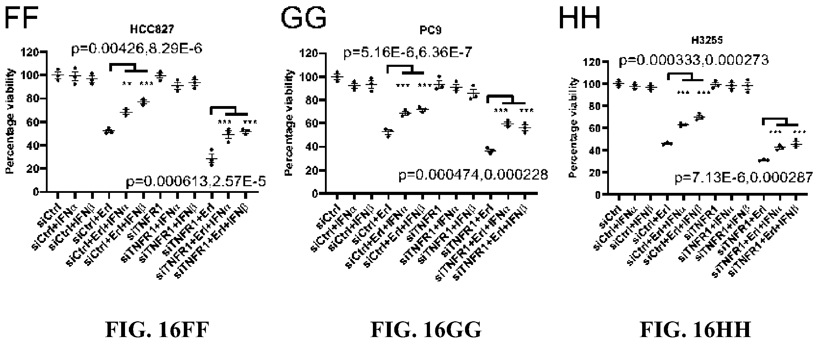

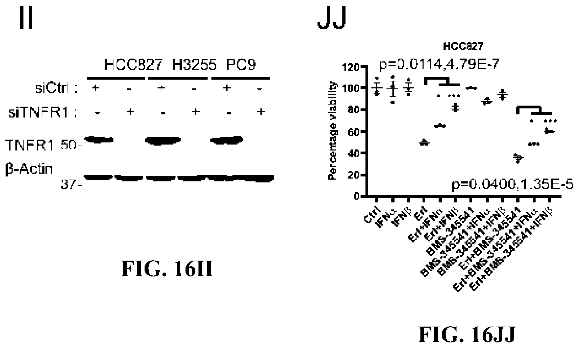

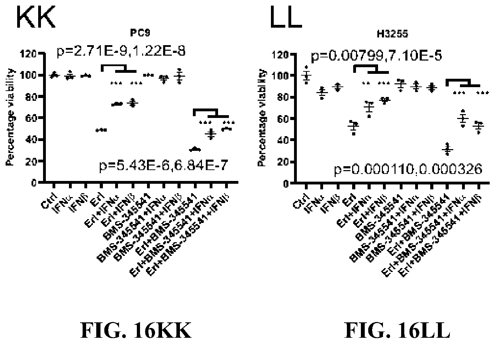

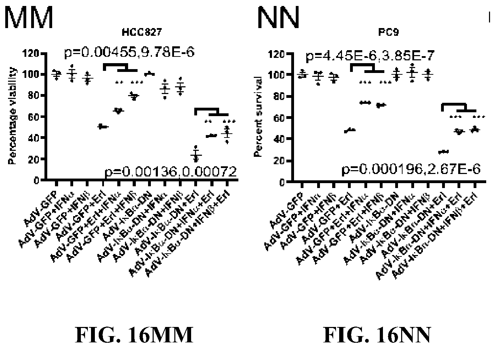

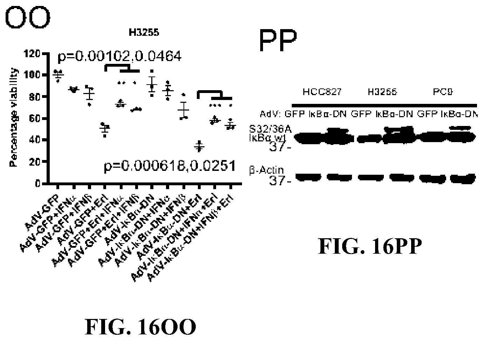

**FIG. 16A****-PP** show representative data illustrating mechanisms and biological effects of EGFR inhibition indued Type I IFN regulation. Data indicates mean ± S.E.M of three independent biological replicates. #: p>0.05, *: p<0.05, **: p<0.01, ***: p<0.001, by two-way (A-AA) or three-way (CC-OO) ANOVA adjusted by Bonferroni's correction. Western blots are representative of 3 independent experiments with similar results. Cropped images are shown. Uncropped Western blot images are shown in Source Data.

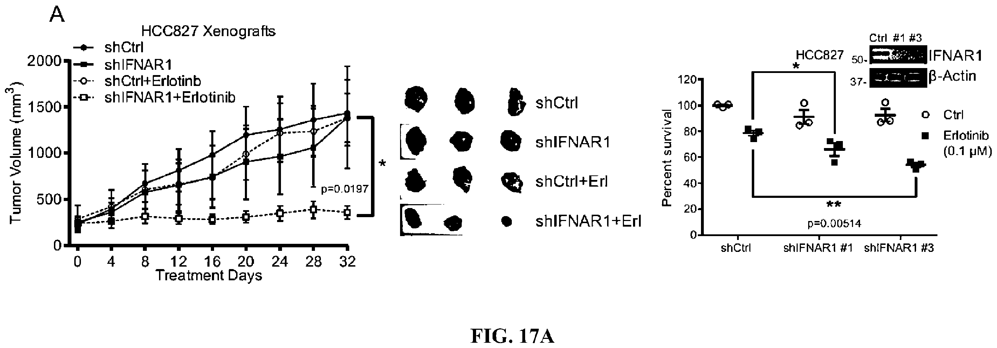

**FIG. 17A-E** show representative data illustrating the synergistic effect of EGFR plus Type I IFN inhibition in mouse models of NSCLC. Data refers to mean ± S.E.M. of tumor sizes, *: p<0.05, **:p<0.01, ***:p<0.001, by two-way ANOVA adjusted by Bonferroni's correction with repeated measures. Westem blots are cropped and representative of three independent repeated experiments with similar results. Uncropped are in Source Data.

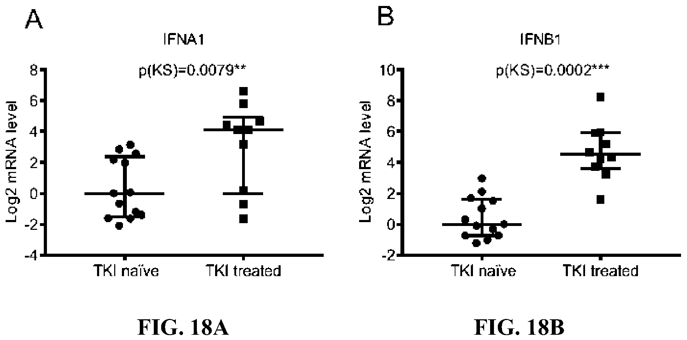

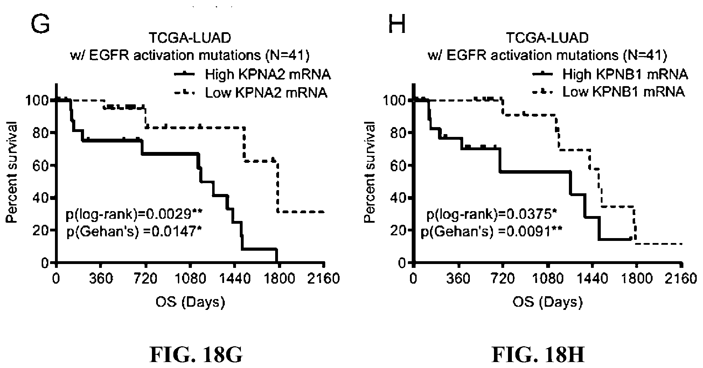

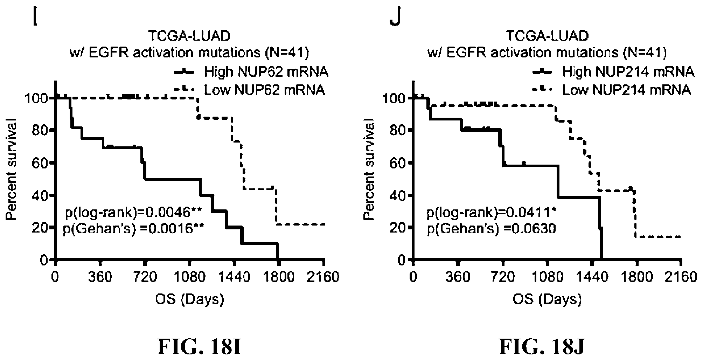

**FIG. 18A-K** show representative data illustrating that Type I IFN level inversely correlates with response to TKI treatment in NSCLC.

Additional advantages of the invention will be set forth in part in the description which follows, and in part will be obvious from the description, or can be learned by practice of the invention. The advantages of the invention will be realized and attained by means of the elements and combinations particularly pointed out in the appended claims. It is to be understood that both the foregoing general description and the following detailed description are exemplary and explanatory only and are not restrictive of the invention, as claimed.

### DETAILED DESCRIPTION

The present invention can be understood more readily by reference to the following detailed description of the invention and the Examples included therein.

In the context of the present invention, the agent that modulates EGFR signaling is erlotinib and the agent that modulates IFN signaling is anifrolumab. Any references to agents modulating EGFR other than erlotinib and to agents modulating IFN signaling other than anifrolumab in the present specification, fall outside the invention and are provided for information only.

Furthermore, in the context of the present invention the cancer to be treated by the claimed combination is NSCLC. Any references to cancers other than NSCLC in the present specification, fall outside the invention and are provided for information only.

### A. DEFINITIONS

As used in the specification and the appended claims, the singular forms "a," "an" and "the" include plural referents unless the context clearly dictates otherwise. Thus, for example, reference to "a functional group," "an alkyl," or "a residue" includes mixtures of two or more such functional groups, alkyls, or residues, and the like.

As used in the specification and in the claims, the term "comprising" can include the aspects "consisting of" and "consisting essentially of."

Ranges can be expressed herein as from "about" one particular value, and/or to "about" another particular value. When such a range is expressed, another aspect includes from the one particular value and/or to the other particular value. Similarly, when values are expressed as approximations, by use of the antecedent "about," it will be understood that the particular value forms another aspect. It will be further understood that the endpoints of each of the ranges are significant both in relation to the other endpoint, and independently of the other endpoint. It is also understood that there are a number of values disclosed herein, and that each value is also herein disclosed as "about" that particular value in addition to the value itself. For example, if the value "10" is disclosed, then "about 10" is also disclosed. It is also understood that each unit between two particular units are also disclosed. For example, if 10 and 15 are disclosed, then 11, 12, 13, and 14 are also disclosed.

As used herein, the terms "about" and "at or about" mean that the amount or value in question can be the value designated some other value approximately or about the same. It is generally understood, as used herein, that it is the nominal value indicated ±10% variation unless otherwise indicated or inferred. The term is intended to convey that similar values promote equivalent results or effects recited in the claims. That is, it is understood that amounts, sizes, formulations, parameters, and other quantities and characteristics are not and need not be exact, but can be approximate and/or larger or smaller, as desired, reflecting tolerances, conversion factors, rounding off, measurement error and the like, and other factors known to those of skill in the art. In general, an amount, size, formulation, parameter or other quantity or characteristic is "about" or "approximate" whether or not expressly stated to be such. It is understood that where "about" is used before a quantitative value, the parameter also includes the specific quantitative value itself, unless specifically stated otherwise.

References in the specification and concluding claims to parts by weight of a particular element or component in a composition denotes the weight relationship between the element or component and any other elements or components in the composition or article for which a part by weight is expressed. Thus, in a compound containing 2 parts by weight of component X and 5 parts by weight component Y, X and Y are present at a weight ratio of 2:5, and are present in such ratio regardless of whether additional components are contained in the compound.

A weight percent (wt. %) of a component, unless specifically stated to the contrary, is based on the total weight of the formulation or composition in which the component is included.

As used herein, "IC₅₀," is intended to refer to the concentration of a substance (e.g., a compound or a drug) that is required for 50% inhibition of a biological process, or component of a process, including a protein, subunit, organelle, ribonucleoprotein, etc. In one aspect, an IC₅₀ can refer to the concentration of a substance that is required for 50% inhibition *in vivo,* as further defined elsewhere herein. In a further aspect, IC₅₀ refers to the half-maximal (50%) inhibitory concentration (IC) of a substance.

As used herein, "EC₅₀," is intended to refer to the concentration of a substance (e.g., a compound or a drug) that is required for 50% agonism of a biological process, or component of a process, including a protein, subunit, organelle, ribonucleoprotein, etc. In one aspect, an EC₅₀ can refer to the concentration of a substance that is required for 50% agonism *in vivo,* as further defined elsewhere herein. In a further aspect, EC₅₀ refers to the concentration of agonist that provokes a response halfway between the baseline and maximum response.

As used herein, the terms "optional" or "optionally" means that the subsequently described event or circumstance can or cannot occur, and that the description includes instances where said event or circumstance occurs and instances where it does not.

As used herein, the term "subject" can be a vertebrate, such as a mammal, a fish, a bird, a reptile, or an amphibian. Thus, the subject of the herein disclosed methods can be a human, non-human primate, horse, pig, rabbit, dog, sheep, goat, cow, cat, guinea pig or rodent. The term does not denote a particular age or sex. Thus, adult and newborn subjects, as well as fetuses, whether male or female, are intended to be covered. In one aspect, the subject is a mammal. A patient refers to a subject afflicted with a disease or disorder. The term "patient" includes human and veterinary subjects.

As used herein, the term "treatment" refers to the medical management of a patient with the intent to cure, ameliorate, stabilize, or prevent a disease, pathological condition, or disorder. This term includes active treatment, that is, treatment directed specifically toward the improvement of a disease, pathological condition, or disorder, and also includes causal treatment, that is, treatment directed toward removal of the cause of the associated disease, pathological condition, or disorder. In addition, this term includes palliative treatment, that is, treatment designed for the relief of symptoms rather than the curing of the disease, pathological condition, or disorder; preventative treatment, that is, treatment directed to minimizing or partially or completely inhibiting the development of the associated disease, pathological condition, or disorder; and supportive treatment, that is, treatment employed to supplement another specific therapy directed toward the improvement of the associated disease, pathological condition, or disorder. In various aspects, the term covers any treatment of a subject, including a mammal *(e.g.,* a human), and includes: (i) preventing the disease from occurring in a subject that can be predisposed to the disease but has not yet been diagnosed as having it; (ii) inhibiting the disease, *i.e.,* arresting its development; or (iii) relieving the disease, *i.e.,* causing regression of the disease. In one aspect, the subject is a mammal such as a primate, and, in a further aspect, the subject is a human. The term "subject" also includes domesticated animals (*e.g.,* cats, dogs, etc.), livestock (*e.g.,* cattle, horses, pigs, sheep, goats, etc.), and laboratory animals (*e.g*., mouse, rabbit, rat, guinea pig, fruit fly, etc.).

As used herein, the term "prevent" or "preventing" refers to precluding, averting, obviating, forestalling, stopping, or hindering something from happening, especially by advance action. It is understood that where reduce, inhibit or prevent are used herein, unless specifically indicated otherwise, the use of the other two words is also expressly disclosed.

As used herein, the term "diagnosed" means having been subjected to a physical examination by a person of skill, for example, a physician, and found to have a condition that can be diagnosed or treated by the compounds, compositions, or methods disclosed herein.

As used herein, the terms "administering" and "administration" refer to any method of providing a pharmaceutical preparation to a subject. Such methods are well known to those skilled in the art and include, but are not limited to, oral administration, transdermal administration, administration by inhalation, nasal administration, topical administration, intravaginal administration, ophthalmic administration, intraaural administration, intracerebral administration, rectal administration, sublingual administration, buccal administration, and parenteral administration, including injectable such as intravenous administration, intra-arterial administration, intramuscular administration, and subcutaneous administration. Administration can be continuous or intermittent. In various aspects, a preparation can be administered therapeutically; that is, administered to treat an existing disease or condition. In further various aspects, a preparation can be administered prophylactically; that is, administered for prevention of a disease or condition.

As used herein, the terms "effective amount" and "amount effective" refer to an amount that is sufficient to achieve the desired result or to have an effect on an undesired condition. For example, a "therapeutically effective amount" refers to an amount that is sufficient to achieve the desired therapeutic result or to have an effect on undesired symptoms, but is generally insufficient to cause adverse side effects. The specific therapeutically effective dose level for any particular patient will depend upon a variety of factors including the disorder being treated and the severity of the disorder; the specific composition employed; the age, body weight, general health, sex and diet of the patient; the time of administration; the route of administration; the rate of excretion of the specific compound employed; the duration of the treatment; drugs used in combination or coincidental with the specific compound employed and like factors well known in the medical arts. For example, it is well within the skill of the art to start doses of a compound at levels lower than those required to achieve the desired therapeutic effect and to gradually increase the dosage until the desired effect is achieved. If desired, the effective daily dose can be divided into multiple doses for purposes of administration. Consequently, single dose compositions can contain such amounts or submultiples thereof to make up the daily dose. The dosage can be adjusted by the individual physician in the event of any contraindications. Dosage can vary, and can be administered in one or more dose administrations daily, for one or several days. Guidance can be found in the literature for appropriate dosages for given classes of pharmaceutical products. In further various aspects, a preparation can be administered in a "prophylactically effective amount"; that is, an amount effective for prevention of a disease or condition.

As used herein, "dosage form" means a pharmacologically active material in a medium, carrier, vehicle, or device suitable for administration to a subject. A dosage forms can comprise inventive a disclosed compound, a product of a disclosed method of making, or a salt, solvate, or polymorph thereof, in combination with a pharmaceutically acceptable excipient, such as a preservative, buffer, saline, or phosphate buffered saline. Dosage forms can be made using conventional pharmaceutical manufacturing and compounding techniques. Dosage forms can comprise inorganic or organic buffers (*e.g*., sodium or potassium salts of phosphate, carbonate, acetate, or citrate) and pH adjustment agents (*e.g*., hydrochloric acid, sodium or potassium hydroxide, salts of citrate or acetate, amino acids and their salts) antioxidants (*e.g.,* ascorbic acid, alpha-tocopherol), surfactants (*e.g.,* polysorbate 20, polysorbate 80, polyoxyethylene9-10 nonyl phenol, sodium desoxycholate), solution and/or cryo/lyo stabilizers (*e.g*., sucrose, lactose, mannitol, trehalose), osmotic adjustment agents (*e.g.,* salts or sugars), antibacterial agents (*e.g.,* benzoic acid, phenol, gentamicin), antifoaming agents (*e.g.,* polydimethylsilozone), preservatives (*e.g.,* thimerosal, 2-phenoxyethanol, EDTA), polymeric stabilizers and viscosity-adjustment agents (*e.g.*, polyvinylpyrrolidone, poloxamer 488, carboxymethylcellulose) and co-solvents (*e.g*., glycerol, polyethylene glycol, ethanol). A dosage form formulated for injectable use can have a disclosed compound, a product of a disclosed method of making, or a salt, solvate, or polymorph thereof, suspended in sterile saline solution for injection together with a preservative.

As used herein, "kit" means a collection of at least two components constituting the kit. Together, the components constitute a functional unit for a given purpose. Individual member components may be physically packaged together or separately. For example, a kit comprising an instruction for using the kit may or may not physically include the instruction with other individual member components. Instead, the instruction can be supplied as a separate member component, either in a paper form or an electronic form which may be supplied on computer readable memory device or downloaded from an internet website, or as recorded presentation.

As used herein, "instruction(s)" means documents describing relevant materials or methodologies pertaining to a kit. These materials may include any combination of the following: background information, list of components and their availability information (purchase information, etc.), brief or detailed protocols for using the kit, trouble-shooting, references, technical support, and any other related documents. Instructions can be supplied with the kit or as a separate member component, either as a paper form or an electronic form which may be supplied on computer readable memory device or downloaded from an internet website, or as recorded presentation. Instructions can comprise one or multiple documents, and are meant to include future updates.

As used herein, the terms "therapeutic agent" include any synthetic or naturally occurring biologically active compound or composition of matter which, when administered to an organism (human or nonhuman animal), induces a desired pharmacologic, immunogenic, and/or physiologic effect by local and/or systemic action. The term therefore encompasses those compounds or chemicals traditionally regarded as drugs, vaccines, and biopharmaceuticals including molecules such as proteins, peptides, hormones, nucleic acids, gene constructs and the like. Examples of therapeutic agents are described in well-known literature references such as the Merck Index (14th edition), the Physicians' Desk Reference (64th edition), and The Pharmacological Basis of Therapeutics (12^{th} edition) , and they include, without limitation, medicaments; vitamins; mineral supplements; substances used for the treatment, prevention, diagnosis, cure or mitigation of a disease or illness; substances that affect the structure or function of the body, or pro-drugs, which become biologically active or more active after they have been placed in a physiological environment. For example, the term "therapeutic agent" includes compounds or compositions for use in all of the major therapeutic areas including, but not limited to, adjuvants; anti-infectives such as antibiotics and antiviral agents; anti-cancer and anti-neoplastic agents such as kinase inhibitors, poly ADP ribose polymerase (PARP) inhibitors and other DNA damage response modifiers, epigenetic agents such as bromodomain and extra-terminal (BET) inhibitors, histone deacetylase (HDAc) inhibitors, iron chelotors and other ribonucleotides reductase inhibitors, proteasome inhibitors and Nedd8-activating enzyme (NAE) inhibitors, mammalian target of rapamycin (mTOR) inhibitors, traditional cytotoxic agents such as paclitaxel, dox, irinotecan, and platinum compounds, immune checkpoint blockade agents such as cytotoxic T lymphocyte antigen-4 (CTLA-4) monoclonal antibody (mAB), programmed cell death protein 1 (PD-1)/programmed cell death-ligand 1 (PD-L1) mAB, cluster of differentiation 47 (CD47) mAB, toll-like receptor (TLR) agonists and other immune modifiers, cell therapeutics such as chimeric antigen receptor T-cell (CAR-T)/chimeric antigen receptor natural killer (CAR-NK) cells, and proteins such as interferons (IFNs), interleukins (ILs), and mAbs; anti-ALS agents such as entry inhibitors, fusion inhibitors, non-nucleoside reverse transcriptase inhibitors (NNRTIs), nucleoside reverse transcriptase inhibitors (NRTIs), nucleotide reverse transcriptase inhibitors, NCP7 inhibitors, protease inhibitors, and integrase inhibitors; analgesics and analgesic combinations, anorexics, anti-inflammatory agents, antiepileptics, local and general anesthetics, hypnotics, sedatives, antipsychotic agents, neuroleptic agents, antidepressants, anxiolytics, antagonists, neuron blocking agents, anticholinergic and cholinomimetic agents, antimuscarinic and muscarinic agents, antiadrenergics, antiarrhythmics, antihypertensive agents, hormones, and nutrients, antiarthritics, antiasthmatic agents, anticonvulsants, antihistamines, antinauseants, antineoplastics, antipruritics, antipyretics; antispasmodics, cardiovascular preparations (including calcium channel blockers, beta-blockers, beta-agonists and antiarrythmics), antihypertensives, diuretics, vasodilators; central nervous system stimulants; cough and cold preparations; decongestants; diagnostics; hormones; bone growth stimulants and bone resorption inhibitors; immunosuppressives; muscle relaxants; psychostimulants; sedatives; tranquilizers; proteins, peptides, and fragments thereof (whether naturally occurring, chemically synthesized or recombinantly produced); and nucleic acid molecules (polymeric forms of two or more nucleotides, either ribonucleotides (RNA) or deoxyribonucleotides (DNA) including both double- and single-stranded molecules, gene constructs, expression vectors, antisense molecules and the like), small molecules (e.g., doxorubicin) and other biologically active macromolecules such as, for example, proteins and enzymes. The agent may be a biologically active agent used in medical, including veterinary, applications and in agriculture, such as with plants, as well as other areas. The term "therapeutic agent" also includes without limitation, medicaments; vitamins; mineral supplements; substances used for the treatment, prevention, diagnosis, cure or mitigation of disease or illness; or substances which affect the structure or function of the body; or pro- drugs, which become biologically active or more active after they have been placed in a predetermined physiological environment.

The term "pharmaceutically acceptable" describes a material that is not biologically or otherwise undesirable, *i.e.,* without causing an unacceptable level of undesirable biological effects or interacting in a deleterious manner.

As used herein, the term "derivative" refers to a compound having a structure derived from the structure of a parent compound (*e.g*., a compound disclosed herein) and whose structure is sufficiently similar to those disclosed herein and based upon that similarity, would be expected by one skilled in the art to exhibit the same or similar activities and utilities as the claimed compounds, or to induce, as a precursor, the same or similar activities and utilities as the claimed compounds. Exemplary derivatives include salts, esters, amides, salts of esters or amides, and N-oxides of a parent compound.

As used herein, the term "pharmaceutically acceptable carrier" refers to sterile aqueous or nonaqueous solutions, dispersions, suspensions or emulsions, as well as sterile powders for reconstitution into sterile injectable solutions or dispersions just prior to use. Examples of suitable aqueous and nonaqueous carriers, diluents, solvents or vehicles include water, ethanol, polyols (such as glycerol, propylene glycol, polyethylene glycol and the like), carboxymethylcellulose and suitable mixtures thereof, vegetable oils (such as olive oil) and injectable organic esters such as ethyl oleate. Proper fluidity can be maintained, for example, by the use of coating materials such as lecithin, by the maintenance of the required particle size in the case of dispersions and by the use of surfactants. These compositions can also contain adjuvants such as preservatives, wetting agents, emulsifying agents and dispersing agents. Prevention of the action of microorganisms can be ensured by the inclusion of various antibacterial and antifungal agents such as paraben, chlorobutanol, phenol, sorbic acid and the like. It can also be desirable to include isotonic agents such as sugars, sodium chloride and the like. Prolonged absorption of the injectable pharmaceutical form can be brought about by the inclusion of agents, such as aluminum monostearate and gelatin, which delay absorption. Injectable depot forms are made by forming microencapsule matrices of the drug in biodegradable polymers such as polylactide-polyglycolide, poly(orthoesters) and poly(anhydrides). Depending upon the ratio of drug to polymer and the nature of the particular polymer employed, the rate of drug release can be controlled. Depot injectable formulations are also prepared by entrapping the drug in liposomes or microemulsions which are compatible with body tissues. The injectable formulations can be sterilized, for example, by filtration through a bacterial-retaining filter or by incorporating sterilizing agents in the form of sterile solid compositions which can be dissolved or dispersed in sterile water or other sterile injectable media just prior to use. Suitable inert carriers can include sugars such as lactose. Desirably, at least 95% by weight of the particles of the active ingredient have an effective particle size in the range of 0.01 to 10 micrometers.

Certain materials, compounds, compositions, and components disclosed herein can be obtained commercially or readily synthesized using techniques generally known to those of skill in the art. For example, the starting materials and reagents used in preparing the disclosed compounds and compositions are either available from commercial suppliers such as Aldrich Chemical Co., (Milwaukee, Wis.), Acros Organics (Morris Plains, N.J.), Strem Chemicals (Newburyport, MA), Fisher Scientific (Pittsburgh, Pa.), or Sigma (St. Louis, Mo.) or are prepared by methods known to those skilled in the art following procedures set forth in references such as Fieser and Fieser's Reagents for Organic Synthesis, Volumes 1-17 (John Wiley and Sons, 1991); Rodd's Chemistry of Carbon Compounds, Volumes 1-5 and supplemental volumes (Elsevier Science Publishers, 1989); Organic Reactions, Volumes 1-40 (John Wiley and Sons, 1991); March's Advanced Organic Chemistry, (John Wiley and Sons, 4th Edition); and Larock's Comprehensive Organic Transformations (VCH Publishers Inc., 1989).

Unless otherwise expressly stated, it is in no way intended that any method set forth herein be construed as requiring that its steps be performed in a specific order. Accordingly, where a method claim does not actually recite an order to be followed by its steps or it is not otherwise specifically stated in the claims or descriptions that the steps are to be limited to a specific order, it is no way intended that an order be inferred, in any respect. This holds for any possible non-express basis for interpretation, including: matters of logic with respect to arrangement of steps or operational flow; plain meaning derived from grammatical organization or punctuation; and the number or type of embodiments described in the specification.

Disclosed are the components to be used to prepare the compositions of the invention as well as the compositions themselves to be used within the methods disclosed herein. These and other materials are disclosed herein, and it is understood that when combinations, subsets, interactions, groups, etc. of these materials are disclosed that while specific reference of each various individual and collective combinations and permutation of these compounds cannot be explicitly disclosed, each is specifically contemplated and described herein. For example, if a particular compound is disclosed and discussed and a number of modifications that can be made to a number of molecules including the compounds are discussed, specifically contemplated is each and every combination and permutation of the compound and the modifications that are possible unless specifically indicated to the contrary. Thus, if a class of molecules A, B, and C are disclosed as well as a class of molecules D, E, and F and an example of a combination molecule, A-D is disclosed, then even if each is not individually recited each is individually and collectively contemplated meaning combinations, A-E, A-F, B-D, B-E, B-F, C-D, C-E, and C-F are considered disclosed. Likewise, any subset or combination of these is also disclosed. Thus, for example, the sub-group of A-E, B-F, and C-E would be considered disclosed. This concept applies to all aspects of this application including, but not limited to, steps in methods of making and using the compositions of the invention. Thus, if there are a variety of additional steps that can be performed it is understood that each of these additional steps can be performed with any specific embodiment or combination of embodiments of the methods of the invention.

It is understood that the compounds and compositions disclosed herein have certain functions. Disclosed herein are certain structural requirements for performing the disclosed functions, and it is understood that there are a variety of structures that can perform the same function that are related to the disclosed structures, and that these structures will typically achieve the same result.

### B. PHARMACEUTICAL COMPOSITIONS

In one aspect, disclosed are pharmaceutical compositions comprising: (a) an agent that modulates EGFR signaling, or a pharmaceutically acceptable salt thereof; (b) an agent that modulates IFN signaling, or a pharmaceutically acceptable salt thereof; and (c) a pharmaceutically acceptable carrier, wherein at least one of the agent that modulates EGFR signaling and the agent that modulates IFN signaling is present in an effective amount.

In various aspects, the compounds and compositions of the invention can be administered in pharmaceutical compositions, which are formulated according to the intended method of administration. The compounds and compositions described herein can be formulated in a conventional manner using one or more physiologically acceptable carriers or excipients. For example, a pharmaceutical composition can be formulated for local or systemic administration, intravenous, topical, or oral administration.

The nature of the pharmaceutical compositions for administration is dependent on the mode of administration and can readily be determined by one of ordinary skill in the art. In various aspects, the pharmaceutical composition is sterile or sterilizable. The therapeutic compositions featured in the invention can contain carriers or excipients, many of which are known to skilled artisans. Excipients that can be used include buffers (for example, citrate buffer, phosphate buffer, acetate buffer, and bicarbonate buffer), amino acids, urea, alcohols, ascorbic acid, phospholipids, polypeptides (for example, serum albumin), EDTA, sodium chloride, liposomes, mannitol, sorbitol, water, and glycerol. The nucleic acids, polypeptides, small molecules, and other modulatory compounds featured in the invention can be administered by any standard route of administration. For example, administration can be parenteral, intravenous, subcutaneous, or oral. A modulatory compound can be formulated in various ways, according to the corresponding route of administration. For example, liquid solutions can be made for administration by drops into the ear, for injection, or for ingestion; gels or powders can be made for ingestion or topical application. Methods for making such formulations are well known and can be found in, for example, Remington's Pharmaceutical Sciences, 18th Ed., Gennaro, ed., Mack Publishing Co., Easton, PA 1990.

In various aspects, the disclosed pharmaceutical compositions comprise the disclosed compounds (including pharmaceutically acceptable salt(s) thereof) as an active ingredient, a pharmaceutically acceptable carrier, and, optionally, other therapeutic ingredients or adjuvants. The instant compositions include those suitable for oral, rectal, topical, and parenteral (including subcutaneous, intramuscular, and intravenous) administration, although the most suitable route in any given case will depend on the particular host, and nature and severity of the conditions for which the active ingredient is being administered. The pharmaceutical compositions can be conveniently presented in unit dosage form and prepared by any of the methods well known in the art of pharmacy.

In various aspects, the pharmaceutical compositions of this invention can include a pharmaceutically acceptable carrier and a compound or a pharmaceutically acceptable salt of the compounds of the invention. The compounds of the invention, or pharmaceutically acceptable salts thereof, can also be included in pharmaceutical compositions in combination with one or more other therapeutically active compounds.

The pharmaceutical carrier employed can be, for example, a solid, liquid, or gas. Examples of solid carriers include lactose, terra alba, sucrose, talc, gelatin, agar, pectin, acacia, magnesium stearate, and stearic acid. Examples of liquid carriers are sugar syrup, peanut oil, olive oil, and water. Examples of gaseous carriers include carbon dioxide and nitrogen.

In preparing the compositions for oral dosage form, any convenient pharmaceutical media can be employed. For example, water, glycols, oils, alcohols, flavoring agents, preservatives, coloring agents and the like can be used to form oral liquid preparations such as suspensions, elixirs and solutions; while carriers such as starches, sugars, microcrystalline cellulose, diluents, granulating agents, lubricants, binders, disintegrating agents, and the like can be used to form oral solid preparations such as powders, capsules and tablets. Because of their ease of administration, tablets and capsules are the preferred oral dosage units whereby solid pharmaceutical carriers are employed. Optionally, tablets can be coated by standard aqueous or nonaqueous techniques.

A tablet containing the composition of this invention can be prepared by compression or molding, optionally with one or more accessory ingredients or adjuvants. Compressed tablets can be prepared by compressing, in a suitable machine, the active ingredient in a free-flowing form such as powder or granules, optionally mixed with a binder, lubricant, inert diluent, surface active or dispersing agent. Molded tablets can be made by molding in a suitable machine, a mixture of the powdered compound moistened with an inert liquid diluent.

The pharmaceutical compositions of the present invention comprise a compound of the invention (or pharmaceutically acceptable salts thereof) as an active ingredient, a pharmaceutically acceptable carrier, and optionally one or more additional therapeutic agents or adjuvants. The instant compositions include compositions suitable for oral, rectal, topical, and parenteral (including subcutaneous, intramuscular, and intravenous) administration, although the most suitable route in any given case will depend on the particular host, and nature and severity of the conditions for which the active ingredient is being administered. The pharmaceutical compositions can be conveniently presented in unit dosage form and prepared by any of the methods well known in the art of pharmacy.

Pharmaceutical compositions of the present invention suitable for parenteral administration can be prepared as solutions or suspensions of the active compounds in water. A suitable surfactant can be included such as, for example, hydroxypropylcellulose. Dispersions can also be prepared in glycerol, liquid polyethylene glycols, and mixtures thereof in oils. Further, a preservative can be included to prevent the detrimental growth of microorganisms.

Pharmaceutical compositions of the present invention suitable for injectable use include sterile aqueous solutions or dispersions. Furthermore, the compositions can be in the form of sterile powders for the extemporaneous preparation of such sterile injectable solutions or dispersions. In all cases, the final injectable form must be sterile and must be effectively fluid for easy syringability. The pharmaceutical compositions must be stable under the conditions of manufacture and storage; thus, preferably should be preserved against the contaminating action of microorganisms such as bacteria and fungi. The carrier can be a solvent or dispersion medium containing, for example, water, ethanol, polyol (e.g., glycerol, propylene glycol and liquid polyethylene glycol), vegetable oils, and suitable mixtures thereof.

Pharmaceutical compositions of the present invention can be in a form suitable for topical use such as, for example, an aerosol, cream, ointment, lotion, dusting powder, mouth washes, gargles, and the like. Further, the compositions can be in a form suitable for use in transdermal devices. These formulations can be prepared, utilizing a compound of the invention, or pharmaceutically acceptable salts thereof, via conventional processing methods. As an example, a cream or ointment is prepared by mixing hydrophilic material and water, together with about 5 wt% to about 10 wt% of the compound, to produce a cream or ointment having a desired consistency.

Pharmaceutical compositions of this invention can be in a form suitable for rectal administration wherein the carrier is a solid. It is preferable that the mixture forms unit dose suppositories. Suitable carriers include cocoa butter and other materials commonly used in the art. The suppositories can be conveniently formed by first admixing the composition with the softened or melted carrier(s) followed by chilling and shaping in molds.

In addition to the aforementioned carrier ingredients, the pharmaceutical formulations described above can include, as appropriate, one or more additional carrier ingredients such as diluents, buffers, flavoring agents, binders, surface-active agents, thickeners, lubricants, preservatives (including anti-oxidants) and the like. Furthermore, other adjuvants can be included to render the formulation isotonic with the blood of the intended recipient. Compositions containing a compound of the invention, and/or pharmaceutically acceptable salts thereof, can also be prepared in powder or liquid concentrate form.

In various aspects, the agent that modulates EGFR signaling is an EGFR inhibitor. In a further aspect, the EGFR inhibitor is a tyrosine kinase inhibitor. Examples of tyrosine kinase inhibitors include, but are not limited to, erlotinib. In a still further aspect, the EGFR inhibitor is a monoclonal antibody.

In various aspects, the EGFR inhibitor is selected from erlotinib, afatinib, cetuximab, panitumumab, erlotinib HCl, gefitinib, lapatinib, neratinib, lifirafenib, HER2-inhibitor-1, nazartinib, naquotinib, canertinib, AG-490, CP-724714, Dacomitinib, WZ4002, Sapitinib, CUDC-101, AG-1478, PD153035 HCl, pelitinib, AC480, AEE788, AP261 13-analog, OSI-420, WZ3146, WZ8040, AST-1306, rociletinib, genisten, varlitinib, icotinib, TAK-285, WHI-P154, daphnetin, PD168393, tyrphostin9, CNX-2006, AG-18, AZ5104, osimertinib, CL-387785, olmutinib, AZD3759, poziotinib, vandetanib, and necitumumab.

In various aspects, IFN signaling is Type I IFN signaling.

In various aspects, the agent that modulates IFN signaling is an interferon blocking antibody or an interferon neutralizing antibody. In a further aspect, the agent that modulates IFN signaling is an IFN inhibitor. Examples of IFN inhibitors include, but are not limited to, anifrolumab.

In various aspects, the agent that modulates EGFR signaling is an EGFR inhibitor and wherein the agent that modulates IFN signaling is an IFN inhibitor. In a further aspect, the agent that modulates EGFR signaling is erlotinib and the agent that modulates IFN signaling is anifrolumab.

In various aspects, the agent that modulates EGFR signaling and the agent that modulates IFN signaling are co-formulated. In a further aspect, the agent that modulates EGFR signaling and the agent that modulates IFN signaling are co-packaged.

In various aspects, the agent that modulates EGFR signaling and the agent that modulates IFN signaling are administered concurrently. In a further aspect, the agent that modulates EGFR signaling and the agent that modulates IFN signaling are not administered concurrently.

In various aspects, the agent that modulates EGFR signaling is erlotinib and the agent that modulates IFN signaling is anifrolumab.

In a further aspect, an effective amount is a therapeutically effective amount. In a still further aspect, an effective amount is a prophylactically effective amount.

In various aspects, the effective amount is an individually effective amount of the agent that modulates EGFR signaling or the agent that modulates IFN signaling. In a further aspect, the effective amount is an individually effective amount of the agent that modulates EGFR signaling. In a still further aspect, the effective amount is an individually effective amount of the agent that modulates IFN signaling.

In various aspects, the effective amount is a combinatorically effective amount of the agent that modulates EGFR signaling and the agent that modulates IFN signaling.

In a further aspect, the pharmaceutical composition is administered to a mammal. In a still further aspect, the mammal is a human. In an even further aspect, the human is a patient.

In a further aspect, the pharmaceutical composition is used to treat cancers such as, for example, sarcomas, carcinomas, hematological cancers, solid tumors, breast cancer, cervical cancer, gastrointestinal cancer, colorectal cancer, brain cancer, skin cancer, prostate cancer, ovarian cancer, bladder cancer, thyroid cancer, testicular cancer, pancreatic cancer, endometrial cancer, melanomas, gliomas, leukemias, lymphomas, chronic myeloproliferative disorders, myelodysplastic syndromes, myeloproliferative neoplasms, and plasma cell neoplasms (myelomas).

In a further aspect, the cancer is selected from a sarcoma, a carcinoma, a hematological cancer, a solid tumor, breast cancer, cervical cancer, gastrointestinal cancer, colorectal cancer, brain cancer, skin cancer, prostate cancer, ovarian cancer, thyroid cancer, testicular cancer, pancreatic cancer, liver cancer, endometrial cancer, melanoma, a glioma, leukemia, lymphoma, chronic myeloproliferative disorder, myelodysplastic syndrome, myeloproliferative neoplasm, non-small cell lung carcinoma, and plasma cell neoplasm (myeloma).

In a further aspect, the cancer is NSCLC.

In a further aspect, the cancer expresses EGFR wild type. In a still further aspect, the cancer expresses EGFR mutant. In yet a further aspect, the cancer is resistant to EGFR inhibition.

In a further aspect, the cancer is a solid tumor.

In various aspects, the composition is a solid dosage form. In a further aspect, the composition is an oral solid dosage form. In a still further aspect, the solid dosage form is a tablet. In yet a further aspect, the solid dosage form is a capsule.

In various aspects, the composition is an injectable dosage form.

It is understood that the disclosed compositions can be prepared from the disclosed compounds. It is also understood that the disclosed compositions can be employed in the disclosed methods of using.

### C. METHODS FOR MAKING A PHARMACEUTICAL COMPOSITION

In one aspect, disclosed are methods for making a pharmaceutical composition, the method comprising combining: (a) an agent that modulates EGFR signaling, or a pharmaceutically acceptable salt thereof; (b) an agent that modulates IFN signaling, or a pharmaceutically acceptable salt thereof; and (c) a pharmaceutically acceptable carrier, wherein at least one of the agent that modulates EGFR signaling and the agent that modulates IFN signaling is present in an effective amount.

In various aspects, the agent that modulates EGFR signaling is an EGFR inhibitor. In a further aspect, the EGFR inhibitor is a tyrosine kinase inhibitor. Examples of tyrosine kinase inhibitors include, but are not limited to, erlotinib. In a still further aspect, the EGFR inhibitor is a monoclonal antibody.

In various aspects, the EGFR inhibitor is selected from erlotinib, afatinib, cetuximab, panitumumab, erlotinib HCl, gefitinib, lapatinib, neratinib, lifirafenib, HER2-inhibitor-1, nazartinib, naquotinib, canertinib, AG-490, CP-724714, Dacomitinib, WZ4002, Sapitinib, CUDC-101, AG-1478, PD153035 HCl, pelitinib, AC480, AEE788, AP261 13-analog, OSI-420, WZ3146, WZ8040, AST-1306, rociletinib, genisten, varlitinib, icotinib, TAK-285, WHI-P154, daphnetin, PD168393, tyrphostin9, CNX-2006, AG-18, AZ5104, osimertinib, CL-387785, olmutinib, AZD3759, poziotinib, vandetanib, and necitumumab.

In various aspects, IFN signaling is Type I IFN signaling.

In various aspects, the agent that modulates IFN signaling is an interferon blocking antibody or an interferon neutralizing antibody. In a further aspect, the agent that modulates IFN signaling is an IFN inhibitor. Examples of IFN inhibitors include, but are not limited to, anifrolumab.

In various aspects, the agent that modulates EGFR signaling is an EGFR inhibitor and wherein the agent that modulates IFN signaling is an IFN inhibitor. In a further aspect, the agent that modulates EGFR signaling is erlotinib and the agent that modulates IFN signaling is anifrolumab.

In various aspects, the agent that modulates EGFR signaling and the agent that modulates IFN signaling are co-formulated. In a further aspect, the agent that modulates EGFR signaling and the agent that modulates IFN signaling are co-packaged.

In various aspects, the agent that modulates EGFR signaling and the agent that modulates IFN signaling are administered concurrently. In a further aspect, the agent that modulates EGFR signaling and the agent that modulates IFN signaling are not administered concurrently.

In various aspects, the agent that modulates EGFR signaling is erlotinib and the agent that modulates IFN signaling is anifrolumab.

In a further aspect, an effective amount is a therapeutically effective amount. In a still further aspect, an effective amount is a prophylactically effective amount.

In various aspects, the effective amount is an individually effective amount of the agent that modulates EGFR signaling or the agent that modulates IFN signaling. In a further aspect, the effective amount is an individually effective amount of the agent that modulates EGFR signaling. In a still further aspect, the effective amount is an individually effective amount of the agent that modulates IFN signaling.

In various aspects, the effective amount is a combinatorically effective amount of the agent that modulates EGFR signaling and the agent that modulates IFN signaling.

In various aspects, combining is co-formulating the agent that modulates EGFR signaling and the agent that modulates IFN signaling with the pharmaceutically acceptable carrier. In a further aspect, co-formulating provides an oral solid dosage form comprising the agent that modulates EGFR signaling, the agent that modulates IFN signaling, and the pharmaceutically acceptable carrier. In a still further aspect, the solid dosage form is a tablet. In yet a further aspect, the solid dosage form is a capsule.

In various aspects, co-formulating provides an injectable dosage form comprising the agent that modulates EGFR signaling, the agent that modulates IFN signaling, and the pharmaceutically acceptable carrier.

### D. METHODS FOR TREATING CANCER

In one aspect, disclosed are methods for treating cancer in a subject, the method comprising administering to the subject an effective amount of an agent that modulates epidermal growth factor receptor (EGFR) signaling, or a pharmaceutically acceptable salt thereof, and an agent that modulates interferon (IFN) signaling, or a pharmaceutically acceptable salt thereof.

In one aspect, disclosed are methods for treating cancer in a patient in need thereof, said method comprising administering to said patient an effective amount of erlotinib and anifrolumab.

In various aspects, the agent that modulates EGFR signaling is an EGFR inhibitor. In a further aspect, the EGFR inhibitor is a tyrosine kinase inhibitor. Examples of tyrosine kinase inhibitors include, but are not limited to, erlotinib. In a still further aspect, the EGFR inhibitor is a monoclonal antibody.

In various aspects, the EGFR inhibitor is selected from erlotinib, afatinib, cetuximab, panitumumab, erlotinib HCl, gefitinib, lapatinib, neratinib, lifirafenib, HER2-inhibitor-1, nazartinib, naquotinib, canertinib, AG-490, CP-724714, Dacomitinib, WZ4002, Sapitinib, CUDC-101, AG-1478, PD153035 HCl, pelitinib, AC480, AEE788, AP261 13-analog, OSI-420, WZ3146, WZ8040, AST-1306, rociletinib, genisten, varlitinib, icotinib, TAK-285, WHI-P154, daphnetin, PD168393, tyrphostin9, CNX-2006, AG-18, AZ5104, osimertinib, CL-387785, olmutinib, AZD3759, poziotinib, vandetanib, and necitumumab.

In various aspects, IFN signaling is Type I IFN signaling.

In various aspects, the agent that modulates IFN signaling is an interferon blocking antibody or an interferon neutralizing antibody. In a further aspect, the agent that modulates IFN signaling is an IFN inhibitor. Examples of IFN inhibitors include, but are not limited to, anifrolumab.

In various aspects, the agent that modulates EGFR signaling is an EGFR inhibitor and wherein the agent that modulates IFN signaling is an IFN inhibitor. In a further aspect, the agent that modulates EGFR signaling is erlotinib and wherein the agent that modulates IFN signaling is anifrolumab.

In various aspects, the agent that modulates EGFR signaling and the agent that modulates IFN signaling are co-formulated. In a further aspect, the agent that modulates EGFR signaling and the agent that modulates IFN signaling are co-packaged.

In various aspects, the agent that modulates EGFR signaling and the agent that modulates IFN signaling are administered concurrently. In a further aspect, the agent that modulates EGFR signaling and the agent that modulates IFN signaling are not administered concurrently.

In various aspects, the effective amount is a therapeutically effective amount. In a further aspect, the effective amount is a prophylactically effective amount.

In various aspects, the effective amount is an individually effective amount of the agent that modulates EGFR signaling or the agent that modulates IFN signaling. In a further aspect, the effective amount is an individually effective amount of the agent that modulates EGFR signaling. In a still further aspect, the effective amount is an individually effective amount of the agent that modulates IFN signaling.

In various aspects, the effective amount is a combinatorically effective amount of the agent that modulates EGFR signaling and the agent that modulates IFN signaling.

In a further aspect, the subject has been diagnosed with a need for treatment of cancer prior to the administering step. In a still further aspect, the subject is at risk for developing cancer prior to the administering step.

In a further aspect, the subject is a mammal. In a still further aspect, the mammal is a human.

In a further aspect, the method further comprises the step of identifying a subject in need of treatment of cancer.

In a further aspect, the cancer is selected from a sarcoma, a carcinoma, a hematological cancer, a solid tumor, breast cancer, cervical cancer, gastrointestinal cancer, colorectal cancer, brain cancer, skin cancer, prostate cancer, ovarian cancer, thyroid cancer, testicular cancer, pancreatic cancer, liver cancer, endometrial cancer, melanoma, a glioma, leukemia, lymphoma, chronic myeloproliferative disorder, myelodysplastic syndrome, myeloproliferative neoplasm, non-small cell lung carcinoma, and plasma cell neoplasm (myeloma).

In a further aspect, the cancer is NSCLC.

In a further aspect, the cancer expresses EGFR wild type. In a still further aspect, the cancer expresses EGFR mutant. In yet a further aspect, the cancer is resistant to EGFR inhibition.

In a further aspect, the cancer is a solid tumor.

In a further aspect, the method further comprises the step of administering a therapeutically effective amount of at least one chemotherapeutic agent. In yet a further aspect, the chemotherapeutic agent is selected from an alkylating agent, an antimetabolite agent, an antineoplastic antibiotic agent, a mitotic inhibitor agent, and a mTor inhibitor agent.

In various aspects, the antineoplastic antibiotic agent is selected from doxorubicin, mitoxantrone, bleomycin, daunorubicin, dactinomycin, epirubicin, idarubicin, plicamycin, mitomycin, pentostatin, and valrubicin, or a pharmaceutically acceptable salt thereof.

In various aspects, the antimetabolite agent is selected from gemcitabine, 5-fluorouracil, capecitabine, hydroxyurea, mercaptopurine, pemetrexed, fludarabine, nelarabine, cladribine, clofarabine, cytarabine, decitabine, pralatrexate, floxuridine, methotrexate, and thioguanine, or a pharmaceutically acceptable salt thereof.

In various aspects, the alkylating agent is selected from carboplatin, cisplatin, cyclophosphamide, chlorambucil, melphalan, carmustine, busulfan, lomustine, dacarbazine, oxaliplatin, ifosfamide, mechlorethamine, temozolomide, thiotepa, bendamustine, and streptozocin, or a pharmaceutically acceptable salt thereof.

In various aspects, the mitotic inhibitor agent is selected from irinotecan, topotecan, rubitecan, cabazitaxel, docetaxel, paclitaxel, etopside, vincristine, ixabepilone, vinorelbine, vinblastine, and teniposide, or a pharmaceutically acceptable salt thereof.

In various aspects, the mTor inhibitor agent is selected from everolimus, siroliumus, and temsirolimus, or a pharmaceutically acceptable salt, hydrate, solvate, or polymorph thereof.

### E. ADDITIONAL METHODS OF USING THE COMPOUNDS

The compounds and pharmaceutical compositions of the invention are useful in treating or controlling cancer. Examples of cancers include, but are not limited to, sarcomas, carcinomas, hematological cancers, solid tumors, breast cancer, cervical cancer, gastrointestinal cancer, colorectal cancer, brain cancer, skin cancer, prostate cancer, ovarian cancer, bladder cancer, thyroid cancer, testicular cancer, pancreatic cancer, endometrial cancer, melanomas, gliomas, leukemias, lymphomas, chronic myeloproliferative disorders, myelodysplastic syndromes, myeloproliferative neoplasms, and plasma cell neoplasms (myelomas).

To treat or control cancer, the compounds and pharmaceutical compositions comprising the compounds are administered to a subject in need thereof, such as a vertebrate, e.g., a mammal, a fish, a bird, a reptile, or an amphibian. The subject can be a human, non-human primate, horse, pig, rabbit, dog, sheep, goat, cow, cat, guinea pig or rodent. The term does not denote a particular age or sex. Thus, adult and newborn subjects, as well as fetuses, whether male or female, are intended to be covered. The subject is preferably a mammal, such as a human. Prior to administering the compounds or compositions, the subject can be diagnosed with a need for treatment of cancer.

The compounds or compositions can be administered to the subject according to any method. Such methods are well known to those skilled in the art and include, but are not limited to, oral administration, transdermal administration, administration by inhalation, nasal administration, topical administration, intravaginal administration, ophthalmic administration, intraaural administration, intracerebral administration, rectal administration, sublingual administration, buccal administration and parenteral administration, including injectable such as intravenous administration, intra-arterial administration, intramuscular administration, and subcutaneous administration. Administration can be continuous or intermittent. A preparation can be administered therapeutically; that is, administered to treat an existing disease or condition. A preparation can also be administered prophylactically; that is, administered for prevention of cancer.

The therapeutically effective amount or dosage of the compound can vary within wide limits. Such a dosage is adjusted to the individual requirements in each particular case including the specific compound(s) being administered, the route of administration, the condition being treated, as well as the patient being treated. In general, in the case of oral or parenteral administration to adult humans weighing approximately 70 Kg or more, a daily dosage of about 10 mg to about 10,000 mg, preferably from about 200 mg to about 1,000 mg, should be appropriate, although the upper limit may be exceeded. The daily dosage can be administered as a single dose or in divided doses, or for parenteral administration, as a continuous infusion. Single dose compositions can contain such amounts or submultiples thereof of the compound or composition to make up the daily dose. The dosage can be adjusted by the individual physician in the event of any contraindications. Dosage can vary, and can be administered in one or more dose administrations daily, for one or several days.

### 1. USE OF AGENTS AND COMPOSITIONS

In one aspect, the invention relates to the use of a disclosed agent, a disclosed pharmaceutical composition, or a product of a disclosed method. In a further aspect, a use relates to the manufacture of a medicament for the treatment of cancer in a subject.

Also provided are the uses of the disclosed agents, compositions, and products. In one aspect, the invention relates to use of at least one disclosed agent, or a pharmaceutically acceptable salt, hydrate, solvate, or polymorph thereof, or at least one disclosed composition. In a further aspect, the composition used is a product of a disclosed method of making.

In a further aspect, the use relates to a process for preparing a pharmaceutical composition comprising a therapeutically effective amount of a disclosed agent or a product of a disclosed method of making, or a pharmaceutically acceptable salt, solvate, or polymorph thereof, for use as a medicament.

In a further aspect, the use relates to a process for preparing a pharmaceutical composition comprising a therapeutically effective amount of a disclosed agent or a product of a disclosed method of making, or a pharmaceutically acceptable salt, solvate, or polymorph thereof, wherein a pharmaceutically acceptable carrier is intimately mixed with a therapeutically effective amount of the compound or the product of a disclosed method of making.

In various aspects, the use relates to a treatment of cancer in a subject. In one aspect, the use is characterized in that the subject is a human. In one aspect, the use is characterized in that the cancer is NSCLC.

In a further aspect, the use relates to the manufacture of a medicament for the treatment of cancer in a subject.

It is understood that the disclosed uses can be employed in connection with the disclosed agents, products of disclosed methods of making, methods, compositions, and kits. In a further aspect, the invention relates to the use of a disclosed agents or a disclosed product in the manufacture of a medicament for the treatment of cancer in a mammal. In a further aspect, the cancer is NSCLC.

### 2. MANUFACTURE OF A MEDICAMENT

In one aspect, the invention relates to a method for the manufacture of a medicament for treating cancer in a subject having cancer, the method comprising combining a therapeutically effective amount of a disclosed agent, composition, or product of a disclosed method with a pharmaceutically acceptable carrier or diluent.

As regards these applications, the present method includes the administration to an animal, particularly a mammal, and more particularly a human, of a therapeutically effective amount of the agents effective in the treatment of cancer. The dose administered to an animal, particularly a human, in the context of the present invention should be sufficient to affect a therapeutic response in the animal over a reasonable time frame. One skilled in the art will recognize that dosage will depend upon a variety of factors including the condition of the animal and the body weight of the animal.

The total amount of the agent of the present disclosure administered in a typical treatment is preferably between about 0.05 mg/kg and about 100 mg/kg of body weight for mice, and more preferably between 0.05 mg/kg and about 50 mg/kg of body weight for mice, and between about 100 mg/kg and about 500 mg/kg of body weight, and more preferably between 200 mg/kg and about 400 mg/kg of body weight for humans per daily dose. This total amount is typically, but not necessarily, administered as a series of smaller doses over a period of about one time per day to about three times per day for about 24 months, and preferably over a period of twice per day for about 12 months.

The size of the dose also will be determined by the route, timing and frequency of administration as well as the existence, nature and extent of any adverse side effects that might accompany the administration of the agent or composition and the desired physiological effect. It will be appreciated by one of skill in the art that various conditions or disease states, in particular chronic conditions or disease states, may require prolonged treatment involving multiple administrations.

Thus, in one aspect, the invention relates to the manufacture of a medicament comprising combining a disclosed agent, composition, or a product of a disclosed method of making, or a pharmaceutically acceptable salt, solvate, or polymorph thereof, with a pharmaceutically acceptable carrier or diluent.

### 3. KITS

In one aspect, disclosed are kits comprising an agent that modulates EGFR signaling, or a pharmaceutically acceptable salt thereof, and an agent that modulates IFN signaling, or a pharmaceutically acceptable salt thereof, and one or more of: (a) an agent associated with the treatment of cancer; (b) instructions for administering the agent that modulates EGFR signaling and/or the agent that modulates IFN signaling in connection with treating cancer; and (c) instructions for treating cancer.

In various aspects, the agent that modulates EGFR signaling is an EGFR inhibitor. In a further aspect, the EGFR inhibitor is a tyrosine kinase inhibitor. Examples of tyrosine kinase inhibitors include, but are not limited to, erlotinib. In a still further aspect, the EGFR inhibitor is a monoclonal antibody.

In various aspects, the EGFR inhibitor is selected from erlotinib, afatinib, cetuximab, panitumumab, erlotinib HCl, gefitinib, lapatinib, neratinib, lifirafenib, HER2-inhibitor-1, nazartinib, naquotinib, canertinib, AG-490, CP-724714, Dacomitinib, WZ4002, Sapitinib, CUDC-101, AG-1478, PD153035 HCl, pelitinib, AC480, AEE788, AP261 13-analog, OSI-420, WZ3146, WZ8040, AST-1306, rociletinib, genisten, varlitinib, icotinib, TAK-285, WHI-P154, daphnetin, PD168393, tyrphostin9, CNX-2006, AG-18, AZ5104, osimertinib, CL-387785, olmutinib, AZD3759, poziotinib, vandetanib, and necitumumab.

In various aspects, IFN signaling is Type I IFN signaling.

In various aspects, the agent that modulates IFN signaling is an interferon blocking antibody or an interferon neutralizing antibody. In a further aspect, the agent that modulates IFN signaling is an IFN inhibitor. Examples of IFN inhibitors include, but are not limited to, anifrolumab.

In various aspects, the agent that modulates EGFR signaling is an EGFR inhibitor and wherein the agent that modulates IFN signaling is an IFN inhibitor. In a further aspect, the agent that modulates EGFR signaling is erlotinib and wherein the agent that modulates IFN signaling is anifrolumab.

In various aspects, the agent that modulates EGFR signaling and the agent that modulates IFN signaling are co-formulated. In a further aspect, the agent that modulates EGFR signaling and the agent that modulates IFN signaling are co-packaged.

In various aspects, the agent that modulates EGFR signaling and the agent that modulates IFN signaling are administered concurrently. In a further aspect, the agent that modulates EGFR signaling and the agent that modulates IFN signaling are not administered concurrently.

In various aspects, the agent that modulates EGFR signaling is erlotinib and the agent that modulates IFN signaling is anifrolumab.

In various aspects, the agent that modulates EGFR signaling and the agent that modulates IFN signaling are co-formulated. In a further aspect, the agent that modulates EGFR signaling and the agent that modulates IFN signaling are co-packaged.

In a further aspect, the cancer is selected from a sarcoma, a carcinoma, a hematological cancer, a solid tumor, breast cancer, cervical cancer, gastrointestinal cancer, colorectal cancer, brain cancer, skin cancer, prostate cancer, ovarian cancer, thyroid cancer, testicular cancer, pancreatic cancer, liver cancer, endometrial cancer, melanoma, a glioma, leukemia, lymphoma, chronic myeloproliferative disorder, myelodysplastic syndrome, myeloproliferative neoplasm, non-small cell lung carcinoma, and plasma cell neoplasm (myeloma).

In a further aspect, the cancer is a solid tumor.

In a further aspect, the agent is a chemotherapeutic agent. In yet a further aspect, the chemotherapeutic agent is selected from an alkylating agent, an antimetabolite agent, an antineoplastic antibiotic agent, a mitotic inhibitor agent, and a mTor inhibitor agent.

In various aspects, the antineoplastic antibiotic agent is selected from doxorubicin, mitoxantrone, bleomycin, daunorubicin, dactinomycin, epirubicin, idarubicin, plicamycin, mitomycin, pentostatin, and valrubicin, or a pharmaceutically acceptable salt thereof.

In various aspects, the antimetabolite agent is selected from gemcitabine, 5-fluorouracil, capecitabine, hydroxyurea, mercaptopurine, pemetrexed, fludarabine, nelarabine, cladribine, clofarabine, cytarabine, decitabine, pralatrexate, floxuridine, methotrexate, and thioguanine, or a pharmaceutically acceptable salt thereof.

In various aspects, the alkylating agent is selected from carboplatin, cisplatin, cyclophosphamide, chlorambucil, melphalan, carmustine, busulfan, lomustine, dacarbazine, oxaliplatin, ifosfamide, mechlorethamine, temozolomide, thiotepa, bendamustine, and streptozocin, or a pharmaceutically acceptable salt thereof.

In various aspects, the mitotic inhibitor agent is selected from irinotecan, topotecan, rubitecan, cabazitaxel, docetaxel, paclitaxel, etopside, vincristine, ixabepilone, vinorelbine, vinblastine, and teniposide, or a pharmaceutically acceptable salt thereof.

In various aspects, the mTor inhibitor agent is selected from everolimus, siroliumus, and temsirolimus, or a pharmaceutically acceptable salt, hydrate, solvate, or polymorph thereof.

In various aspects, the agent that modulates EGFR signaling and the agent that modulates IFN signaling are administered sequentially. In a further aspect, the agent that modulates EGFR signaling and the agent that modulates IFN signaling are administered simultaneously.

In various aspects, the agent that modulates EGFR signaling and the chemotherapeutic agent are administered sequentially. In a further aspect, the agent that modulates EGFR signaling and the chemotherapeutic agent are administered simultaneously.

In various aspects, the agent that modulates IFN signaling and the chemotherapeutic agent are administered sequentially. In a further aspect, the agent that modulates IFN signaling and the chemotherapeutic agent are administered simultaneously.

In various aspects, the agent that modulates EGFR signaling, the agent that modulates IFN signaling, and the chemotherapeutic agent are administered sequentially. In a further aspect, the agent that modulates EGFR signaling, the agent that modulates IFN signaling, and the chemotherapeutic agent are administered simultaneously.

The kits can also comprise compounds and/or products co-packaged, co-formulated, and/or co-delivered with other components. For example, a drug manufacturer, a drug reseller, a physician, a compounding shop, or a pharmacist can provide a kit comprising a disclosed compound and/or product and another component for delivery to a patient.

It is understood that the disclosed kits can be prepared from the disclosed compounds, products, and pharmaceutical compositions. It is also understood that the disclosed kits can be employed in connection with the disclosed methods of using.

The foregoing description illustrates and describes the disclosure.

### F. REFERENCES

Honda, K. & Taniguchi, T. IRFs: master regulators of signalling by Toll-like receptors and cytosolic pattern-recognition receptors. Nat Rev Immunol 6, 644-658, doi:nri1900 [pii] 10.1038/nri1900 (2006).

Au, W. C., Moore, P. A., Lowther, W., Juang, Y. T. & Pitha, P. M. Identification of a member of the interferon regulatory factor family that binds to the interferon-stimulated response element and activates expression of interferon-induced genes. Proc Natl Acad Sci U S A 92, 11657-11661 (1995).

Liu, S. et al. Phosphorylation of innate immune adaptor proteins MAVS, STING, and TRIF induces IRF3 activation. Science 347, aaa2630, doi:10.1126/science.aaa2630 (2015).

Negishi, H., Taniguchi, T. & Yanai, H. The Interferon (IFN) Class of Cytokines and the IFN Regulatory Factor (IRF) Transcription Factor Family. Cold Spring Harb Perspect Biol 10, doi:10.1101/cshperspect.a028423 (2018).

Yoneyama, M., Suhara, W. & Fujita, T. Control of IRF-3 activation by phosphorylation. J Interferon Cytokine Res 22, 73-76, doi:10.1089/107999002753452674 (2002).

Fitzgerald, K. A. et al. IKKepsilon and TBK1 are essential components of the IRF3 signaling pathway. Nat Immunol 4, 491-496, doi:10.1038/ni921 [pii] (2003).

Ivashkiv, L. B. & Donlin, L. T. Regulation of type I interferon responses. Nat Rev Immunol 14, 36-49, doi:10.1038/nri3581 (2014).

Budhwani, M., Mazzieri, R. & Dolcetti, R. Plasticity of Type I Interferon-Mediated Responses in Cancer Therapy: From Anti-tumor Immunity to Resistance. Front Oncol 8, 322, doi:10.3389/fonc.2018.00322 (2018).

Snell, L. M., McGaha, T. L. & Brooks, D. G. Type I Interferon in Chronic Virus Infection and Cancer. Trends Immunol 38, 542-557, doi:10.1016/j.it.2017.05.005 (2017).

Zitvogel, L., Galluzzi, L., Kepp, O., Smyth, M. J. & Kroemer, G. Type I interferons in anticancer immunity. Nat Rev Immunol 15, 405-414, doi: 10.1038/nri3845 (2015).

Trinchieri, G. Type I interferon: friend or foe? J Exp Med 207, 2053-2063, doi: 10.1084/jem.20101664 (2010).

Dunn, G. P. et al. A critical function for type I interferons in cancer immunoediting. Nat Immunol 6, 722-729, doi:10.1038/ni1213 (2005).

Ye, Z. et al. Prevalent Homozygous Deletions of Type I Interferon and Defensin Genes in Human Cancers Associate with Immunotherapy Resistance. Clin Cancer Res 24, 3299-3308, doi:10.1158/1078-0432.CCR-17-3008 (2018).

Rusch, V. et al. Differential expression of the epidermal growth factor receptor and its ligands in primary non-small cell lung cancers and adjacent benign lung. Cancer Res 53, 2379-2385 (1993).

Chong, C. R. & Janne, P. A. The quest to overcome resistance to EGFR-targeted therapies in cancer. Nature medicine 19, 1389-1400, doi:10.1038/nm.3388 (2013).

Liu, Q. et al. EGFR-TKIs resistance via EGFR-independent signaling pathways. Mol Cancer 17, 53, doi:10.1186/s12943-018-0793-1 (2018).

Dutu, T. et al. Differential expression of biomarkers in lung adenocarcinoma: a comparative study between smokers and never-smokers. Ann Oncol 16, 1906-1914, doi:10.1093/annonc/mdi408 (2005).

Sharma, S. V., Bell, D. W., Settleman, J. & Haber, D. A. Epidermal growth factor receptor mutations in lung cancer. Nat Rev Cancer 7, 169-181, doi: 10.1038/nrc2088 (2007).

Volante, M. et al. Epidermal growth factor ligand/receptor loop and downstream signaling activation pattern in completely resected nonsmall cell lung cancer. Cancer 110, 1321-1328, doi:10.1002/cncr.22903 (2007).

Hsieh, E. T., Shepherd, F. A. & Tsao, M. S. Co-expression of epidermal growth factor receptor and transforming growth factor-alpha is independent of ras mutations in lung adenocarcinoma. Lung cancer 29, 151-157 (2000).

Guo, G. et al. Ligand-Independent EGFR Signaling. Cancer Res 75, 3436-3441, doi:10.1158/0008-5472.CAN-15-0989 (2015).

Chakraborty, S. et al. Constitutive and ligand-induced EGFR signalling triggers distinct and mutually exclusive downstream signalling networks. Nat Commun 5, 5811, doi:10.1038/ncomms6811 (2014).

Endres, N. F. et al. Conformational coupling across the plasma membrane in activation of the EGF receptor. Cell 152, 543-556, doi:S0092-8674(12)01554-1 [pii] 10.1016/j.cell.2012.12.032 (2013).

Yu, H. A. et al. Analysis of tumor specimens at the time of acquired resistance to EGFR-TKI therapy in 155 patients with EGFR-mutant lung cancers. Clin Cancer Res 19, 2240-2247, doi:10.1158/1078-0432.CCR-12-2246 (2013).

Engelman, J. A. et al. MET amplification leads to gefitinib resistance in lung cancer by activating ERBB3 signaling. Science 316, 1039-1043, doi:1141478 [pii] 10.1126/science.1141478 (2007).

Sun, C. & Bernards, R. Feedback and redundancy in receptor tyrosine kinase signaling: relevance to cancer therapies. Trends in biochemical sciences 39, 465-474, doi:10.1016/j.tibs.2014.08.010 (2014).

Lee, H. J. et al. Drug resistance via feedback activation of Stat3 in oncogene-addicted cancer cells. Cancer Cell 26, 207-221, doi:10.1016/j.ccr.2014.05.019 (2014).

Blakely, C. M. et al. NF-kappaB-activating complex engaged in response to EGFR oncogene inhibition drives tumor cell survival and residual disease in lung cancer. Cell Rep 11, 98-110, doi:10.1016/j.celrep.2015.03.012 (2015).

Gong, K. et al. TNF-driven adaptive response mediates resistance to EGFR inhibition in lung cancer. J Clin Invest 128, 2500-2518, doi:10.1172/JCI96148 (2018).

Guo, G. et al. A TNF-JNK-Axl-ERK signaling axis mediates primary resistance to EGFR inhibition in glioblastoma. Nat Neurosci 20, 1074-1084, doi:10.1038/nn.4584 (2017).

Furie, R. et al. Anifrolumab, an Anti-Interferon-alpha Receptor Monoclonal Antibody, in Moderate-to-Severe Systemic Lupus Erythematosus. Arthritis Rheumatol 69, 376-386, doi:10.1002/art.39962 (2017).

Khodarev, N. N. et al. STAT1 is overexpressed in tumors selected for radioresistance and confers protection from radiation in transduced sensitive cells. Proc Natl Acad Sci USA 101, 1714-1719, doi:10.1073/pnas.0308102100 (2004).

Khodarev, N. N. et al. Signal transducer and activator of transcription 1 regulates both cytotoxic and prosurvival functions in tumor cells. Cancer Res 67, 9214-9220, doi:10.1158/0008-5472.CAN-07-1019 (2007).

Tsai, M. H. et al. Gene expression profiling of breast, prostate, and glioma cells following single versus fractionated doses of radiation. Cancer Res 67, 3845-3852, doi:10.1158/0008-5472.CAN-06-4250 (2007).

Weichselbaum, R. R. et al. An interferon-related gene signature for DNA damage resistance is a predictive marker for chemotherapy and radiation for breast cancer. Proc Natl Acad Sci U S A 105, 18490-18495, doi:10.1073/pnas.0809242105 (2008).

Cheon, H. et al. IFNbeta-dependent increases in STAT1, STAT2, and IRF9 mediate resistance to viruses and DNA damage. EMBO J 32, 2751-2763, doi:10.1038/emboj.2013.203 (2013).

Duarte, C. W. et al. Expression signature of IFN/STAT1 signaling genes predicts poor survival outcome in glioblastoma multiforme in a subtype-specific manner. PLoS One 7, e29653, doi: 10.1371/journal .pone.0029653 (2012).

Rickardson, L. et al. Identification of molecular mechanisms for cellular drug resistance by combining drug activity and gene expression profiles. Br J Cancer 93, 483-492, doi:10.1038/sj.bjc.6602699 (2005).

Meissl, K., Macho-Maschler, S., Muller, M. & Strobl, B. The good and the bad faces of STAT1 in solid tumours. Cytokine 89, 12-20, doi:10.1016/j.cyto.2015.11.011 (2017).

Lazzari, E. & Meroni, G. TRIM32 ubiquitin E3 ligase, one enzyme for several pathologies: From muscular dystrophy to tumours. Int J Biochem Cell Biol 79, 469-477, doi:10.1016/j.biocel.2016.07.023 (2016).

Zhang, J., Hu, M. M., Wang, Y. Y. & Shu, H. B. TRIM32 protein modulates type I interferon induction and cellular antiviral response by targeting MITA/STING protein for K63-linked ubiquitination. J Biol Chem 287, 28646-28655, doi:10.1074/jbc.M112.362608 (2012).

Tu, D. et al. Structure and ubiquitination-dependent activation of TANK-binding kinase 1. Cell Rep 3, 747-758, doi:10.1016/j.celrep.2013.01.033 (2013).

Song, G. et al. E3 ubiquitin ligase RNF128 promotes innate antiviral immunity through K63-linked ubiquitination of TBK1. Nat Immunol 17, 1342-1351, doi:10.1038/ni.3588 (2016).

Wang, L., Li, S. & Dorf, M. E. NEMO binds ubiquitinated TANK-binding kinase 1 (TBK1) to regulate innate immune responses to RNA viruses. PLoS One 7, e43756, doi:10.1371/joumal.pone.0043756 (2012).

Chow, K. T., Gale, M., Jr. & Loo, Y. M. RIG-I and Other RNA Sensors in Antiviral Immunity. Annu Rev Immunol 36, 667-694, doi:10.1146/annurev-immunol-042617-053309 (2018).

Kitajima, S. et al. Suppression of STING Associated with LKB1 Loss in KRAS-Driven Lung Cancer. Cancer Discov 9, 34-45, doi:10.1158/2159-8290.CD-18-0689 (2019).

Terai, H. et al. ER Stress Signaling Promotes the Survival of Cancer "Persister Cells" Tolerant to EGFR Tyrosine Kinase Inhibitors. Cancer Res 78, 1044-1057, doi:10.1158/0008-5472.CAN-17-1904 (2018).

Ye, M. et al. Activation of the Aryl Hydrocarbon Receptor Leads to Resistance to EGFR TKIs in Non-Small Cell Lung Cancer by Activating Src-mediated Bypass Signaling. Clin Cancer Res 24, 1227-1239, doi:10.1158/1078-0432.CCR-17-0396 (2018).

Liu, Y. et al. Tumor-Repopulating Cells Induce PD-1 Expression in CD8(+) T Cells by Transferring Kynurenine and AhR Activation. Cancer Cell 33, 480-494 e487, doi:10.1016/j.ccell.2018.02.005 (2018).

Akbay, E. A. et al. Activation of the PD-1 pathway contributes to immune escape in EGFR-driven lung tumors. Cancer Discov 3, 1355-1363, doi:10.1158/2159-8290.CD-13-0310 (2013).

Thungappa, S. et al. Immune checkpoint inhibitors in lung cancer: the holy grail has not yet been found. ESMO Open 2, e000162, doi:10.1136/esmoopen-2017-000162 (2017).

Gainor, J. F. et al. EGFR Mutations and ALK Rearrangements Are Associated with Low Response Rates to PD-1 Pathway Blockade in Non-Small Cell Lung Cancer: A Retrospective Analysis. Clin Cancer Res 22, 4585-4593, doi:10.1158/1078-0432.CCR-15-3101 (2016).

Muhlbauer, M. et al. PD-L1 is induced in hepatocytes by viral infection and by interferon-alpha and -gamma and mediates T cell apoptosis. J Hepatol 45, 520-528, doi:10.1016/j.jhep.2006.05.007 (2006).

Bald, T. et al. Immune cell-poor melanomas benefit from PD-1 blockade after targeted type I IFN activation. Cancer Discov 4, 674-687, doi:10.1158/2159-8290.CD-13-0458 (2014).

Yoshida, T. et al. Tyrosine phosphoproteomics identifies both codrivers and cotargeting strategies for T790M-related EGFR-TKI resistance in non-small cell lung cancer. Clin Cancer Res 20, 4059-4074, doi:10.1158/1078-0432.CCR-13-1559 (2014).

Zhang, Z. et al. Activation of the AXL kinase causes resistance to EGFR-targeted therapy in lung cancer. Nat Genet 44, 852-860, doi:10.1038/ng.2330 (2012).

Akbay, E. A. & Kim, J. Autochthonous murine models for the study of smoker and never-smoker associated lung cancers. Transl Lung Cancer Res 7, 464-486, doi:10.21037/tlcr.2018.06.04 (2018).

Kruspig, B. et al. The ERBB network facilitates KRAS-driven lung tumorigenesis. Sci Transl Med 10, doi:10.1126/scitranslmed.aao2565 (2018).

Moll, H. P. et al. Afatinib restrains K-RAS-driven lung tumorigenesis. Sci Transl Med 10, doi:10.1126/scitranslmed.aao2301 (2018).

Chandarlapaty, S. et al. AKT inhibition relieves feedback suppression of receptor tyrosine kinase expression and activity. Cancer Cell 19, 58-71, doi:10.1016/j.ccr.2010.10.031 (2011).

Prahallad, A. et al. Unresponsiveness of colon cancer to BRAF(V600E) inhibition through feedback activation of EGFR. Nature 483, 100-103, doi:10.1038/nature10868 (2012).

Duncan, J. S. et al. Dynamic reprogramming of the kinome in response to targeted MEK inhibition in triple-negative breast cancer. Cell 149, 307-321, doi:10.1016/j.cell.2012.02.053 (2012).

Corcoran, R. B. et al. EGFR-mediated re-activation of MAPK signaling contributes to insensitivity of BRAF mutant colorectal cancers to RAF inhibition with vemurafenib. Cancer Discov 2, 227-235, doi:10.1158/2159-8290.CD-11-0341 (2012).

Sun, C. et al. Intrinsic resistance to MEK inhibition in KRAS mutant lung and colon cancer through transcriptional induction of ERBB3. Cell Rep 7, 86-93, doi:10.1016/j.celrep.2014.02.045 (2014).

Fallahi-Sichani, M. et al. Systematic analysis of BRAF(V600E) melanomas reveals a role for JNK/c-Jun pathway in adaptive resistance to drug-induced apoptosis. Mol Syst Biol 11, 797, doi:10.15252/msb.20145877 (2015).

Puliyappadamba V.T. et al. Opposing effect of EGFRwt on EGFRvIII mediated NF-kappaB activation with RIP1 as a cell death switch. Cell Reports 4, 764-775. 2013.

Trudgian, D. C. et al. Comparative evaluation of label-free SINQ normalized spectral index quantitation in the central proteomics facilities pipeline. Proteomics 11, 2790-2797 (2011).

### G. EXAMPLES

Here, it is reported that EGFR inhibition reprograms cellular signaling and results in a remarkable cooptation of antiviral signaling pathways in NSCLC. This antiviral response mediates resistance to EGFR inhibition in both EGFR wild type and EGFR mutant NSCLC. NSCLC cells respond to EGFR inhibition with a rapid increase in Type I interferon levels and the IFN upregulation was detected in all NSCLC cell lines examined, in animal tumor tissue, and in archival tissue from patients. In EGFRwt expressing NSCLCs, the increase in IFNs is sufficient to protect cells from loss of EGFR signaling. In NSCLCs with EGFR-activating mutations the IFN driven adaptive response is only partially protective and observed after treatment with low concentrations of EGFR inhibitors. This is also true for other adaptive bypass signaling mechanisms such as STAT3, or TNF-NF-κB that are triggered by EGFR inhibition in EGFR mutant NSCLC and do not inhibit the initial clinical response in patients but may play a role in the development of secondary resistance. Importantly, exogenous IFNα or IFNβ via activation of STAT1 protects NSCLC cells with mutant EGFR activating from cell death resulting from EGFR inhibition, further supporting an important role for Type I interferons in mediating resistance to EGFR inhibition in NSCLC.

The adaptive response to inhibition of RTK pathways is broad and leads to substantial reprogramming of signaling pathways that attempt to restore homeostasis. However, targeted inhibition of one or a small number of pathways may cripple the adaptive response and overcome therapeutic resistance in such cancers. Here, it is shown that combined inhibition of EGFR and type I IFN signaling is highly effective in suppressing the growth of NSCLC tumors in multiple animal models.

Although most patients with EGFR activating mutations initially respond to EGFR TKIs, they inevitably develop resistance, implying the persistence of subsets of cancer cells. Primary or intrinsic resistance to inhibition of EGFRwt could occur because an adaptive response prevents cell death in response to EGFR inhibition. Currently the EGFRwt does not appear to be a useful target for treatment, because EGFR inhibition is ineffective in EGFRwt expressing NSCLC. However EGFRwt is widely expressed and recent studies suggest that targeting the EGFR signaling network inhibition may also hold promise in EGFRwt/KRas NSCLC. Here, it is proposed that the primary resistance to EGFR inhibition in EGFRwt NSCLC does not necessarily indicate that EGFR signaling is irrelevant to the malignant phenotype. Rather, EGFR inhibition may not work in because an adaptive survival mechanism triggered by EGFR inhibition negates its effect. A combined inhibition of EGFR+adaptive response either unmasks a requirement for EGFR signaling for survival and/or sets up synthetic lethal conditions.

EGFR inhibition results in an increase in Type I IFN levels via distinct mechanisms depending on whether EGFR is mutant or wild type. In EGFR mutant tumors, a RIGI-TRIM32-TBK1-IRF3 axis mediates induction of IFNs and resistance to EGFR inhibition. Here, it is shown that TRIM32, an E3-ubiquitin ligase, associates with TBK1 upon EGFR inhibition leading to K-63 linked ubiquitination of TBK1. TRIM32 is required for EGFR inhibition induced TBK1 and IRF3 phosphorylation and resistance to EGFR inhibition. In contrast, inhibition of EGFRwt tumors, upregulates Type I interferons via an NF-κB dependent pathway.

In a previous study it was demonstrated that inhibition of the EGFR in lung cancer cells resulted in a rapid increase in TNF secretion via an effect on TNF mRNA stability mediated by miR-21and leading to a NF-κB driven survival pathway that protected cells from a loss of EGFR signaling. Here, it is shown that EGFR inhibition results in a distinct adaptive mechanism that activates an anti-viral signaling pathway mediated by upregulation of Type I IFNs. A combined inhibition of EGFR and type I interferons using the clinically available antibody anifrolumab enhances the effectiveness of EGFR inhibition in EGFR mutant cells and is able to overcome the primary resistance of EGFRwt NSCLC including the subset with KRas mutant. Intriguingly, it was also found that EGFR inhibition may lead to an upregulation of PD-L1 via an interferon dependent pathway, providing a possible explanation for the failure of immunotherapy in EGFR mutant NSCLC, and the possibility that anifrolumab may render such tumors responsive to immunotherapy.

The data herein indicate that Type I IFN signaling is a major targetable mechanism of resistance to EGFR TKI inhibition in EGFR mutant and EGFRwt NSCLC. The chromosomal locus for the Type I interferon genes is 9p23.1, one of the most common sites for homozygous deletions of tumor suppressive genes. Homozygous deletion of Type I IFN genes has been reported in multiple tumor types and in about 10% of NSCLC, and correlates with a worse prognosis. However, the data herein indicate that in the context of EGFR inhibition, type I interferons mediate therapeutic resistance and confer a worse prognosis. Whether the combined effect of Type I IFN and EGFR inhibition is synergistic in highly resistant EGFRwt/KRas mutant models and in EGFR mutant models when a low concentration of erlotinib is used was explored. Together these findings provide a therapeutic opportunity. Targeting a biologically significant upregulation of Type I interferons upon EGFR inhibition could greatly expand the reach and impact of EGFR targeted treatment in NSCLC. Thus, inhibiting the EGFR with a combination of TKI plus an IFN inhibitor such as the FDA approved anifrolumab may be effective in the treatment of NSCLCs that express EGFRwt. In tumors with EGFR activating mutations, a combined treatment with EGFR and IFN inhibition may result in a more effective elimination of tumor cells during the initial treatment and perhaps eliminate or delay secondary resistance to TKI treatment, and may also be useful in treating secondary resistance.

The following examples are put forth so as to provide those of ordinary skill in the art with a complete disclosure and description of how the compounds, compositions, articles, devices and/or methods claimed herein are made and evaluated, and are intended to be purely exemplary of the invention and are not intended to limit the scope of what the inventors regard as their invention. Efforts have been made to ensure accuracy with respect to numbers (e.g., amounts, temperature, etc.), but some errors and deviations should be accounted for. Unless indicated otherwise, parts are parts by weight, temperature is in °C or is at ambient temperature, and pressure is at or near atmospheric.

The Examples are provided herein to illustrate the invention, and should not be construed as limiting the invention in any way. Examples are provided herein to illustrate the invention and should not be construed as limiting the invention in any way.

### 1. MATERIALS AND METHODS

### a. CELL LINES

A549 and U87MG cells were purchased from American Type Culture Collection (ATCC). HCC827/ER3, HCC827/ER4(A), and HCC827/ER5⁵⁶ were obtained from Dr. Trever Bivona, University of California (San Francisco, CA). HCC827/ER4(B)⁵⁵ and were obtained from Dr. Eric Haura, Moffitt Cancer Center (Tampa, FL). All other NSCLC cell lines were from the Hamon Center for Therapeutic Oncology Research at the University of Texas Southwestern Medical Center. NSCLC cells were cultured in RPMI-1640 containing 5% FBS, and U87MG in DMEM medium with 10% FBS. Cell lines were authenticated by DNA fingerprints for cell-line individualization using Promega StemElite ID system, a short tandem repeat (STR)-based assay, at UT Southwestem genomics core. Cells were tested for mycoplasma contamination using an e-Myco kit (Boca Scientific).

### b. WESTERN BLOT, ANTIBODIES, PLASMIDS AND REAGENTS

Western blot and immunoprecipitation were performed according to standard protocols. Western blot results are representative of at least 3 independent experiments. EGFR (06-847) antibody was from EMD Millipore (Billerica, MA); p-EGFR (Tyr1068) (2236), p-TBK1 (Ser172) (5483), TBK1 (3504), IKKε (2905), IRF3 (11904), K63-Ub (12930), RIG-I (3743), STAT1 (9172), p-STAT1 (Tyr701) (9167), AhR (83200), LAMIN A/C (4777), STING (3337), p-STING (Ser366) (19781), PD1 (86163), PD-L1 (13684), PD-L2 (82723) and IrBα (4814) antibodies were from Cell Signaling Technology (Danvers, MA); IFNAR1 (sc-7391), IFNGR1 (sc-12755), TNFR1 (sc-8346), and β-Actin (sc-47778) were from Santa Cruz Biotechnology (Dallas, TX); TRIM32 (Mab6515) antibody was from R&D (Minneapolis, MN); p-IRF3 (Ser386) (ab76493) antibody was from Abcam (Cambridge, MA).

Recombinant human IFNα1 (z02866) was purchased from Genscript (Piscataway, NJ); IFNβ1 (300-02BC) and TNFα (300-01A) was obtained from PeproTech (Rocky Hill, NJ). Mouse anti-mouse IFNAR1 antibody (BE0241) was purchased from Bioxcell (West Lebanon, NH). Anifrolumab, an anti-IFNAR1 antibody was obtained from Creative-Biolabs (Shirley, NY) (TAB-722). Entanercept (Enbrel), a fusion protein of TNF receptor and IgG, was purchased from Mckesson Medical Supply (San Francisco CA). NF-κB inhibitor BMS-345541 was obtained from MilliporeSigma (Burlington, MA). LPS (19661), TBK1 inhibitor BX795, and EGFR inhibitor erlotinib and afatinib for *in vitro* studies were obtained from Cayman Chemical (Ann Arbor, MI). Erlotinib for animal treatment was purchased from LC Laboratories (Woburn, MA). pCMV2-IRF3 plasmid was a kind gift from Dr. John Hiscott (McGill University, Montreal, Canada). NFκB luciferase reporter plasmid was provided by Dr. Ezra Burstein (UT Southwestem).

### C. CELL VIABILITY ASSAY

Cell viability assays were conducted with AlamarBlue Cell Viability Reagent from ThermoFisher (Waltham, MA), following the manufacturer's protocol. Cells were cultured in Coming (Corning, NY) 96-well black plates with clear bottom and detected by the POLARstar Omega Microplate Reader (BMG LABTECH) (excitation at 544 nm and emission at 590 nm). At least 3 independent experiments were done. In oncogene addicted EGFR mutant cells, a lower dose of erlotinib 0.01 µM was used in cell viability assays to detect synergistic effects of combination therapy while a higher dose of 0.1 µM was used to detect the protection from erlotinib-induced cell death when IFNα or IFNβ were used.

### d. LUCIFERASE ASSAYS

Cells were plated in 48 well dishes followed by plasmid transfection with two reporters detecting activation of IRF3, ISRE-luciferase reporter or IFI27-ISRE luciferase reporter²², using lipofectamine 2000. A dual-luciferase reporter assay system was used according to the instructions of the manufacturer (Promega, Madison WI). Firefly luciferase activity was measured in the POLARstar Omega Microplate Reader (BMG LABTECH) and normalized based on Renilla luciferase activity. Three independent experiments were done in triplicate.

### e. REAL-TIME PCR

Total RNA was isolated by TRIzol Reagent (Fisher Scientific). cDNA Reverse Transcription was performed by using High-Capacity cDNA Reverse Transcription Kit (Applied Biosystems). PCR primers were synthesized by IDT (Coralville, IA). Each PCR reaction was carried out in triplicate in a 20µl volume using SYBR Green Master Mix (Applied Biosystems) for 15 minutes at 95 °C for initial denaturing, followed by 40 cycles of 95 °C for 15 s and 60 °C for 60s in ViiA 7 Real-Time PCR System (Applied Biosystems). At least three independent experiments were done. Values for each gene were normalized to expression levels of ACTB (β-Actin) mRNA. Primer sequences were as follows. IFNA1: 5'-GTGAGGAAATACTTCCAAAGAATCAC-3' (forward) (SEQ ID NO: 1), 5'-TCTCATGATTTCTGCTCTGACAA-3' (reverse) (SEQ ID NO:2); IFNB1: 5'-AGCTGAAGCAGTTCCAGAAG-3' (forward) (SEQ ID NO:3), 5'-AGTCTCATTCCAGCCAGTGC-3' (reverse) (SEQ ID NO:4); IFNG: 5'-GGGTAACTGACTTGAATGTCC-3' (forward) (SEQ ID NO:5), 5'-TTTTCGCTTCCCTGTTTTAG-3' (reverse) (SEQ ID NO:6); ACTB: 5'-CATGTACGTTGCTATCCAGGC-3' (forward) (SEQ ID NO:7), 5'-CTCCTTAATGTCACGCACGAT-3' (reverse) (SEQ ID NO:8).

### f. RNASEQ

RNA sequencing was performed at UT Southwestern Genomics and Microarray Core Facility. Total RNA was isolated by TRIzol Reagent (Fisher Scientific). RNA quality was determined by Agilent 2100 Bioanalyzer (RIN > 8), and quantity was measured by Qubit fluorometer. 1 µg RNA was then prepared with the TruSeq Stranded Total RNA LT Sample Prep Kit from Illumina. Poly-A RNA (mRNAseq) is purified and fragmented before strand specific cDNA synthesis. cDNA are then a-tailed and indexed adapters are ligated. After adapter ligation, samples are PCR amplified and purified with Ampure XP beads, then run on the Illumina NextSeq 500/550 system (Kits V2.5) with 75 bp single end reads to product about 25 Million reads per sample.

Sequencing data were further processed at UT Southwestem Bioinformatics Core Facility. Differential expression was analyzed by DESeq2. Pathway analysis was performed based on Gene Set Enrichment Analysis (GSEA, http://software. broadinstitute. org/gsea/index.jsp).

### g. ELISA (ENZYME-LINKED IMMUNOSORBENT ASSAY)

To detect IFNα and IFNβ levels in medium, cells were cultured in serum free medium and treated with indicated drugs for 48 hours. Supernatant was then collected and concentrated using a Pierce protein concentrator (Thermo-Fisher). To test IFNα and IFNβ in lysates, cell and tumor lysates were extracted using RIPA buffer. Total protein concentrations were determined by Pierce BCA Protein Assay Kit (Fisher Scientific). Then, the levels of IFNα and IFNβ protein were measured by ELISA using human IFNα ELISA kit (41100) and human IFNβ ELISA kit (41440), from PBL Assay Science (Piscataway, NJ) according to the manufacturer's protocol.

### h. IMMUNOFLUORESCENT STAINING

Cells were cultured on coverslips in plates, after treatment cells were fixed with 4% Paraformaldehyde (PFA) followed by cell membrane permeabilization in 0.5 % Triton X-100/PBS and blocked in 1% BSA/PBS. The primary anti-AhR antibody (Santa Cruz Biotechnology, sc-101104, 1:200) was incubated at 4 °C overnight, followed with Alexa555-conjugated secondary antibodies (Cell Signaling, 4413, 1:500) at room temperature for 2 hours. Cell nuclei were counterstained with DAPI (Invitrogen) at 0.1 µg/ml. The cells were examined using fluorescence microscope.

### i. MASS SPECTROMETRY

HCC827 cells were treated with 0.1 µM erlotinib for 0, 2, 6, and 24 hours. Cell lysates were immunoprecipitated with TBK1 antibody. Antibody enriched protein samples were run on SDS-PAGE gels and submitted to UT Southwestern Proteomics Core Facility for Mass Spectrometry. Protein gel pieces were digested overnight with trypsin (Pierce) following reduction and alkylation with DTT and iodoacetamide (Sigma-Aldrich). The samples then underwent solid-phase extraction cleanup with Oasis HLB microelution plates (Waters) and the resulting samples were analyzed by LC/MS/MS, using an Orbitrap Fusion Lumos mass spectrometer (Thermo Electron) coupled to an Ultimate 3000 RSLC-Nano liquid chromatography systems (Dionex). Samples were injected onto a 75 µm i.d., 50-cm long EasySpray column (Thermo), and eluted with a gradient from 1-28% buffer B over 60 min. Buffer A contained 2% (v/v) ACN and 0.1% formic acid in water, and buffer B contained 80% (v/v) ACN, 10% (v/v) trifluoroethanol, and 0.1% formic acid in water. The mass spectrometer operated in positive ion mode with a source voltage of 2.55 kV and an ion transfer tube temperature of 275 °C. MS scans were acquired at 120,000 resolution in the Orbitrap and up to 10 MS/MS spectra were obtained in the ion trap for each full spectrum acquired using higher-energy collisional dissociation (HCD) for ions with charges 2-7. Dynamic exclusion was set for 25 s after an ion was selected for fragmentation.

Raw MS data files were converted to a peak list format and analyzed using the central proteomics facilities pipeline (CPFP), version 2.0.3. Peptide identification was performed using the X!Tandem and open MS search algorithm (OMSSA) search engines against the human protein database from Uniprot, with common contaminants and reversed decoy sequences appended. Fragment and precursor tolerances of 20 ppm and 0.6 Da were specified, and three missed cleavages were allowed. Carbamidomethylation of Cys was set as a fixed modification and oxidation of Met was set as a variable modification. Label-free quantitation of proteins across samples was performed using SINQ normalized spectral index Software⁶².

### j. RNAI

siRNA knockdown was conducted with siRIG-I(sc-61480), siTRIM32(sc-61714), siTBK1(sc-39058), siIRF3(sc-35710), siIFNAR1(sc-35637), siSTAT1(sc-44123), siIFNGR1(sc-29357), siTNFR1(sc-29507), siSTING(sc-92042), and Control siRNA (sc-37007), purchased from Santa Cruz Biotechnology (Dallas, TX).

Lentiviruses for establishing stable cell lines used for xenograft experiments were obtained from Santa Cruz Biotechnology (Dallas, TX), including shTBK1(sc-39058-V), shIRF3(sc-35710-V), shIFNAR1(sc-35637-V) Human Lentiviral Particles, and Control shRNA Lentiviral Particles-A(sc-108080). GFP adenovirus (1060) and IkBα (S32A/S36A)-DN (Dominant-negative) adenovirus (1028) were obtained from Vector Biolabs (Malvem, PA). A Multiplicity of infection (MOI) of 10 was used in the experiments. Cells were infected with shRNA lentiviral particles following the manufacturer's protocol and 0.6 µg/mL puromycin was added for selecting stable clones.

### k. ANIMAL EXPERIMENTS

Cell lines: Female nude mice (088) at four- to six-week-old were purchased from Charles River Laboratories (Wilmington, MA). One million A549 cells (1x10⁶), or two million (2x10⁶) HCC827 cells (including stable cell lines derived) were injected subcutaneously (s.c.) into the flanks of nude mice. About two weeks later, mice would develop subcutaneous tumors. The mice were randomly divided into indicated groups. Mice were treated with drugs using the doses described in the figure legends. For combination treatment, both drugs were given concurrently for indicated periods. Tumor dimensions were measured every 4 days and tumor volumes calculated by the formula: volume = 0.5 × length × width × width. Mice were sacrificed when tumors reached over 20 millimeter (mm) of length or after the indicated number of days.

Patient-derived xenograft (PDX): The NSCLC specimens (P0) for HCC4087 and HCC4190 PDXs were surgically resected from a patient diagnosed with NSCLC at UT Southwestern, after obtaining Institutional Review Board approval and informed consent. HCC4087 has KRAS G13C mutation but no EGFR activating mutations, HCC4190 harbors EGFR L858R mutation identified by Exome sequencing. 4 to 6 weeks old female NOD SCID mice (394) were purchased from Charles River Laboratories. The PDX tumor tissues were cut into small pieces (~20 mm3) and subcutaneously implanted in NOD SCID mice of serial generations (P1, P2, etc.). P4 tumor bearing SCID mice were used in this study.

LSL-Kras G12D mice (008179): were purchased from Jackson laboratories and the colony was expanded by breeding heterozygous LSL-Kras G12D mice with wildtype mice. Genotyping was performed per the protocol on Jackson website. Lung tumors were induced in mice carrying the LSL-Kras G12D allele with intranasal administration of 2.5 x 10⁸ PFU Adeno-CMV-Cre (University of Iowa). Treatments were initiated once the tumors were confirmed by with Magnetic resonance imaging (MRI) at about 10-12 weeks after tumor induction.

### 1. MRI IMAGING

MRI Imaging was conducted at UT Southwestern Mouse MRI Core, Advanced Imaging Research Center, using a 7T small animal MRI scanner (Bruker, Rheinstetten, Germany) equipped with a 40 mm quadrature Radiofrequency (RF) coil (ExtendMR LLC, Milpitas, CA). Under anesthesia by inhalation of 1.5 - 3% isoflurane mixed in with medical-grade oxygen via nose-cone, the animals were placed supine on a mouse holder, with a pneumatic respiratory sensor and electrocardiography (ECG) electrodes for cardiac sensing, head first with the lung centered with respect to the center of the RF coil. The mice's chests were shaved and conducting hydrogels were applied to optimize ECG contact between electrodes and mouse. All MRI acquisitions were gated using both cardiac and respiratory triggering. The bore temperature was kept at 23 ± 2 °C to assure adequate and constant heart rate. Two-dimensional (2D) scout images on three orthogonal planes (transverse, coronal and sagittal) were acquired to determine the positioning. Then, lower resolution gradient echo (T₁_FLASH) images were acquired on transverse plane to fine-adjust the slice position. Finally, higher resolution gradient echo images were recorded on the transverse plane, with the major parameters as follows: The repetition time (TR) = 200 ms (Note: the actual TR is changing according to ECG R-R interval, in range of 200 ms to 240 ms), the echo time (TE) = 1.966 ms, the flip angle (FA) = 45°, the number of average = 12, the field of view (FOV) = 32 x 32 mm², the matrix size = 256 x 256, the slice number = 17-21 (changed upon the mouse lung size), and the slice thickness = 1 mm without any gap. The image analyses were performed using ImageJ.

### m. PATIENT DATA

Before vs After treatment: Formalin fixed paraffin-embedded (FFPE) tissues from 23 NSCLC patients were obtained from The Jackson Laboratory or UT Southwestern according to IRB-approved protocols. Thirteen specimens were obtained from UT Southwestern and ten from The Jackson Laboratory. Thirteen patients had no EGFR TKI treatment, and ten patients had undergone EGFR TKI treatment.

To assess the effects of Interferons on Overall Survival we reviewed the medical records of NSCLC patients treated at UT Southwestern. 30 advanced (stages IIIB & IV) NSCLC patients harbored classical TKI-sensitive mutations, L858R or exon 19 deletion, but no T790M mutation at initial diagnosis, and all of them had TKI treatment history. Their FFPE tissues from initial diagnosis were collected for this study.

TCGA: Data were downloaded from https://portal.gdc.cancer.gov/. 42 TCGA-LUAD patients (any stages) with classical TKI-sensitive mutations, L858R or exon 19 deletion, but without T790M mutation, were achieved with their Copy Number Variation (CNV) and Survival data. 41/42 have RNAseq data.

### n. STUDY APPROVAL

All animal studies were done under IACUC-approved protocols at UT Southwestern and North Texas VA Medical Center (Dallas, Texas, USA). Patient tissues and medical records were obtained from UT Southwestern with IRB approval.

### o. STATISTICS AND REPRODUCIBILITY

Error bars represent the means ± S.E.M. of 3 independent experiments unless indicated otherwise. The combination effects *in vivo* and *in vitro* were analyzed by two-way or three-way ANOVA with Bonferroni's correction to adjust the significance level for multiple comparisons. One-way ANOVA with Dunnett's test was used to determine adjusted p value for comparison between control and more than one treated sample. The familywise error rate (FWER) was set at 0.05. Kaplan-Meier survival curves were constructed and compared by log-rank test and Gehan's test. The patient data comparison was shown as median ± IQR, analyzed by Kolmogorov-Smimov test. RNAseq data were analyzed by DESeq2 and GSEA. All other data were analyzed for significance between the indicated treated group and control group, with two-tailed two-sample Student's t-test. All analyses above were performed using GraphPad Prism 8 software. A P value or an adjusted P value for multiple comparison less than 0.05 was considered statistically significant (*P < 0.05, **P < 0.01, ***P < 0.001, ****P < 0.0001; #: p>0.05, not statistically significant).

The Reproducibility information is provided in Reporting Summary, including sample size predetermination, randomization, blinding, and replication. There are no data exclusions.

### 2. DATA AVAILABILITY

RNA-seq data that support the findings of this study have been deposited in the Sequence Read Archive (SRA) under accession code PRJNA593064.

Mass spectrometry data have been deposited in ProteomeXchange with the primary accession code PXD016558.

The human lung adenocarcinoma data were derived from the TCGA Research Network: http://cancergenome.nih. gov/.

Uncropped western blot images and raw digital data have been proved in Source Data.

Information regarding codes used in this study have been proved in Reporting Summary. They are either commercially available or open-source.

### 3. EGFR INHIBITION LEADS TO ACTIVATION OF TYPE I INTERFERON SIGNALING IN NSCLC

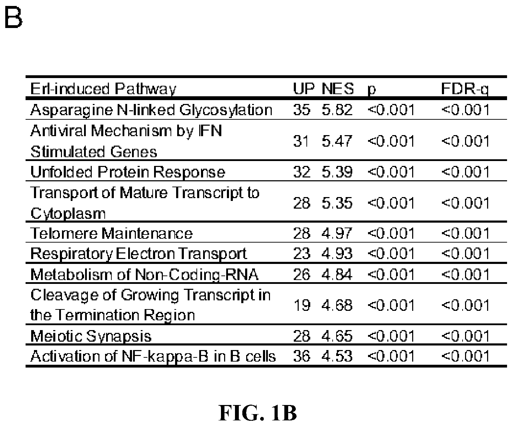

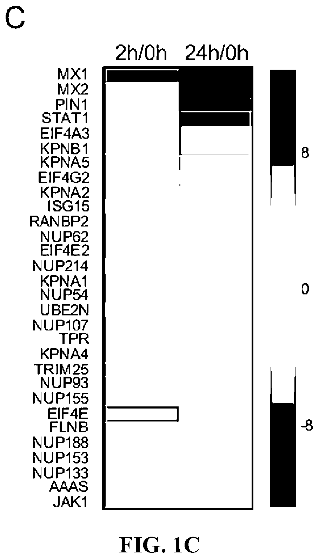

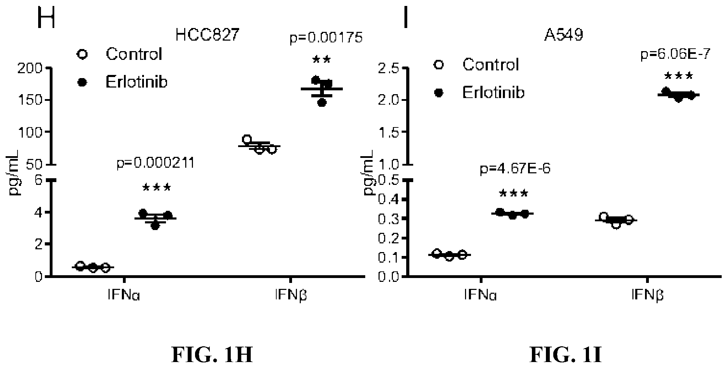

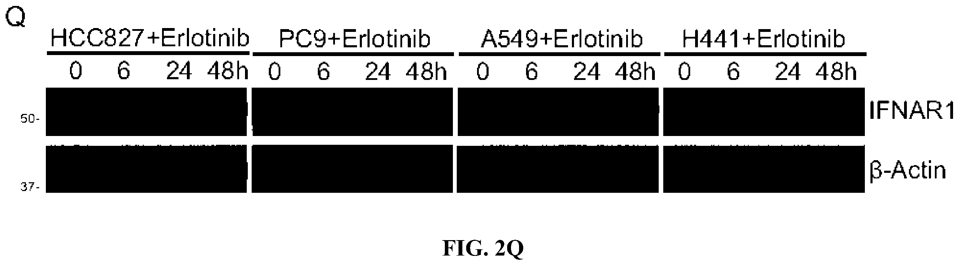

To better understand the early adaptive response to EGFR inhibition, RNA sequencing was undertaken in the *EGFRwt*/*KRas* mutant A549 cells following exposure to erlotinib. The transcriptional response to EGFR inhibition is quite broad and affects a large number of genes (FIG. 1A). Pathway analysis revealed that a Type I IFN gene signature was prominent among the signaling changes triggered by EGFR inhibition in these cells (FIG. 1B and FIG. 1C). EGFR inhibition in multiple NSCLC lines harboring EGFR mutant or EGFRwt with KRas mutation or other genetic alterations resulted in an upregulation of Type I interferons as determined by qPCR (FIG. 1D-G and FIG. 2A-X) and by ELISA (FIG. 1H, FIG. 1I, FIG. 3A, FIG. 3B, and FIG. 3K-Z). Upregulation of Type I IFNs was also found in HCC827 and A549 xenografts and in PDX models of EGFRwt/KRas mutant and EGFR mutant NSCLC when erlotinib was administered to tumor bearing mice (FIG. 3C-J). The level of type I IFN receptor was unchanged in response to erlotinib (FIG. 2Q). An upregulation of interferon gamma was also detected in response to erlotinib, but IFN gamma siRNA knockdown did not result in a synergy with EGFR inhibition in cell survival assays (FIG. 3AA-MM).

Referring to FIG. 1A, A549 cells were treated with 1 µM erlotinib for 0, 2, and 24 hours. Three biologically independent RNA samples per group were sequenced. R-package DESeq2 was used to calculate the fold-change and p-value (unadjusted). Up-regulated and down-regulated genes were identified by the cutoff of p<0.05. Volcano plot shows the distribution of differentially expressed genes.

Referring to FIG. 1B, pathway analysis was performed via Gene Set Enrichment Analysis (GSEA), from http://software.broadinstitute.org/gsea/index.jsp. The top erlotinib upregulated pathways are presented, ranked by normalized enrichment scores (NES), with numbers of upregulated genes and p-values.

Referring to FIG. 1C, the heatmap shows the up-regulated genes at 24 hours in the Reactome pathway "Antiviral mechanism by IFN-stimulated genes," representing mean values of normalized log-ratio between untreated and treated group.

Referring to FIG. 1D-G, HCC827 and A549 cells were treated by 0.1 or 1 µM erlotinib respectively, and then qPCR was performed to detect IFNα1 and IFNβ1 mRNA. ATCB (β-Actin) expression was the loading control.

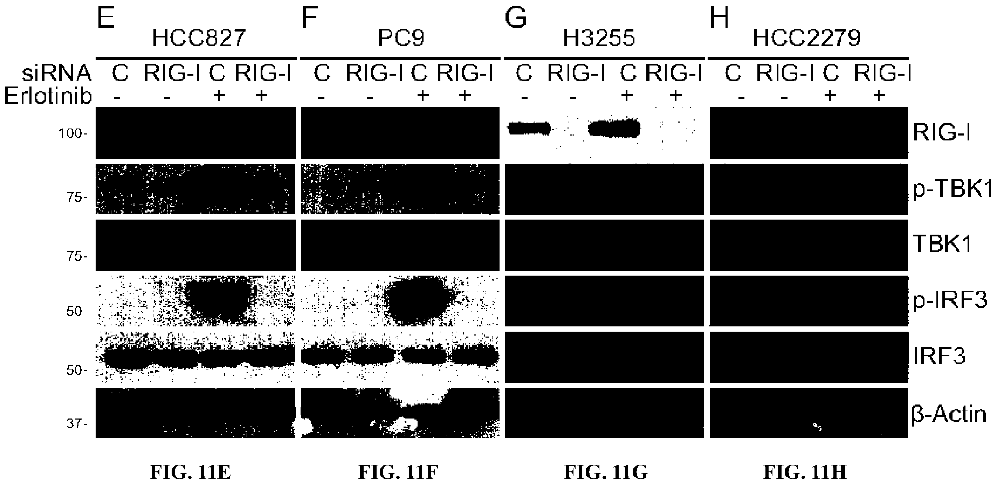

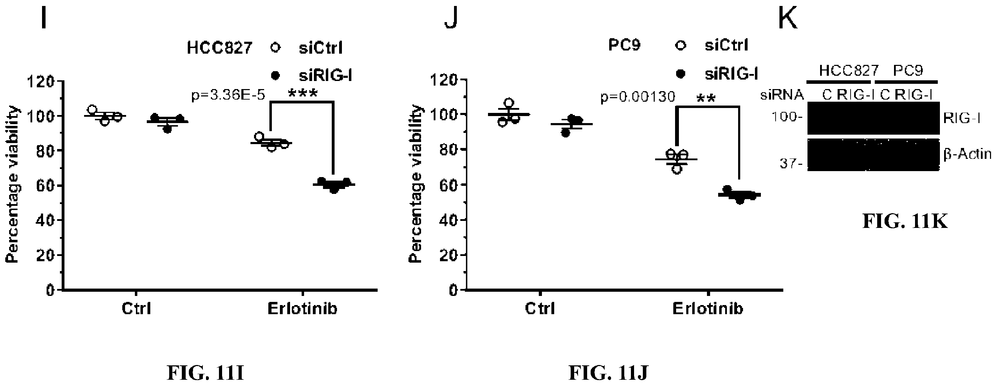

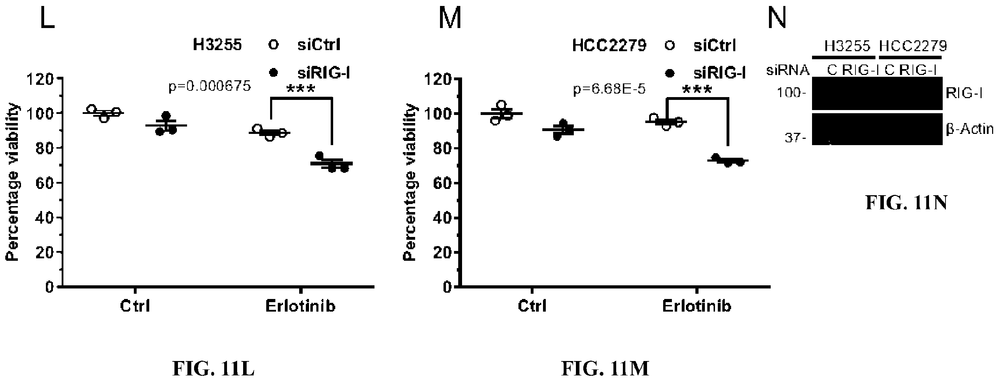

Referring to FIG. 1H and FIG. 11, HCC827 and A549 were treated with 0.1 µM or 1 µM Erlotinib for two days, the concentration of IFNα1 and IFNβ1 was measured in supernatants by ELISA.

Referring to FIG. 2A-H, four EGFR mutant cell lines, H3255, PC9, HCC2279, and H1650 were treated with 0.1 µM Erlotinib for the indicated time points. IFNα1 and IFNβ1 mRNA levels were detected by qPCR with β-Actin as the loading control.

Referring to FIG. 2I-P, a similar experiment was conducted in four EGFR wt cell lines: H441, H2122, H1373, and H1573, exposed to 1 µM erlotinib.

Referring to FIG. 2Q, two EGFR mutant cells (HCC827 and PC9), and two EGFRwt cell lines (A549 and H441) were treated with 0.1 µM or 1 µM erlotinib respectively for the indicated time points. Cell lysates were collected for detecting IFNAR1 expression by Western blot.

Referring to FIG. 2R-X, four NSCLC cell lines carrying the indicated drivers (EML4/ALK, ROS1, MET, BRAF) were treated with 1 µM Erlotinib for the indicated time points. IFNα1 and IFNβ1 mRNA levels were detected by qPCR. β-Actin was used as the loading control. Data refers to mean ± S.E.M, n=3 independent repeated experiments. One-way ANOVA with Dunnett's test was used to determine adjusted p value for comparison between untreated and each treated sample. *: p<0.05, **: p<0.01, ***: p<0.001. H1666 is reported to harbor IFNA1 homodeletion in COSMIC (Catalogue Of Somatic Mutations In Cancer)-v90 http://cancer.sanger.ac.uk/cosmic (Updated 5 September 2019), also in Data from a CPRIT (Cancer Prevention & Research Institute of Texas)-funded NGS (next generation sequencing) project by Dr. John Minna, UT Southwestern Medical Center, and Data from Dr. Adi Gazdar, UT Southwestern Medical Center. All other cell lines used in this research were searched on those databases above and confirmed to harbor neither IFNA1 nor IFNB1 homodeletion. Western blots are representative of 3 independent experiments with similar results. Cropped images are shown. Uncropped Western blot images are shown in Source Data.

Referring to FIG. 3A-D, HCC827 and A549 were treated with 0.1 µM or 1 µM Erlotinib for 2 days. The protein concentration of IFNα1 and IFNβ1 in cell lysates were measured by ELISA.

Referring to FIG. 3C-J, nude mice were injected subcutaneously (s.c.) with HCC827 or A549 cells. NOD-SCID mice were s.c. implanted with HCC4190 (EGFR mutant) or HCC4087 (EGFR wt) NSCLC PDX. After tumor formation, erlotinib at 50 mg/kg for EGFR mutant or 100 mg/kg for EGFR wt was given to mice daily for indicated days. Tumors were removed and subjected to ELISA for IFNα1 and IFNβ1.

Referring to FIG. 3K-R, EGFR mutant cell lines were treated with 0.1 µM Erlotinib. 48 hours later cell lysates (K-N) and supernatants (O-R) were collected for IFN ELISA. S-Z. A similar experiment was conducted in four EGFR wt cell lines.

Referring to FIG. 3AA-FF, EGFR mutant and wt NSCLC cell lines were treated with 0.1 or 1 µM Erlotinib for the indicated time points. IFNG mRNA levels were detected by qPCR. β-Actin was used as the loading control.

Referring to FIG. 3GG-MM, NSCLC cell lines were transfected with IFNGR1 siRNA or control siRNA for 48 hours followed by exposure to erlotinib for 72h, followed by AlamarBlue assay. siRNA knockdown of IFNGR1 was confirmed with Western blot. Data refers to mean ± S.E.M, n=3 independent repeated experiments. ELISA was analyzed by two-sided t-test, and one-way ANOVA with Dunnett's test for animal tumors. In qPCR, one-way ANOVA with Dunnett's test was used to determine adjusted p value for comparison between untreated and each treated sample. In AlamarBlue assay, two-way ANOVA adjusted by Bonferroni's correction was used. #: p>0.05, *: p<0.05, **: p<0.01, ***: p<0.001. Western blots are representative of 3 independent experiments with similar results. Cropped images are shown. Uncropped Western blot images are shown in Source Data.

### 4. BIOLOGICAL SIGNIFICANCE OF TYPE I IFN UPREGULATION BY EGFR INHIBITION IN NSCLC

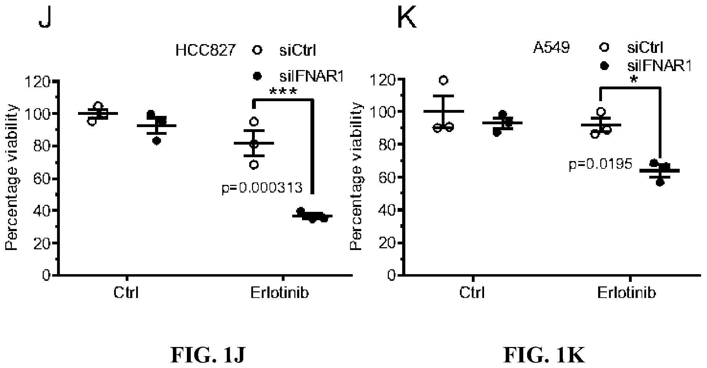

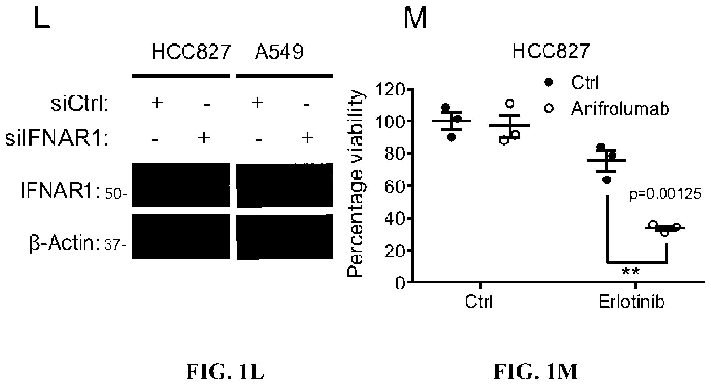

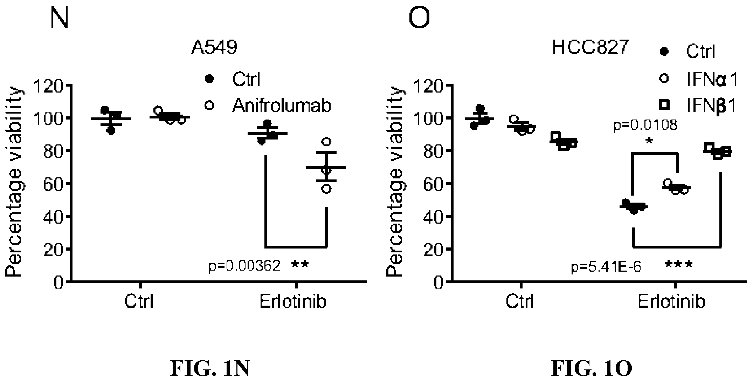

Next, it was found that siRNA knockdown of IFNAR1, the receptor for Type I interferons, resulted in enhanced sensitivity to EGFR inhibition in EGFR mutant NSCLC (FIG. 1J, FIG. 1L, and FIG. 4A-D). In addition, silencing of IFNAR1 conferred sensitivity to EGFR inhibition in resistant EGFRwt/KRas cell lines (FIG. 1K, FIG. 1L, and FIG. 4E-H). The effect of anifrolumab, a monoclonal antibody directed against IFNAR that inhibits the binding of Type I IFNs to its receptor and is in clinical trials for lupus, was also examined. Anifrolumab enhanced sensitivity to erlotinib in EGFR mutant NSCLC lines (FIG. 1M and FIG. 4I-K) and in multiple resistant EGFRwt/KRas cell lines (FIG. 1N and FIG. 4L-N). Additionally, IFN inhibition resulted in sensitivity to erlotinib in EGFRwt NSCLC cell lines with *Ros1* mutation, *EML4*/*ALK* fusion, *Met* amplification. and *Braf* mutation (FIG. 40-W).

Referring to FIG. 1J-N, HCC827 and A549 cells were transfected with IFNAR1 or control siRNA for 48h and then exposed to 0.01 µM or 1 µM Erlotinib for 72h, or concurrently treated by 0.01 µM or 1 µM Erlotinib, together with 10 µg/mL Anifrolumab for 72h, followed by AlamarBlue assay. Western blot confirming the silencing of IFNAR1.

Referring to FIG. 4A-D, EGFR mutant NSCLC cell lines PC9, H3255 and HCC2279 were transfected with IFNAR1 siRNA or control siRNA for 48 hours followed byexposure to 0.01 µM erlotinib for 72h, followed by AlamarBlue assay. siRNA knockdown of IFNAR1 was confirmed with Western blot.

Referring to FIG. 4E-H, EGFR wt NSCLC cell lines H441, H2122 and H1373 were transfected with IFNAR1 siRNA or control siRNA for 48 hours followed by exposure to 1 µM erlotinib for 72h, followed by AlamarBlue assay. siRNA knockdown of IFNAR1 was confirmed with Western blot.

Referring to FIG. 4I-N, NSCLC cells were concurrently treated by Erlotinib at 0.01 µM (EGFR mutant), or 1 µM (EGFR wt), together with 10 µg/mL Anifrolumab for 72h, followed by AlamarBlue assay.

Referring to FIG. 4O-W, NSCLC cell lines carrying the indicated drivers (EML4/ALK, ROS1, MET, BRAF) were transfected with IFNAR1 siRNA or control siRNA for 48 hours followed by exposure to erlotinib for 72h, or concurrently treated with erlotinib together with 10 µg/mL Anifrolumab for 72h, followed by AlamarBlue assay. siRNA knockdown of IFNAR1 was confirmed with Western blot. Data represents mean ± S.E.M. of three independent repeated experiments. *: p<0.05, **: p<0.01, ***: p<0.001, by two-way ANOVA adjusted by Bonferroni's correction. Western blots are representative of 3 independent experiments with similar results. Cropped images are shown. Uncropped Western blot images are shown in Source Data.

Next, it was found that exogenous Type I IFNs protect from erlotinib-induced cell death in oncogene addicted cells in cell survival assays (FIG. 1O-R). It was found that prolonged culture in the presence of erlotinib alone resulted in the emergence of resistant cells. However, a combined exposure to erlotinib plus anifrolumab inhibited the development of secondary resistance (FIG. 1S).

Referring to FIG. 1O-R, four EGFR mutant cell lines were treated by 0.1 µM Erlotinib and exogenous IFNα1 or IFNβ1 at 50 ng/mL for 72h followed by AlamarBlue assay.

Referring to FIG. 1S, HCC827 cells were plated in a 96-well plate with 0.1 µM erlotinib and/or 10 µg/mL Anifrolumab and cultured for extended periods as indicated. When cells reach 100% confluence, they were considered resistant.

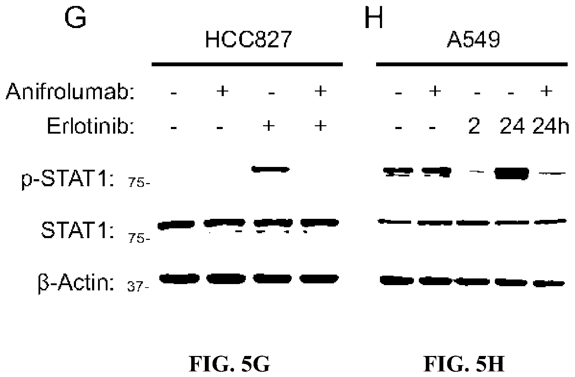

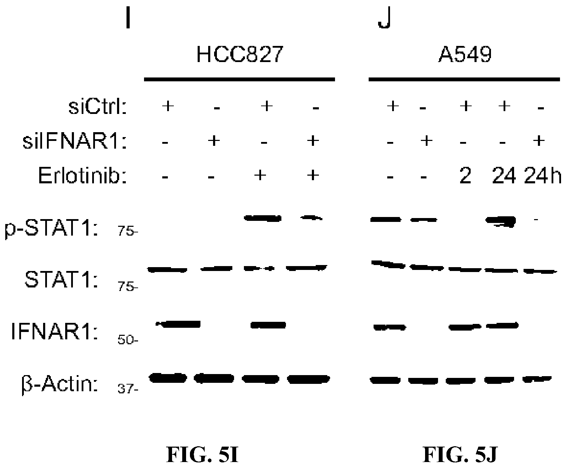

Although the Type I interferons have a known role in cytotoxicity, they also have a pro-survival role and mediate resistance to radiation and chemotherapy, primarily through STAT1 activation. It was found that EGFR inhibition results in activation of STAT1 in multiple EGFR wild type and EGFR mutant NSCLC cell lines (FIG. 5A-F). Furthermore, anifrolumab or siRNA knockdown of IFNAR1 blocks erlotinib induced activation of STAT1 confirming that erlotinib induced Type I IFN upregulation is required for STAT1 activation. (FIG. 5G-J and FIG. 6A-H). It was also found that siRNA knockdown of STAT1 synergizes with EGFR inhibition in cell survival assays (FIG. 5I-M and FIG. 6I-N) and abrogates the ability of exogenous Type I IFNs to rescue cells from EGFR inhibition induced cell death in EGFR mutant NSCLCs (FIG. 5N-Q). Thus, STAT1 activation provides a mechanistic explanation for the pro-survival effect of Type I IFNs in the context of EGFR inhibition.

Referring to FIG. 5A-F, three EGFR mutant (HCC827, H3255, and HCC2279), as well as three EGFRwt (A549, H441, H1573), were treated with 0.1 or 1 µM Erlotinib for the indicated time points. Cell lysates were collected and subjected to Western blot for detection of total and phosphorylated STAT1 expression. β-Actin was the loading control.

Referring to FIG. 5G and FIG. 5H, HCC827 and A549 cells were concurrently treated by 0.1 or 1 µM Erlotinib for 24h or the indicated time points, with or without 10 µg/mL Anifrolumab.

Referring to FIG. 5I and FIG. 5J, HCC827 and A549 cells were transfected with IFNAR1 or control siRNA for 48h, followed by 0.1 or 1 µM Erlotinib for 24h or the indicated time points. Western blot was performed to detect total and phosphorylated STAT1.

Referring to FIG. 5K-M, HCC827 and A549 cells were transfected with STAT1 or control siRNA for 48h, followed by indicated doses of Erlotinib for 72h, and then cell viability was measured by AlamarBlue assay. STAT1 siRNA was confirmed by Western blot.

Referring to FIG. 5N-Q, three EGFR mutant cells were transfected with STAT1 or control siRNA for 48h, and then cells were concurrently treated by 0.1 µM Erlotinib and exogenous IFNα1 or IFNβ1 at 50 ng/mL as indicated for 72h followed by AlamarBlue assay. STAT1 siRNA was confirmed by Western blot.

Referring to FIG. 6A and FIG. 6B, two EGFR mutant NSCLC cell lines H3255 and HCC2279 were concurrently treated by 0.1 µM Erlotinib with or without 10 µg/mL Anifrolumab.

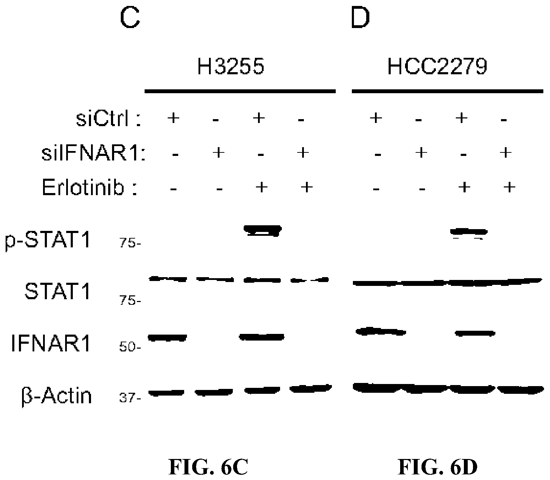

Referring to FIG. 6C and FIG. 6D, H3255 and HCC2279 were transfected with IFNAR1 or control siRNA for 48h, followed by 0.1 Erlotinib for 24h. Western blot was performed to detect total and phosphorylated STAT1.

Referring to FIG. 6E-H, similar experiments were performed on two EGFRwt NSCLC cell lines H441 and H1573, while Erlotinib was used at 1 µM Erlotinib for the indicated time points.

Referring to FIG. 6I-N, EGFR mutant and EGFRwtcells were transfected with STAT1 or control siRNA for 48h, followed by indicated doses of Erlotinib for 72h, and then cell viabilities were measured by AlamarBlue assay. STAT1 siRNA was confirmed by Western blot. Data represent mean ± S.E.M. n=3 independent repeated experiments. #: p>0.05, *: p<0.05, **: p<0.01, ***: p<0.001, by two-way ANOVA adjusted by Bonferroni's correction. Western blots are representative of 3 independent experiments with similar results. Cropped images are shown. Uncropped Western blot images are shown in Source Data.

### 5. EGFR INHIBITION RESULTS IN ACTIVATION OF A TBK1/IRF3 SIGNALING AXIS IN NSCLCS WITH EGFR ACTIVATING MUTATIONS

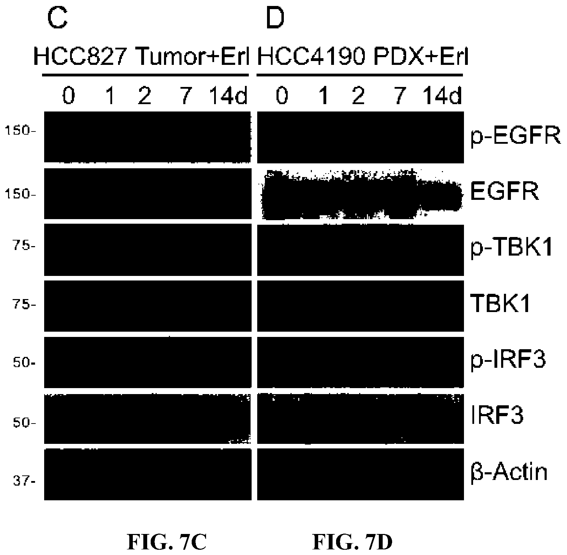

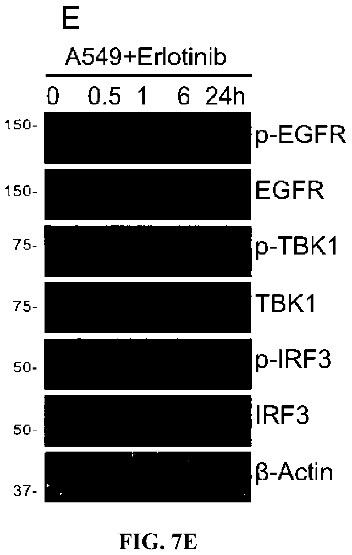

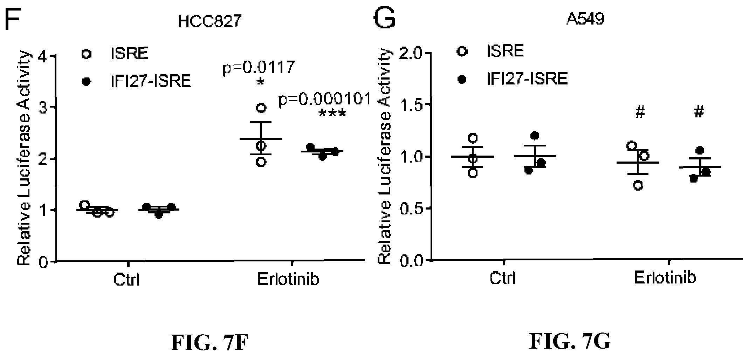

The transcription factor IRF3 plays a central role in transcription of Type I interferons. IRF3 is activated by TBK1. EGFR inhibition led to a rapid and robust activation of TBK1 and IRF3 in NSCLC cell lines harboring EGFR-activating mutations (FIG. 7A, FIG. 7B, FIG. 8A, and FIG. 8B) and in animal models (FIG. 7C and FIG. 7D). However, EGFR inhibition-induced TBK1 or IRF3 activation was not detected in EGFRwt cell lines, or in EGFRwt animal models (FIG. 7E and FIG. 8C-E). Similarly, increased IRF3 transcriptional activity was detected upon EGFR inhibition in multiple EGFR mutant NSCLC cell lines but not in EGFRwt NSCLC cell lines (FIG. 7F, FIG. 7G, and FIG. 8F-K). It was found that siRNA knockdown of TBK1 could not abrogate the ability of exogenous Type I IFNs to rescue EGFR mutant cells from EGFR inhibition induced cell death in EGFR presumably because TBK1 is upstream of IFN signaling (FIG. 8L-O). It was confirmed that pharmacological inhibition of TBK1 using BX-795 or siRNA knockdown of TBK1 results in a loss of EGFR inhibition-induced IRF3 phosphorylation (FIG. 8P-U). In addition, there is a loss of erlotinib-induced IRF3 transcriptional activity in multiple EGFR mutant cell lines in response to chemical or biological inhibition of TBK1 (FIG. 8V-BB). IKKε is another kinase involved in the activation of IRF3 but does not appear to be expressed in NSCLC cell lines (FIG. 8CC).

Referring to FIG. 7A and FIG. 7B, two EGFR mutant lines were treated with 0.1 µM erlotinib for the indicated time points followed by collection of lysates and Western blot.

Referring to FIG. 7C, nude mice bearing HCC827 xenografts were treated with erlotinib 50 mg/kg for 1-14 days followed by removal of tumors and Western blot.

Referring to FIG. 7D, a similar experiment was performed with HCC4190 PDX in NOD-SCID mice. Mice treated with erlotinib 50 mg/kg for 1-14 days.

Referring to FIG. 7E, erlotinib (1 µM) was used to treat A549 cells for different time points followed by Western blot.

Referring to FIG. 7F and FIG. 7G, HCC827 and A549 cells were transfected with ISRE-Luc or IFI27-Luc reporters for 48 hours, then treated with erlotinib at 0.1 or 1 µM for 24h followed by a luciferase assay.

Referring to FIG. 8A-C, cells were treated with 0.1 (A-B) or 1 µM (C) Erlotinib.

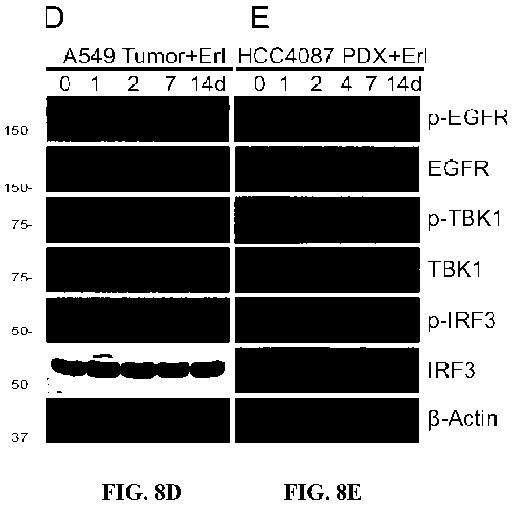

Referring to FIG. 8D and FIG. 8E, nude mice bearing A549 xenografts (8D) and NOD-SCID mice with HCC4087 PDX (8E) were treated with erlotinib 100 mg/kg, followed by Western blot.

Referring to FIG. 8F-K, cells were transfected with ISRE or IFI27-ISRE reporter for 48 hours and treated with erlotinib for 24h, followed by a luciferase reporter assay.

Referring to FIG. 8L-O, EGFR mutant NSCLC lines were transfected with TBK1 siRNA for 48 hours followed by 0.1 µM erlotinib for 72h, concurrently with exogenous IFNα1 or IFNβ1 at 50 ng/mL, followed by AlamarBlue assay. TBK1 siRNA was confirmed with Western blot.

Referring to FIG. 8P-R, EGFR mutant cells were concurrently treated with 0.1 µM Erlotinib and/or 1 µM BX795 for 24 hours, followed by Western blot. S-U. EGFR mutant lines were transfected with TBK1 siRNA for 48h followed by 0.1 µM erlotinib for an additional 24h, followed by Western blot.

Referring to FIG. 8V-X, EGFR mutant cells were transfected with ISRE reporter for 48h followed by treatment with erlotinib 0.1 µM and/or 1 µM BX795 for an additional 24h followed by a luciferase assay.

Referring to FIG. 8Y-BB, EGFR mutant cell lines were transfected with siRNA for TBK1 or control siRNA and a luciferase reporter for ISRE for 48h followed by 0.1 µM Erlotinib and for an additional 24h followed by a luciferase assay. Silencing of TBK1 was confirmed by Western blot.

Referring to FIG. 8CC, Western blotting for IKKε expression in NSCLC lines is shown. U87MG cells were used as a positive control. Data represents mean ± S.E.M. of three independent biological replicates. #: p>0.05, *: p<0.05, **: p<0.01, ***: p<0.001, by two-sided t-test unadjusted for multiple comparisons in luciferase assay (F-K), three-way (L-N) and two-way ANOVA (V-AA) adjusted by Bonferroni's correction. Western blots are representative of three independent experiments with similar results. Cropped images are shown. Uncropped images are shown in Source Data.

### 6. IRF3 AND TBK1 ACTIVATION PROTECT EGFR MUTANT CELLS FROM A LOSS OF EGFR SIGNALING

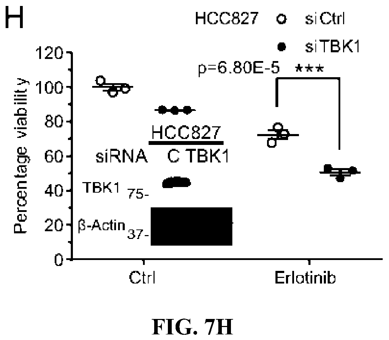

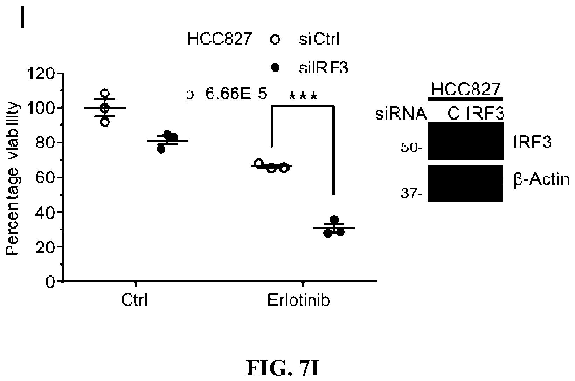

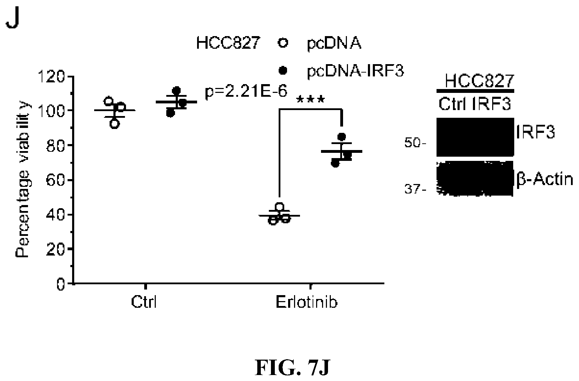

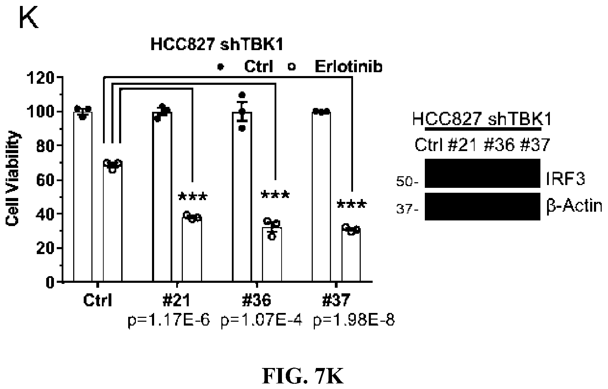

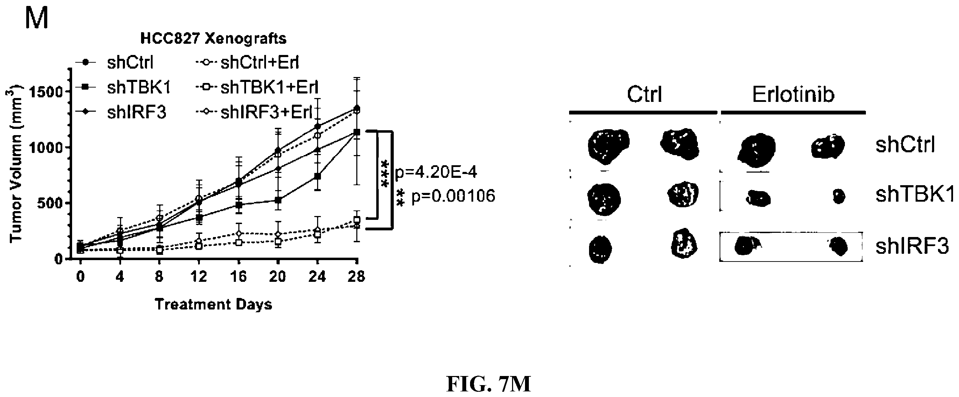

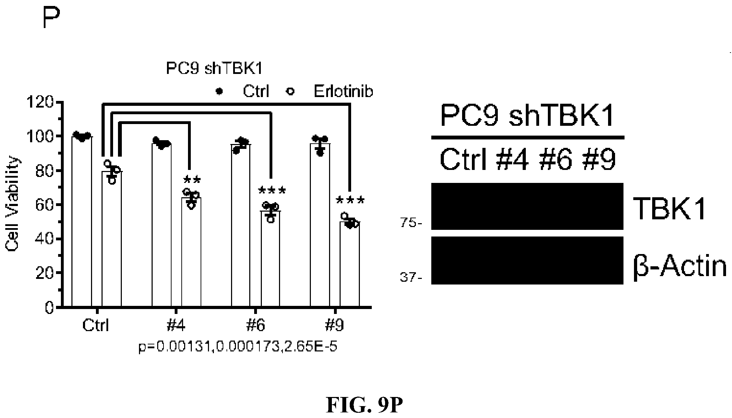

It was found that pharmacological inhibition of TBK1 with BX-795 or siRNA knockdown of TBK1 synergized with EGFR inhibition in multiple NSCLC cell lines with mutant EGFR in cell viability assays (FIG. 7H and FIG. 9A-H). Also, siRNA knockdown of IRF3 synergizes with EGFR inhibition in multiple NSCLC cell lines with mutant EGFR in cell viability assays (FIG. 7I and FIG. 9I-L). Conversely, overexpression of IRF3 in EGFR mutant cells results in resistance to EGFR inhibition-induced cell death in EGFR mutant NSCLC cell lines (FIG. 7J and FIG. 9M-O). Next, shRNA was used to stably silence TBK1 in EGFR mutant cell lines (FIG. 7K). It was confirmed that cells with stable silencing of TBK1 or IRF3 were sensitized to EGFR inhibition (FIG. 7K-L). Next, HCC827 cells with shTBK1, shIRF3, or with control shRNA were injected into the flanks of mice to form subcutaneous tumors. Once tumors became visible, treatment was started with control vehicle, or erlotinib. Stable silencing of TBK1 or IRF3 resulted in enhanced sensitivity of xenografted HCC827 and PC9 tumors to erlotinib (FIG. 7M and FIG. 9P-R).

Referring to FIG. 7H and FIG. 7I, HCC827 cells were transfected with the indicated TBK1, IRF or control siRNA for 48 hours, and then treated with 0.01 µM erlotinib for 72 hours followed by AlamarBlue assay.

Referring to FIG. 7J, HCC827 cells were transfected with IRF3 expressing plasmid or empty vector for 48 hours, and then incubated with 0.1 µM Erlotinib for 72 hours. Cell viability was detected by AlamarBlue assay and overexpression of IRF3 was confirmed by Western blot.

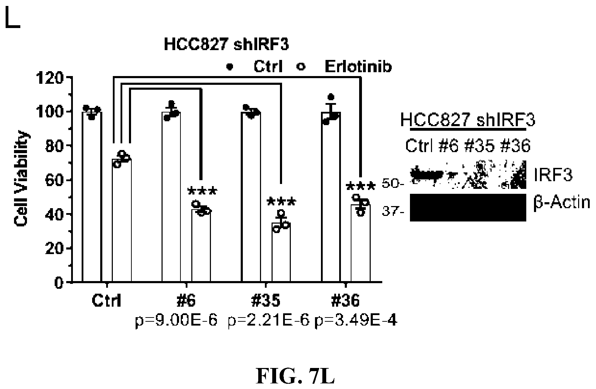

Referring to FIG. 7K and FIG. 7L, AlamarBlue cell survival assays were conducted with multiple stably silenced HCC827 TBK1 and HCC827 IRF3 clones, with or without erlotinib 0.1 µM. Gene silencing was confirmed by Western blot in K-L.

Referring to FIG. 7M, animal experiments with stably silenced TBK1 or IRF3. Eight nude mice per group were injected with HCC827 shTBK1 (clone #37), shIRF3 (clone #35), or control lentivirus-infected stable cells per group and the rate of tumor formation was 5-8 as shown in Source Data. Mice received 6.25 mg/kg/d Erlotinib or Vehicle by oral gavage. Tumor sizes were monitored as described in the methods section and representative tumor images are shown.

Referring to FIG. 9A-D, PC9, H3255, and HCC2279 cells were transfected with the TBK1 siRNA for 48h followed by exposure to 0.1 µM Erlotinib for 72 hours and AlamarBlue assay. siRNA knockdown of TBK1 was confirmed with Western blot.

Referring to FIG. 9E-H, AlamarBlue assay was done on four EGFR mutant cells after cotreatment with 0.01 µM Erlotinib and/or 1 µM BX795 for 72 hours.

Referring to FIG. 9I-L, PC9, H3255, and HCC2279 cells were transfected with IRF3 or control siRNA for 48h followed by treatment with 0.01 µM Erlotinib for 72 hours and AlamarBlue assay. Silencing of IRF3 was confirmed with Western blot.

Referring to FIG. 9M-O, EGFR mutant cell lines were transfected with IRF3 expressing plasmid or empty vector for 48 hours, followed by incubation with 0.1 µM Erlotinib for 72 hours. Cell viability was detected by AlamarBlue assay. Overexpression of IRF3 was confirmed with Western blot.

Referring to FIG. 9P-Q, PC9 cells were stably infected with lentivirus control shRNA (shCtrl) or shRNA for TBK1 or IRF3 lentivirus and Western blot was conducted to confirm silencing. Silenced clones were studied in AlamarBlue cell survival assays following erlotinib exposure for 72h.

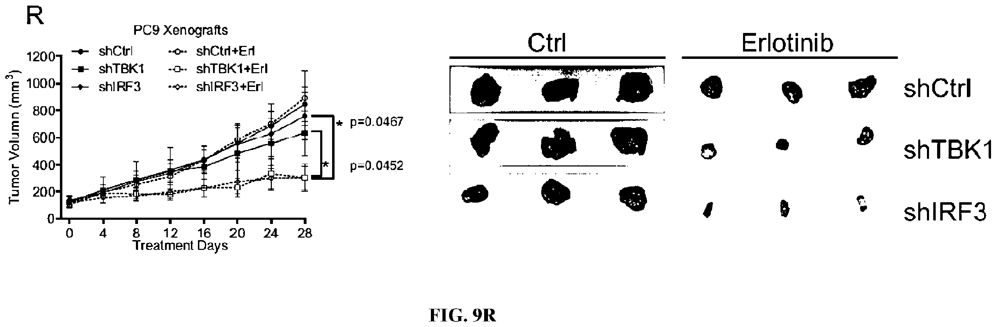

Referring to FIG. 9R, PC9 cells with stable silencing of TBK1 (clone #9) or IRF3 (clone #9) were injected subcutaneously into eight nude mice per group and the rate of tumor formation was 5-8 per group as shown in Source Data. Erlotinib was administered daily at 6.25 mg/kg by oral gavage. Tumor sizes were monitored as described in the Methods section. Representative tumor images are shown. Data refers to mean ± S.E.M of three independent biological replicates or tumor sizes. *: p<0.05, **: p<0.01, ***: p<0.001, by two-way ANOVA adjusted by Bonferroni's correction. Western blots are representative of 3 independent experiments with similar results. Cropped images are shown. Uncropped Western blot images are shown in Source Data.

### 7. TBK1 UNDERGOES A TRIM32 DEPENDENT K63-LINKED UBIQUITINATION IN RESPONSE TO EGFR INHIBITION

To understand the mechanism of EGFR inhibition dependent TBK1 activation, proteins that associate with TBK1 in response to EGFR inhibition were analyzed using mass spectrometry (FIG. 10A). Tripartite motif-containing protein 32 (TRIM32) was chosen for further investigation as a potential candidate in the activation of TBK1. TRIM32 has an E3-ubiquitin ligase activity, and has previously been reported to have a role in Type I interferon induction in response to viral infection. TRIM32 forms a complex with TBK1 in response to erlotinib in EGFR mutant NSCLC cell lines (FIG. 10B-D). TRIM32 can mediate K63-linked ubiquitination resulting in specific pathway activation. Importantly, several studies have reported a key role for K63-linked ubiquitination in TBK1 phosphorylation and activation. It was found that EGFR inhibition leads to K63-linked ubiquitination of TBK1 in NSCLC cells (FIG. 10B-D). Importantly, siRNA knockdown of TRIM32 inhibits EGFR inhibition-induced K63 ubiquitination of TBK1 (FIG. 10E-G) and phosphorylation (FIG. 10H-J). Without wishing to be bound by theory, these data indicate that TRIM32 is required for TBK1 and IRF3 activation in response to EGFR inhibition.

Referring to FIG. 10A, HCC827 cells were treated with 0.1 µM erlotinib for 0, 2, 6, and 24 hours. Cell lysates were immunoprecipitated by TBK1 antibody and Mass spectrometry was performed. The heatmap indicates the proteins that binds to TBK1 after 24h of erlotinib treatment with an affinity increase of over two folds.

Referring to FIG. 10B-D, three EGFR mutant cell lines were treated with 0.1 µM Erlotinib for 0, 2, 6, and 24 hours followed by preparation of cellular lysates. This was followed by immunoprecipitation with a TBK1 antibody and Western blot with TRIM32, K63-Ubiquitin, and TBK1 antibodies. TRIM32 and β-Actin from the input samples were also tested.

Referring to FIG. 10E-G, cells were transfected with TRIM32 siRNA or control siRNA for 48 hours. This was followed by treatment with 0.1 µM erlotinib for 24 hours, followed by immunoprecipitation with TBK1 antibodies and Western blot with K-63 ubiquitin or TBK1 antibody as described.

Referring to FIG. 10H-J, EGFR mutant cell lines with silenced TRIM32 were treated with erlotinib (24h) followed by Western blot with pTBK1 or pIRF3 antibodies. Western blots are cropped and representative of three independent repeated experiments with similar results. Uncropped Western blots are shown in Source Data.

### 8. UPREGULATION OF RIG-I BUT NO ACTIVATION OF STING OR AHR IN RESPONSE TO EGFR INHIBITION

Retinoic acid inducible gene I (RIG-I) is a pattern sensing receptor that plays a key role in sensing RNA viruses. It was found that RIG-I is strongly induced by EGFR inhibition in multiple EGFR mutant NSCLC cell lines (FIG. 11A-D). Also, siRNA knockdown of RIG-I blocked TBK1 and IRF3 activation in response to EGFR inhibition (FIG. 11E-H). Furthermore, a loss of RIG-I enhances the sensitivity of EGFR mutant cell lines to erlotinib in cell survival assays (FIG. 11I-N). Without wishing to be bound by theory, these data suggest that RIG-I is upregulated in response to EGFR inhibition and leads to activation of IRF3 culminating in resistance to EGFR inhibition.

Referring to FIG. 11A-D, EGFR mutant cells were treated with 0.1 µM Erlotinib for the time points indicated followed by Western blot with RIG-I and β-Actin antibodies.

Referring to FIG. 11E-H, cells were transfected with RIG-I siRNA or control siRNA for 48h and then exposed to 0.1 µM erlotinib for 24h followed by Western blots.

Referring to FIG. 11I-N, cells were transfected with RIG-I siRNA or control siRNA for 48h and then exposed to 0.01 µM erlotinib for 72h and cell viability was tested using AlamarBlue assay. RIG-I siRNA interference was confirmed by Western blot.

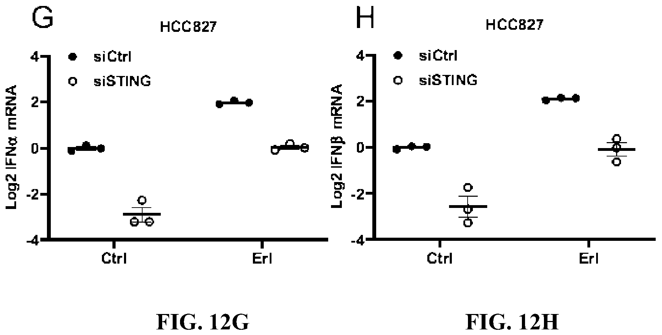

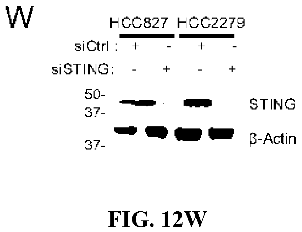

cGAS/STING activates IRF3 leading to induction of Type I IFNs. STING is upregulated in EGFR TKI persister cells. However, increased phosphorylation of STING in response to erlotinib was unable to be detected in either EGFR mutant or EGFRwt cell lines (FIG. 12A-F). It was found that siRNA knockdown of STING decreased the basal level of Type I IFNs in EGFR mutant and EGFRwt NSCLC cell lines. However, the erlotinib induced upregulation of IFNs does not require STING in any of the cell lines tested (FIG. 12G-V). Finally, siRNA knockdown of STING does not synergize with EGFR inhibition in cell survival assays (FIG. 12W-BB).

Referring to FIG. 12A-F, three EGFR mutant and three EGFRwt NSCLC cell lines were treated by 0.1 or 1 µM Erlotinib for the indicated time points and cell lysates were analyzed by Western blot for detection of total and phosphorylated STING expression. STING was undetectable in PC9 and A549 cells. Phosphorylated STING can only be detected in HCC2279 cell line.

Referring to FIG. 12G and FIG. 12H, HCC827 cells were transfected with STING or control siRNA for 48h, and then exposed to 0.1 µM Erlotinib for 24h, followed by qPCR for detection of IFNA1 and IFNB1 mRNA.

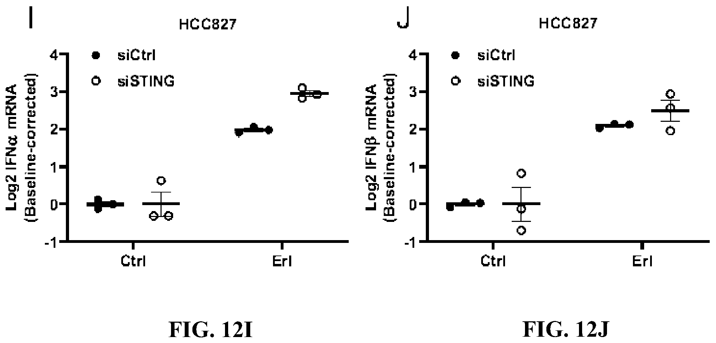

Referring to FIG. 12I and FIG. 12J, as STING siRNA alone was found to be able to decrease the basal IFN mRNA levels by two-way ANOVA adjusted by Bonferroni's correction, the baseline correction set on Erlotinib untreated groups was performed via GraphPad Prism 8.

Referring to FIG. 12K-V, similar experiments and corrections were performed on other three NSCLC cell lines, while Erlotinib was used at 0.1 or 1 µM forEGFR mutant and EGFRwt cells respectively.

Referring to FIG. 12W-BB, two EGFR mutant and two EGFR wt NSCLC cell lines which do have STING expression were transfected with STING or control siRNA for 48h and then treated with the indicated doses of erlotinib for 72h followed by AlamarBlue assay. STING siRNA were confirmed by Western blot. Data represents to mean ± S.E.M. ofthree independent biological replicates. #: p>0.05, *: p<0.05, **: p<0.01, ***:p<0.001, by two-way ANOVA adjusted by Bonferroni's correction. Western blots are representative of 3 independent experiments with similar results. Cropped images are shown. Uncropped Western blot images are shown in Source Data.

The aryl hydrocarbon receptor (Ahr) has been implicated as a mechanism of resistance to EGFR TKIs in EGFR mutant NSCLC. It was found that Ahr levels are not altered in response to erlotinib (FIG. 13A-D). Ahr is localized to the nucleus when activated. Erlotinib-induced nuclear localization of Ahr was not detected (FIG. 13E-J), suggesting that Ahr activation is not a component of the adaptive response to EGFR inhibition. It certainly remains possible that Ahr may modulate the sensitivity to EGFR inhibition.

Referring to FIG. 13A-D, EGFR mutant (13A and 13B) and EGFR wt (13C and 13D) NSCLC cells were treated by 0.1 or 1 µM Erlotinib, followed by Western blot for Ahr.

Referring to FIG. 13E-H, NSCLC cells were treatedby 0.1 or 1 µM Erlotinib for 24h, respectively. AhR nuclear translocation was detected by Western blot. Lamin A/C was used as a loading control. LPS treatment at 10 µg/mL for 2 h was used as the positive control.

Referring to FIG. 13I-J, PC9 andH2122 cells were treated with 0.1 or 1 µM Erlotinib for 24h, respectively. Cells were then fixed, stained with the AhR antibody (red) and counterstained with the DAPI (blue). LPS treatment at 10 µg/mL for 2 h was used as the positive control. Scale bar represents 25 µm.

### 9. REGULATION OF PD-L1 BY EGFR INHIBITION

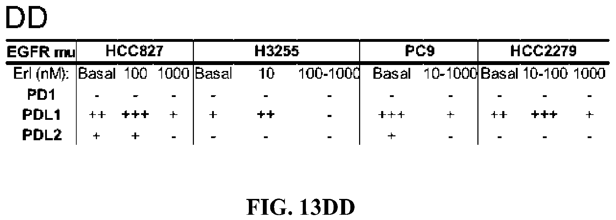

Although EGFR inhibition leads to decreased levels of PD-L1 in EGFR mutant lung cancer cell lines, immunotherapy is not effective in EGFR mutant NSCLC. Since type I IFNs regulate PD-L1 expression, the PD-1 pathway was examine next, and it was found that erlotinib affects primarily PD-L1 levels (FIG. 13DD-EE). Interestingly, while a high concentration of erlotinib leads to decreased PD-L1 levels (FIG. 13K-M), low concentrations of erlotinib leads to upregulation of PD-L1 in EGFR mutant NSCLC cell lines and in animal tumors (FIG. 13N-Q). In EGFRwt NSCLC lines, an increase in PD-L1 levels was not detected in response to erlotinib (FIG. 13V-Y). Importantly, this erlotinib-induced upregulation of PD-L1 in EGFR mutant NSCLCs can be abrogated by anifrolumab or siRNA knockdown of IFNAR1 (FIG. 13Z-CC), suggesting a possible explanation for why immunotherapy with PD1 inhibitors is not effective in EGFR mutant NSCLC. Thus, prior treatment with erlotinib may result in elevated PD-L1 levels in EGFR mutant tumors and render them resistant to immunotherapy. Such resistance could potentially be averted by the use of anifrolumab.

Referring to FIG. 13K-Y, three EGFR mutant NSCLC cells lines were treated as indicated (13K-P), nude mice bearing HCC827 xenografts were treated with erlotinib 50 mg/kg (13Q), and four EGFRwt NSCLC cell lines (13R-Y) were treated as indicated, PD-L1 expression was detected by Western blot.

Referring to FIG. 13Z-AA, HCC827 and H3255 cells were concurrently treated with 100 or 10 nM erlotinib for 24h, with or without 10 µg/mL Anifrolumab.

Referring to FIG. 13BB-CC, HCC827 and H3255 cells were transfected with IFNAR1 or control siRNA for 48h, and then treated with 100 or 10 nM erlotinib for 24h. PD-L1 expression in cell lysates above, and effects of IFNAR1 siRNA were detected by Western blot.

Referring to FIG. 13DD-EE, four EGFR mutant and four EGFRwt cells were treated by indicated doses of Erlotinib for 24 hours. PD-1, PD-L1, and PD-L2 expression was detected by Western blot. PD-L1 expression levels were partly shown above. Signalstrength was represented by symbols. -: undetected, +: weak, ++: expressed, +++: strong signals. Western blot and Immunofluorescent staining images are representative of 3 independent experiments with similar results. Cropped images are shown. Uncropped Western blot images are shown in Source Data.

### 10. THE MECHANISM OF EGFR INHIBITION-INDUCED IFN INDUCTION IS DISTINCT IN EGFR MUTANT AND EGFRWT NSCLC

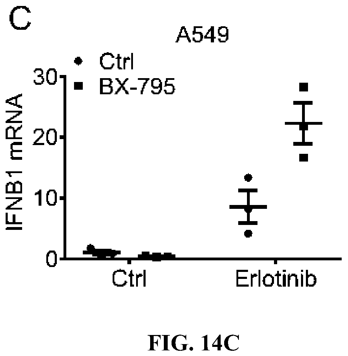

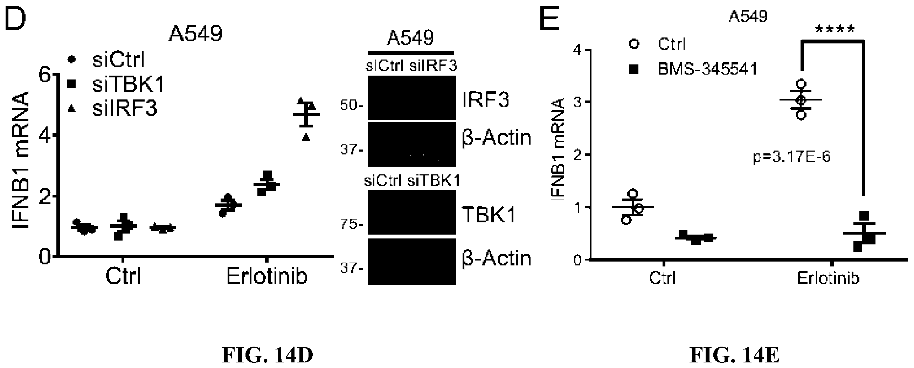

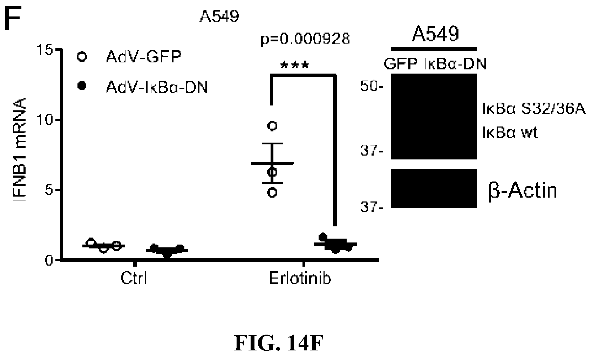

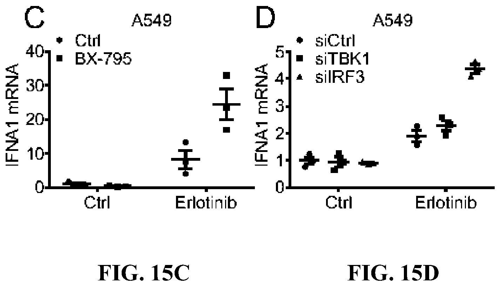

EGFR inhibition in EGFR mutant NSCLCs results in activation of a TBK1-IRF3 signaling axis. Also, pharmacological inhibition of TBK1 using BX-795 or siRNA knockdown of TBK1 or IRF3 completely suppressed erlotinib-induced upregulation of IFN in EGFR mutant cell lines (FIG. 14A, FIG. 14B, FIG. 15A, FIG. 15B, and FIG. 15L-U) but not in EGFRwt cells (FIG. 14C, FIG. 14D, FIG. 15C, FIG. 15D, and FIG. 15V-Z). Thus, EGFR inhibition-induced IFN upregulation requires TBK1/IRF3 in EGFR mutant but not in EGFRwt NSCLC lines. Previous studies have reported that EGFR inhibition leads to a rapid activation of NF-κB in both EGFR mutant and EGFRwt NSCLC. It was found that a pharmacological inhibitor of NF-κB (BMS-345541) or a dominant negative IkBα mutant blocks EGFR inhibition-induced upregulation of IFN in EGFRwt expressing NSCLC cell lines (FIG. 14E, FIG. 14F, FIG. 15C-F, and FIG. 15V-JJ) but not in EGFR mutant lines (FIG. 15G-K and FIG. 15KK-OO). Next, it was found that Etanercept, a specific TNF blocker, or siRNA knockdown of TNFR1 failed to inhibit erlotinib induced upregulation of type I IFNs in EGFR mutant lines, while it efficiently blocked TNF-induced activation of NF-κB (FIG. 16A-BB).

Referring to FIG. 14A, HCC827 cells were treated with 1 µM BX795 and 0.1 µM erlotinib for 24h followed by qPCR for IFNB1 mRNA.

Referring to FIG. 14B, HCC827 cells were transfected with siRNA for TBK1 or IRF3 for 48h. Then cells were exposed to 0.1 µM erlotinib for 24h followed by qPCR for IFNB1 mRNA. Western blot showing silencing of TBK1 and IRF3.

Referring to FIG. 14C and FIG. 14C, similar experiments were performed on A549 cells with erlotinib (1 µM).

Referring to FIG. 14E, A549 cells were concurrently treated with 1 µM erlotinib and 0.1 µM BMS-345541 for 24h followed by qPCR for IFNB1 mRNA.

Referring to FIG. 14F, A549 cells were infected with IκBα-DN/GFP adenoviruses for 24h followed by exposure to erlotinib (1 µM) for 48h and qPCR for IFNB1 mRNA. Western blot demonstrating expressing of mutant IκBα.

Referring to FIG. 15A and FIG. 15B, HCC827 cells were concurrently treated by 1 µM BX795 and 0.1 µM erlotinib for 24h, or pre-transfected with siRNA for TBK1 or IRF3 for 48 hours followed by 0.1 µM erlotinib for 24h, followed by qPCR for IFNA1 mRNA. Silencing of TBK1 and IRF3 were confirmed in Figure 14B.

Referring to FIG. 15C and FIG. 15D, similar experiments were performed on A549 cells with 1 µM erlotinib. siRNA knockdown was confirmed in Figure 6D. E-F. A549 cells were concurrently treated by 1 µM erlotinib and 0.1 µM BMS-345541 for 24h or infected with IκBα-DN/GFP adenoviruses for 48h followed by Erlotinib (1 µM) for 24h, followed by qPCR for IFNA1 mRNA. Expression of mutant IκBα is shown in Figure 14F.

Referring to FIG. 15G-J, similar experiments were performed on HCC827, with 100 nM Erlotinib.

Referring to FIG. 15K, expression of mutant IκBα detected with Western blotis shown.

Referring to FIG. 15L-P, PC9 cells were treated with 1 µM BX795 and 0.1 µM erlotinib for 24h, or transfected with TBK1, IRF3 siRNA for 48 hours and then treated with 0.1 µM erlotinib for 24h, followed by qPCR for IFNA1 and IFNB1 mRNA. Silencing of TBK1 and IRF3 was confirmed by Western blot.

Referring to FIG. 15Q-Z, similar experiments were conducted with H3255 and H441 cells.

Referring to FIG. 15AA-EE, H441 cells were co-treated by 0.1 µM BMS-345541 and 1 µM Erlotinib for 24 hours, or infected with IκBα-DN or GFP adenoviruses for 48 hours followed with 1 µM Erlotinib for 24 hours. IFNA1 andIFNB1 mRNA levels were tested by qPCR. IκBα-DN overexpression was detected by Western blot.

Referring to FIG. 15FF-OO, similar experiments were performed on H2122 and PC9 cells. Data indicates mean ± S.E.M of three independent biological replicates. #: p>0.05, *: p<0.05, **: p<0.01, ***: p<0.001, by two-wayANOVA adjusted by Bonferroni's correction. Western blots are representativeof 3 independent experiments with similar results. Cropped images are shown. Uncropped images are shown in Source Data.

Referring to FIG. 16A-F, EGFR mutant lines were concurrently treated with 0.1 µM Erlotinib and 10 µg/mL Etanercept (Enbrel) for 24 hours, followed by qPCR for detection of IFNA1 and IFNB1 mRNA.

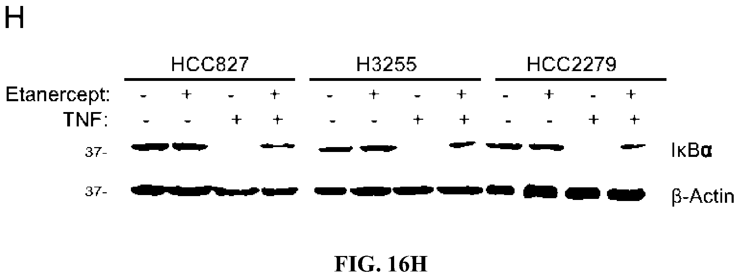

Referring to FIG. 16G and FIG. 16H, EGFR mutant lines were transfected with an NF-κB reporter for 48h, followed by 10 µg/mL Etanercept for 1h and then 10ng/mL TNF for 24h, followed by a luciferase assay or Western blot.

Referring to FIG. 16I-O, EGFR mutant cell lines were transfected with TNFR1 siRNA for 48 hours, then treated with 0.1 µM erlotinib for 24h followed by qPCR for IFNA1 and IFNB1 mRNA. Silencing of TNFR1 was confirmed by Western blot.

Referring to FIG. 16P-BB, similar experiments were performed on EGFRwt cell lines while 1 µM Erlotinib was used.

Referring to FIG. 16CC-EE, three EGFR mutant NSCLC cell lines were concurrently treated with 50 µg/mL Etanercept, 0.1 µM Erlotinib, 50 ng/mL IFNα1 or IFNβ1 for 72h.

Referring to FIG. 16FF-II, EGFR mutant cells were transfected with TNFR1 for 48 hours, then treated with 0.1 µM erlotinib, 50 ng/mL IFNα1 or IFNβ1 for 72h, followed by AlamarBlue assay. Silencing of TNFR1 was confirmed by Western blot.

Referring to FIG. 16JJ-LL, EGFR mutant cells were concurrently treated with 0.1 µM Erlotinib, 0.1 µM BMS-345541, 50 ng/mL IFNα1 or IFNβ1 as indicated for 72h followed by AlamarBlue assay.

Referring to FIG. 16MM-PP, EGFR mutant cells were infected with or IκBα-DN or GFP adenoviruses for 48h, then concurrently treated with 0.1 µM Erlotinib,50 ng/mL IFNα1 or IFNβ1 for 72h followed by AlamarBlue assay. IκBα-DN overexpression was confirmed by Western blot. Data indicates mean ± S.E.M of three independent biological replicates. #: p>0.05, *: p<0.05, **: p<0.01, ***:p<0.001, by two-way (A-AA) or three-way (CC-OO) ANOVA adjusted by Bonferroni's correction. Western blots are representative of 3 independent experiments with similar results. Cropped images are shown. UncroppedWestern blot images are shown in Source Data.

To investigate whether TNF-NF-κB signaling plays a role in the pro-survival effects of Type I IFNs in the context of EGFR inhibition, whether etanercept is able to inhibit the pro-survival effect of exogenous Type I IFNs was examined (FIG. 1N-Q). Addition of etanercept or TNFR1 siRNA fails to block the pro-survival effects of IFNs in the presence of EGFR inhibition (FIG. 16CC-II). Similarly, chemical inhibition of NF-κB using BMS-34551, or biological inhibition of NF-κB using a dominant negative IKBα mutant fail to abrogate the protective effect of type I IFNs in the context of EGFR inhibition (FIG. 16JJ-PP).

### 11. THE ROLE OF IFN SIGNALING IN SECONDARY RESISTANCE TO EGFR INHIBITION

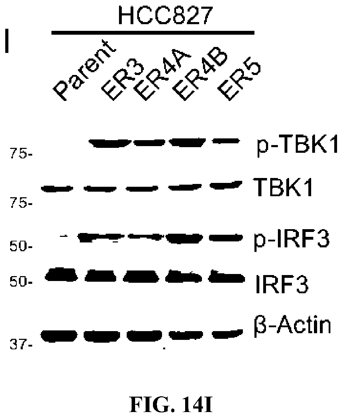

To examine the role of Type I IFN signaling in NSCLCs in secondary resistance to EGFR inhibition, NSCLC EGFR mutant cell lines rendered experimentally resistant to EGFR TKIs by two independent groups were examined. Four independent clones were analyzed. It was found that Type I IFN levels are high in all clones (FIG. 14G-H). The T790M mutation or Met amplification were not detected in these lines. Also, it was found that TBK1/IRF3 is activated in these cell lines (FIG. 14I). Importantly, it is possible to restore sensitivity to erlotinib in these cell lines if IFN is inhibited using anifrolumab or TNFR1 siRNA (FIG. 14J-S). H1975 cells that have dual L858R/T790M mutations were also examined, and it was found that type IFNs can be induced by afatinib (FIG. 14T-U). Furthermore, these cells are rendered sensitive to afatinib if IFN signaling is blocked (FIG. 14V-X). Without wishing to be bound by theory, these data suggest that the TBK1-IRF3-IFN pathway activation may be an independent mechanism of secondary resistance to EGFR inhibition in NSCLC.

Referring to FIG. 14G-I, three biologically independent RNA and protein samples from HCC827 cell line (parent) and its derived secondary erlotinib-resistant lines (ER3, ER4A, ER4B, ER5) were collected and analyzed by qPCR for IFNA1 and IFNB1 mRNA, or by Western blot for the indicated proteins shown in one representative sample.

Referring to FIG. 14J-S, HCC827 derived ER3 ER4A, ER4B, and ER5 were concurrently treated with 10 µg/mL Anifrolumab, or pre-transfected with IFNAR1 siRNA for 48 hours, then treated with erlotinib for 72 hours followed by AlamarBlue assay. IFNAR1 siRNA was confirmed by Western blot.

Referring to FIG. 14T-U, H1975 cells were treated with 0.1µM Afatinib for indicated time points, IFNA1 and IFNB1 mRNA levels were detected by qPCR.

Referring to FIG. 14V-X, H1975 cells were co-treated with 10 µg/mL Anifrolumab, or pre-transfected with IFNAR1 siRNA for 48h and then treated with 0.1µM Afatinib for 72 hours followed by AlamarBlue assay. IFNAR1 siRNA was confirmed by Western blot.

### 12. TYPE I INTERFERONS REGULATE SENSITIVITY TO EGFR INHIBITION IN

### ANIMAL MODELS

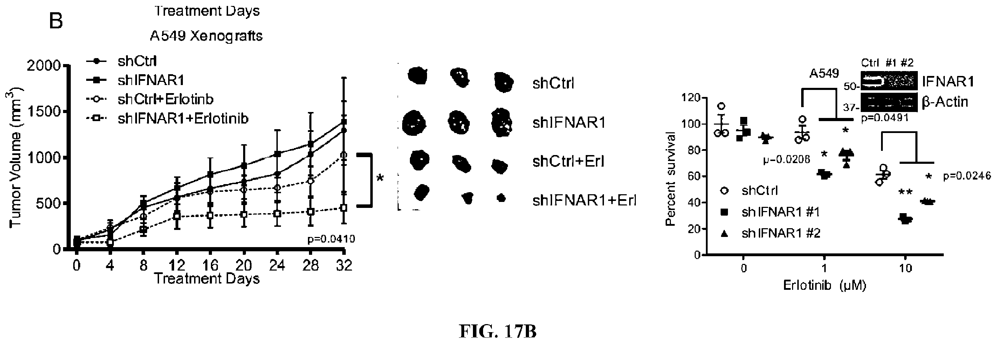

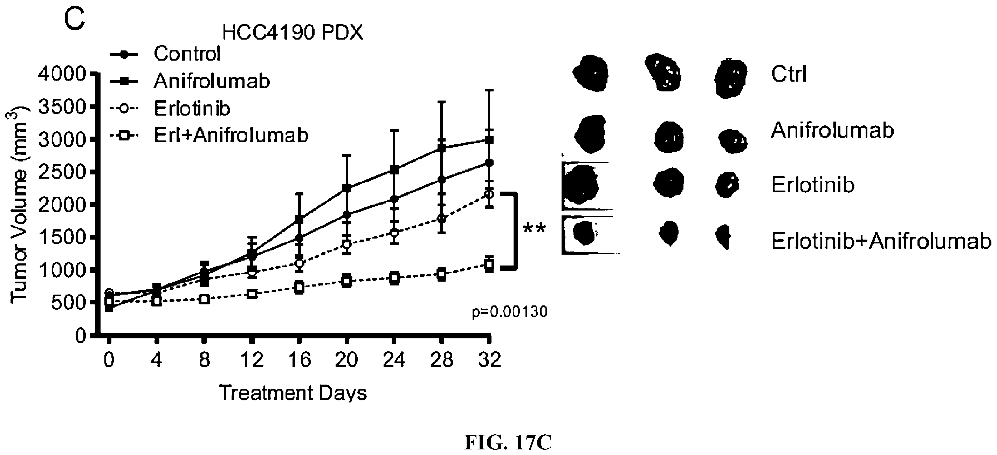

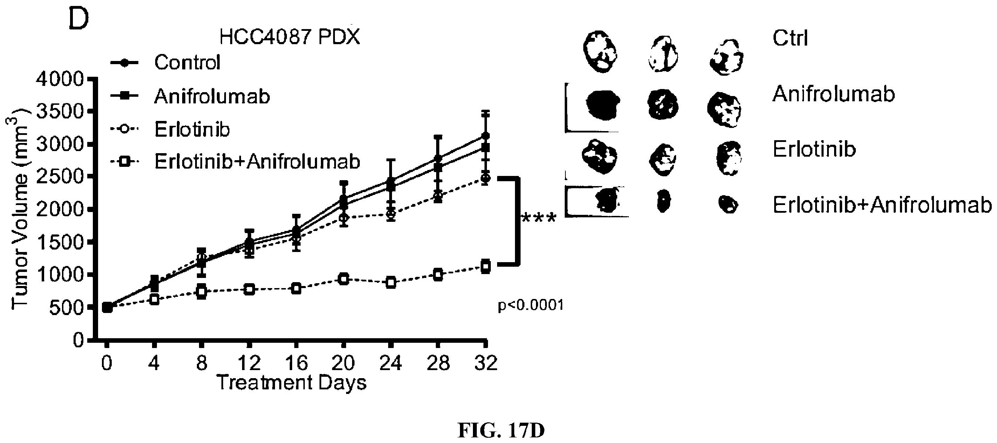

Next, whether a combined inhibition of Type I IFNs and EGFR would influence sensitivity to erlotinib was examined in mouse xenograft models. The Type I interferon receptor IFNAR is composed of two subunits IFNAR1 and IFNAR2. HCC827 cells with stable silencing of IFNAR1 or control shRNA were generated and tested in cell viability assays followed by injection into the flanks of athymic mice to form subcutaneous tumors (FIG. 17A). Once tumors became visible, treatment was started with control vehicle or low dose erlotinib. While low dose erlotinib failed to inhibit the growth of control tumors, there was a significant suppression of tumor growth in the IFNAR1 silenced group (FIG. 17A). Next, A549 cells with stable silencing of IFNA1R or control shRNA were generated and tested by cell viability assays followed by injection into the flanks of athymic mice to form subcutaneous tumors (FIG. 17B). While erlotinib failed to inhibit the growth of control tumors, there was a significant suppression of tumor growth in the IFNAR silenced group (FIG. 17B). Next, whether a combination of EGFR plus IFN inhibition acts synergistically controlling tumor growth in an EGFR mutant NSCLC PDX model (L858R, HCC4190) was examined. Anifrolumab, a monoclonal antibody directed against the IFNAR, was used in this experiment. While erlotinib alone produced a minor suppression of tumor growth that was not statistically significant, a combination of erlotinib plus anifrolumab results in a significant suppression of tumor growth (FIG. 17C). The combination of erotinib plus anifrolumab was also found to be highly effective in inhibiting the growth of an EGFRwt/KRas mutant PDX model HCC4087 (FIG. 17D).

Referring to FIG. 17A, HCC827 cells were stably infected with lentivirus control shRNA (shCtrl) or shRNA for IFNAR1 lentivirus and Western blot was conducted to confirm silencing. Silenced clones were studied in AlamarBlue cell survival assays following erlotinib exposure for 72h. HCC827 cells with stable silencing of IFNAR1 (clone #3) or control shRNA were subcutaneously injected into 8 nude mice per group. The rate of tumor formation was 5-8 per group as shown in the Source Data. Erlotinib was administered daily at 6.25 mg/kg by oral gavage. Tumor sizes were monitored as described in the Methods section. Representative tumor images are shown.

Referring to FIG. 17B, a similar in vivo experiments were performed with A549 xenografts (shIFNAR1 clone #2). Eight nude mice were injected per group and the rate of tumor formation was 5-8 as shown in the source data. Erlotinib was used at 100 mg/kg/d.

Referring to FIG. 17C, HCC4190 EGFR mutant PDX was subcutaneously implanted on NOD-SCID mice. Eight nude mice were injected per group and the rate of tumor formation was 7-8 as shown in the source data. Mice were treated with 6.25 mg/kg/d Erlotinib by oral gavage and/or i.p. injected with 2 mg/kg/d Anifrolumab, a monoclonal IFNAR1 antibody. Representative tumor images are shown.

Referring to FIG. 17D, a similar PDX experiment was performed with HCC4087, which harbor mutant KRAS and wild-type EGFR. Eight nude mice were injected per group and all 8 mice formed tumors. Erlotinib was used at a dose of 100 mg/kg/d.

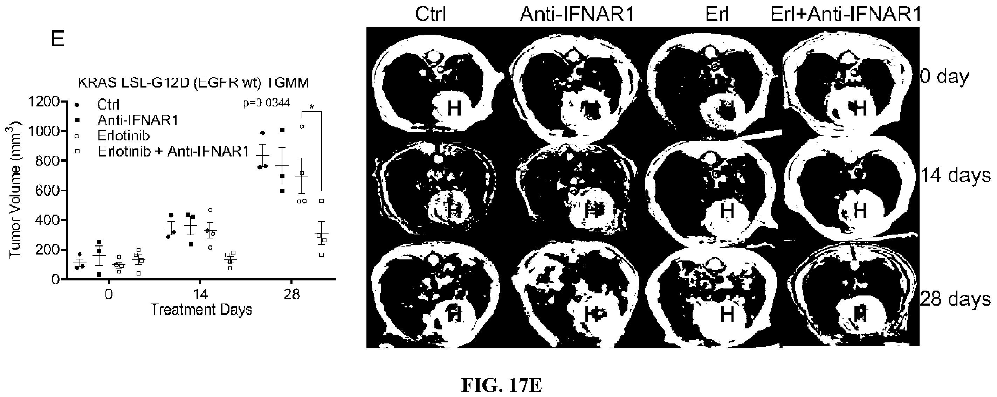

Because the type I interferons play a key role in innate immunity, a well-established immunocompetent transgenic mouse model of KRas mutant lung cancer that is driven by Adeno-CMV-Cre-mediated induction of KRas G12D expression was used. These tumors express EGFRwt and previous studies have shown that ErbB receptors play an important role in driving the growth of KRas mutant tumors. Since anifrolumab is specifically directed against the human IFNAR, a Type I IFN neutralizing antibody directed against the mouse IFNAR was used. Once tumors were detected by magnetic resonance imaging (MRI) following adenovirus administration, treatment was started with control vehicle, erlotinib, IFN antibody, or erlotinib plus IFN antibody followed by periodic MRI imaging. While there is robust tumor growth in control, erlotinib alone, and IFN antibody treatment groups, a combination of erlotinib plus IFN antibody was highly effective in suppressing growth of these tumors resulting in significantly diminished tumor growth compared to other groups (FIG. 17E). Thus, a combination of EGFR+ IFN inhibition is an effective treatment strategy in this immunocompetent model.

Referring to FIG. 17E, KRAS LSL-G12D transgenic mice were generated as in the Methods section, and randomly divided into 4 groups (n as number of dots), receiving vehicle, oral Erlotinib of 100 mg/kg/d, i.p. injection of mouse anti-mouse IFNAR1 antibody at 3 mg/kg/d, and combination administration of Erlotinib plus IFNAR1 antibody for 28 continuous days. Bi-weekly MRI scanning was used to monitor tumor growth. Tumor sizes were calculated by ImageJ. Representative MRI images are shown. The tumors grow as diffuse lung opacities and "H" refers to heart.

### 13. THE IFN ADAPTIVE RESPONSE TO EGFR INHIBITION IN NSCLC

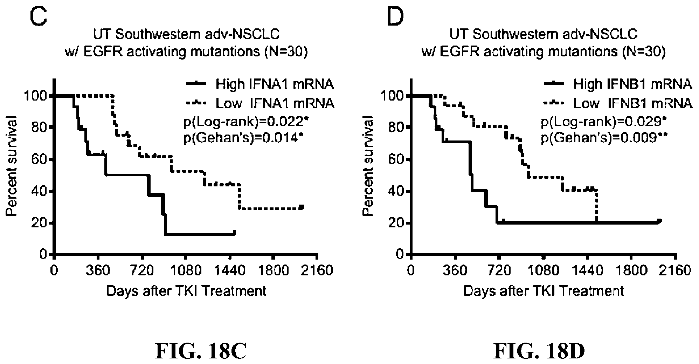

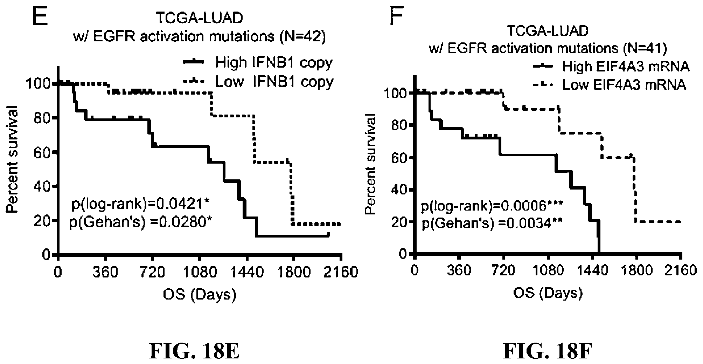

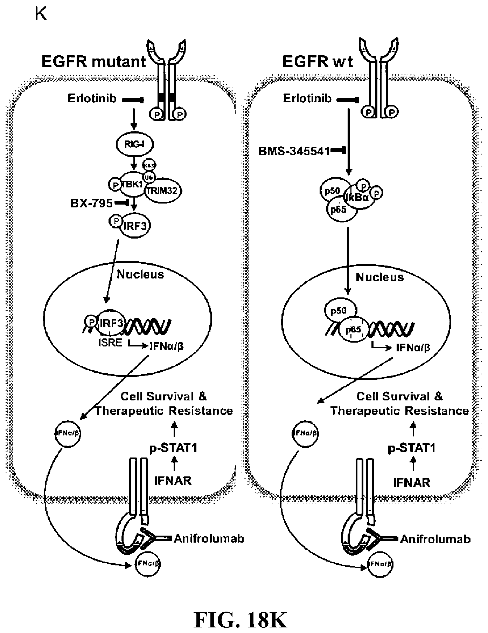

Next, whether EGFR inhibition induces a Type I interferon upregulation in tumor tissue derived from patients was studied. 13 TKI naive and 9 TKI treated NSCLC patients were examined, and it was found that erlotinib treated patients had higher Type I IFN levels compared to TKI naive patients (FIG. 18A and FIG. 18B). The experimental data indicate that a high level of Type I IFN protects cancer cells from the effects of EGFR TKIs. Thus, tissue from a cohort of EGFR mutant NSCLC patients treated at UT Southwestern with EGFR TKIs was examined. Type I IFN levels in paraffin-embedded pre-treatment tumor tissue were measured by qPCR and the effect of IFN levels on prognosis examined. Consistent with the experimental data, it was found that increased levels of IFNA1 mRNA or IFNB1 mRNA confer resistance to EGFR TKIs and a worse prognosis in these patients (FIG. 18C and FIG. 18D). Importantly, an analysis of TCGA data also reveals a correlation between high Type I IFN DNA copy number and worse prognosis in EGFR mutant NSCLC (FIG. 18E). Furthermore, expression of IFN target genes may also correlate negatively with prognosis in EGFR mutant tumors (FIG. 18F-J). Without wishing to be bound by theory, these data support the model that Type I IFN upregulation plays an important role in resistance to EGFR inhibition in NSCLC (FIG. 18K).

Referring to FIG. 18A and FIG. 18B, IFNA1 and IFNB1 mRNA was detected by qPCR from 23 NSCLC patients' FFPE tissues (13 untreated and 10 TKI-treated), collected from UT Southwestern Medical Center and The Jackson Laboratory. Data represents median ± IQR, **: p<0.01, ***: p<0.001, by Kolmogorov-Smimov test (KS test).

Referring to FIG. 18C and FIG. 18D, IFNA1 and IFNβ1 mRNA from 30 advanced (stages IIIB & IV) NSCLC patients at UT Southwestern with either one of two classical TKI-sensitive mutations, L858R or exon 19 deletion, but without T790M mutation were examined. Specifically, IFNA1 and IFNB1 mRNA by were examined qPCR from these 30 patients' FFPE tumor obtained before TKI-treatment. They were divided into high-50% and low-50% (n=15) by relative values and the effect of IFN level on overall survival was examined.

Referring to FIG. 18E, 42 EGFR activating mutant NSCLC patients from TCGA-LUAD database were divided into high-50% and low-50% groups by IFNB1 DNA copy numbers and the effect on survival was examined.

Referring to FIG. 18F-J, 41 EGFR activating mutant NSCLC patients (one patient has DNAseq but lacks RNAseq data) from TCGA-LUAD database were divided into high-50% and low-50% groups by mRNA levels of these erlotinib-induced Type I IFN target genes and the effect on survival was examined. Kaplan-Meier Overall-Survival curves (C-E) were drawn and compared by Log-rank test and Gehan's test.

Referring to FIG. 18K, a schematic representation of the EGFR inhibition induced Type I interferon driven adaptive response in NSCLC is shown.

## Claims

1. A combination for use in the treatment of non-small cell lung cancer (NSCLC), the combination comprising an effective amount of erlotinib and anifrolumab.

2. The combination for use according to claim 1, wherein erlotinib and anifrolumab are co-formulated.

3. The combination for use of claim 1, wherein erlotinib and anifrolumab are not administered concurrently.

4. The combination for use according to any one of claims 1 to 3, wherein the NSCLCexpresses EGFR wild type.

5. The combination for use according to any one of claims 1 to 3, wherein the NSCLC expresses EGFR mutant.

6. The combination for use according to any one of claims 1 to 5, wherein the NSCLC is resistant to EGFR inhibition.

7. A pharmaceutical composition comprising:
(a) erlotinib;
(b) anifrolumab; and
(c) a pharmaceutically acceptable carrier.

## Patentansprüche

1. Kombination zur Verwendung bei der Behandlung von nicht-kleinzelligem Lungenkrebs (NSCLC), wobei die Kombination eine wirksame Menge an Erlotinib und Anifrolumab umfasst.

2. Die Kombination zur Verwendung gemäß Anspruch 1, wobei Erlotinib und Anifrolumab gemeinsam formuliert sind.

3. Die Kombination zur Verwendung gemäß Anspruch 1, wobei Erlotinib und Anifrolumab nicht gleichzeitig verabreicht werden.

4. Die Kombination zur Verwendung gemäß einem der Ansprüche 1 bis 3, wobei der NSCLC EGFR-Wildtyp exprimiert.

5. Die Kombination zur Verwendung gemäß einem der Ansprüche 1 bis 3, wobei das NSCLC EGFR-Mutanten exprimiert.

6. Die Kombination zur Verwendung gemäß einem der Ansprüche 1 bis 5, wobei das NSCLC gegen EGFR-Hemmung resistent ist.

7. Pharmazeutische Zusammensetzung, umfassend:
(a) Erlotinib;
(b) Anifrolumab; und
(c) einen pharmazeutisch akzeptablen Träger.

## Revendications

1. Une combinaison destinée à être utilisée dans le traitement du cancer du poumon non à petites cellules (CPNPC), l'association comprenant une quantité efficace d'erlotinib et d'anifrolumab.

2. La combinaison destinée à être utilisée selon la revendication 1, dans laquelle l'erlotinib et l'anifrolumab sont coformulés.

3. La combinaison destinée à être utilisée selon la revendication 1, dans laquelle l'erlotinib et l'anifrolumab ne sont pas administrés simultanément.

4. La combinaison destinée à être utilisée selon l'une quelconque des revendications 1 à 3, dans laquelle le CPNPC exprime l'EGFR de type sauvage.

5. La combinaison destinée à être utilisée selon l'une quelconque des revendications 1 à 3, dans laquelle le CPNPC exprime un EGFR mutant.

6. Combinaison destinée à être utilisée selon l'une quelconque des revendications 1 à 5, dans laquelle le CPNPC est résistant à l'inhibition de l'EGFR.

7. Composition pharmaceutique comprenant :
(a) de l'erlotinib ;
(b) de l'anifrolumab ; et
(c) un excipient pharmaceutiquement acceptable.
